# EUROPEAN PATENT APPLICATION

(11) **EP 2 070 932 A1**
(43) Date of publication of application: **17.06.2009**
(21) Application number: 07024204.5
(22) Date of filing: 13.12.2007
(51) Int. Cl.: C07D 487/04, A61K 31/53, A61K 31/4162

(54) **Pyrazolotriazines**

(71) Applicant: 4SC AG, 82152 Planegg - Martinsried (DE)
(72) Inventor: The designation of the inventor has not yet been filed
(74) Representative: Vossius & Partner

(57) **Abstract**

Compounds of a certain formula I, in which R1, R2, A, R4, B, R5 and R6 have the meanings indicated in the description, are effective compounds with anti-proliferative and/or apoptosis inducing activity.

## Description

### Field of application of the invention

The invention relates to pyrazolotriazine derivatives, which can be used in the pharmaceutical industry for the production of pharmaceutical compositions.

### Known technical background

Eg5 (also called kinesin spindle protein) is a protein which is essential for the assembly and function of the bipolar mitotic spindle during cell division and which is a target for the discovery of novel cancer therapies.

In the document Hotha et al., Angew. Chem. 2003, 115, 2481-2484, the indolopyridine compound HR22C16 is described as inhibitor of cell division by targeting Eg5.

US application US2004/0242596 contains pyrazolo[3,4-d] pyrimidones which are said to be useful as anti-cancer agents and to induce mitotic arrest.

### Description of the invention

It has now been found, that the pyrazolotriazine derivatives, which are described in detail below, have surprising and advantageous properties. Particularly, they act as inhibitors of the mitotic kinesin Eg5.

### The invention thus relates to compounds of formula I

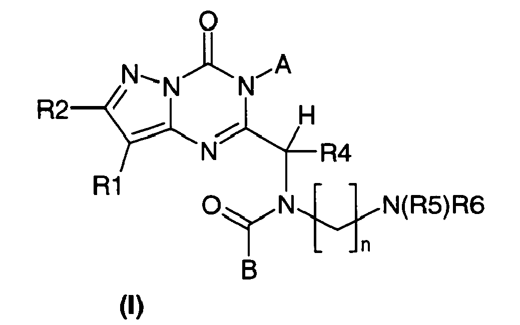

in which
- R1: is hydrogen or halogen,
- R2: is 1-4C-alkyl, 1-4C-alkoxy, hydrogen, 1-2C-haloalkoxy
- A: is Aryl-1-4C-alkyl, in which
Aryl is phenyl, or R3- and R31-substituted phenyl, in which
- R3: is 1-4C-alkyl, trifluoromethyl, 1-4C-alkoxy, hydroxyl, halogen, or completely or predominantly fluorine-substituted 1-4C-alkoxy,
- R31: is hydrogen, halogen, 1-4C-alkyl or 1-4C-alkoxy,
- R4: is hydrogen, 1-4C-alkyl, 3-7C-cycloalkyl or 3-7C-cycloalkyl-1-4C-alkyl,
- R5: is hydrogen or 1-4C-alkyl,
- R6: is hydrogen or 1-4C-alkyl,
- B: is phenyl, or R7- and R71-substituted phenyl, in which
- R7: is 1-4C-alkyl, trifluoromethyl, cyano, 1-4C-alkoxy, halogen, carboxyl, 1-4C-alkylcarbonyl, 1-4C-alkoxy-1-4C-alkyl, hydroxy-1-4C-alkyl, or completely or predominantly fluorine-substituted 1-4C-alkoxy,
- R71: is hydrogen, halogen, 1-4C-alkyl or 1-4C-alkoxy, or
- R7 and R71: when located in ortho position to each other and taken together are methylenedioxy or ethylenedioxy,
- n: is 2, 3, 4, 5 or 6,
and the salts, stereoisomers and the salts of the stereoisomers of these compounds.

1-4C-Alkyl represents straight-chain or branched alkyl groups having 1 to 4 carbon atoms. Examples which may be mentioned are the n-butyl, isobutyl, sec-butyl, tert-butyl, n-propyl, isopropyl, ethyl and the methyl group, of which n-propyl, isopropyl, ethyl and methyl are preferred.

3-7C-Cycloalkyl represents a monocyclic saturated aliphatic hydrocarbon group having 3 to 7 carbon atoms. Examples which may be mentioned are the cyclopropyl, cyclobutyl, cyclopentyl, cyclohexyl and the cycloheptyl group, of which cyclobutyl and cyclopentyl are preferred and cyclopropyl is particularly preferred.

3-7C-Cycloalkyl-1-4C-alkyl represents one of the aforementioned 1-4C-alkyl groups, which is substituted by one of the aforementioned 3-7C-cycloalkyl groups. Examples which may be mentioned are the 3-7C-cycloalkylmethyl groups, of which cyclobutylmethyl, cyclopentylmethyl are preferred and cyclopropylmethyl is particularly preferred.

Halogen within the meaning of the invention includes iodine, bromine, chlorine and fluorine, of which bromine, chlorine and fluorine are preferred.

Hydroxy-1-4C-alkyl represents one of the aforementioned 1-4C-alkyl groups which are substituted by a hydroxyl group. Examples which may be mentioned are the hydroxymethyl, the 2-hydroxyethyl and the 3-hydroxypropyl group, of which hydroxymethyl is preferred.

1-4C-Alkoxy represents groups, which in addition to the oxygen atom contain a straight-chain or branched alkyl group having 1 to 4 carbon atoms. Examples which may be mentioned are the n-butoxy, isobutoxy, sec-butoxy, tert-butoxy, n-propoxy, isopropoxy, methoxy and the ethoxy group, of which n-propoxy and isopropoxy are preferred and methoxy and ethoxy are particularly preferred.

1-2C-haloalkoxy represents groups, which, in addition to the oxygen atom, contain an alkyl group having 1 to 2 carbon atoms, which are substituted by one or more halogen groups. Examples which may be mentioned are the halogenated ethoxy and the halogenated methoxy group, of which difluoromethoxy is preferred.

1-4C-Alkoxy-1-4C-alkyl represents one of the aforementioned 1-4C-alkyl groups, which is substituted by one of the aforementioned 1-4C-alkoxy groups. Examples which may be mentioned are the methoxymethyl, ethoxymethyl, isopropoxymethyl, 2-methoxyethyl, 2-ethoxyethyl and the 2-isopropoxyethyl group, of which ethoxymethyl and isopropoxymethyl are preferred and methoxymethyl is particularly preferred.

1-4C-Alkylcarbonyl represents a group, which in addition to the carbonyl group contains one of the aforementioned 1-4C-alkyl groups. An example which may be mentioned is the acetyl group.

Completely or predominantly fluorine-substituted 1-4C-alkoxy is, for example, the 2,2,3,3,3-pentafluoro-n-propoxy, the perfluoroethoxy, the 1,2,2-trifluoroethoxy and in particular the 1,1,2,2-tetrafluoroethoxy, the 2,2,2-trifluoroethoxy, the trifluoromethoxy and the difluoromethoxy group, of which the trifluoromethoxy and the difluoromethoxy groups are preferred. "Predominantly" in this connection means that more than half of the hydrogen atoms of the 1-4C-alkoxy groups are replaced by fluorine atoms.

Hydroxy-1-4C-alkoxy represents one of the abovementioned 1-4C-alkoxy groups, which is substituted by a hydroxyl group. Examples which may be mentioned are the 2-hydroxyethoxy and the 3-hydroxy-n-propoxy group.

Aryl represents a phenyl group, or a R3- and R31-substituted phenyl group.

Aryl-1-4C-alkyl represents one of the aforementioned 1-4C-alkyl groups, which is substituted by one of the aforementioned aryl groups. Examples which may be mentioned are the aryl-methyl and the 2-aryl-ethyl group, of which aryl-methyl is preferred.

Further examples of the Aryl-1-4C-alkyl group which may be mentioned are the benzyl and the phenethyl group, of which benzyl is preferred, and each of which is optionally substituted by R3 and R31 on the phenyl moiety.

Constituents which are optionally substituted as stated herein, may be substituted, unless otherwise noted, at any possible position.

When any variable occurs more than one time in any constituent, each definition is independent.

In a preferred embodiment, the invention relates to compounds of formula I, in which
- R1: is hydrogen or halogen,
- R2: is 1-2C-alkyl, 1-4C-alkoxy, hydrogen, 1-2C-haloalkoxy
- A: is Aryl-1-2C-alkyl, in which
- Aryl: is phenyl, or R3- and R31-substituted phenyl, in which
- R3: is 1-2C-alkyl, trifluoromethyl, 1-2C-alkoxy, hydroxyl, halogen, or completely or predominantly fluorine-substituted 1-2C-alkoxy,
- R31: is hydrogen, halogen, 1-2C-alkyl or 1-2C-alkoxy,
- R4: is hydrogen, 1-4C-alkyl, cyclopropyl or cyclopropyl-1-2C-alkyl,
- R5: is hydrogen or 1-3C-alkyl,
- R6: is hydrogen or 1-3C-alkyl,
- B: is phenyl, or R7- and R71-substituted phenyl, in which
- R7: is 1-2C-alkyl, trifluoromethyl, cyano, 1-2C-alkoxy, halogen, carboxyl, 1-2C-alkylcarbonyl, 1-2C-alkoxy-1-2C-alkyl, hydroxy-1-2C-alkyl, or completely or predominantly fluorine-substituted 1-2C-alkoxy,
- R71: is hydrogen, halogen, 1-2C-alkyl or 1-2C-alkoxy, or
- R7 and R71: when located in ortho position to each other and taken together are methylenedioxy or ethylenedioxy,
- n: is 2, 3 or 4,
and the salts, stereoisomers and the salts of the stereoisomers of these compounds.

In a further preferred embodiment, the invention relates to compounds of formula I, in which
- R1: is hydrogen or halogen,
- R2: is methyl or ethyl, methoxy or ethoxy, hydrogen, halomethoxy or haloethoxy,
- A: is Arylmethyl, in which
- Aryl: is phenyl, or R3- and R31-substituted phenyl, in which
- R3: is methyl, ethyl, trifluoromethyl, methoxy, ethoxy, hydroxyl, halogen, difluoromethoxy or trifluoromethoxy,
- R31: is hydrogen, halogen, methyl, ethyl, methoxy or ethoxy,
- R4: is methyl, ethyl, n-propyl, isopropyl, sec-butyl, cyclopropyl or cyclopropylmethyl,
- R5: is hydrogen, methyl, ethyl, n-propyl or isopropyl,
- R6: is hydrogen, methyl, ethyl, n-propyl or isopropyl,
- B: is phenyl, or R7- and R71-substituted phenyl, in which
- R7: is methyl, ethyl, trifluoromethyl, cyano, methoxy, ethoxy, halogen, carboxyl, acetyl, methoxymethyl, hydroxymethyl, difluoromethoxy or trifluoromethoxy,
- R71: is hydrogen, halogen, methyl, ethyl, methoxy or ethoxy, or
- R7 and R71: when located in ortho position to each other and taken together are methylenedioxy or ethylenedioxy,
- n: is 2, 3 or 4,
and the salts, stereoisomers and the salts of the stereoisomers of these compounds.

In a further preferred embodiment, the invention relates to compounds of formula I, in which
- R1: is hydrogen, fluorine, chlorine or bromine,
- R2: is methyl or ethyl, methoxy or ethoxy, hydrogen, halomethoxy or haloethoxy,
- A: is Arylmethyl, in which
- Aryl: is phenyl, or R3- and R31-substituted phenyl, in which
- R3: is methyl, ethyl, trifluoromethyl, methoxy, ethoxy, hydroxyl or halogen,
- R31: is hydrogen, halogen, methyl or methoxy,
- R4: is ethyl, n-propyl, isopropyl, sec-butyl, cyclopropyl or cyclopropylmethyl,
- R5: is hydrogen, methyl, ethyl, n-propyl or isopropyl,
- R6: is hydrogen, methyl, ethyl, n-propyl or isopropyl,
- B: is phenyl, or R7- and R71-substituted phenyl, in which
- R7: is methyl, ethyl, trifluoromethyl, cyano, methoxy, ethoxy, halogen, carboxyl, methoxymethyl or hydroxymethyl,
- R71: is hydrogen, halogen, methyl, ethyl or methoxy, or
- R7 and R71: when located in ortho position to each other and taken together are methylenedioxy or ethylenedioxy,
- n: is 2, 3 or 4,
and the salts, stereoisomers and the salts of the stereoisomers of these compounds.

In a further preferred embodiment, the invention relates to compounds of formula I, in which
- R1: is hydrogen, chlorine or bromine,
- R2: is methyl or ethyl, methoxy or ethoxy, hydrogen, halomethoxy or haloethoxy,
- A: is benzyl, halobenzyl (e.g. fluorobenzyl, chlorobenzyl or bromobenzyl), methylbenzyl, methylhalobenzyl, methoxybenzyl, hydroxybenzyl, dihalobenzyl (e.g. dichlorobenzyl), or dimethoxybenzyl,
- R4: is ethyl, n-propyl, isopropyl, sec-butyl, cyclopropyl or cyclopropylmethyl,
- R5: is hydrogen, methyl, ethyl, n-propyl or isopropyl,
- R6: is hydrogen, methyl, ethyl, n-propyl or isopropyl,
- B: is phenyl, or R7- and R71-substituted phenyl, in which
- R7: is methyl, ethyl, trifluoromethyl, cyano, methoxy, ethoxy, halogen, carboxyl, methoxymethyl or hydroxymethyl,
- R71: is hydrogen, halogen, methyl or ethyl, or
- R7 and R71: when located in ortho position to each other and taken together are methylenedioxy or ethylenedioxy, such as, for example,
- B: is phenyl, methylphenyl, ethylphenyl, halophenyl, methylhalophenyl, (hydroxymethyl)phenyl, methoxyphenyl, ethoxyphenyl, trifluoromethylphenyl, halo(trifluoromethyl)phenyl, dihalophenyl, methylenedioxyphenyl, ethylenedioxyphenyl, (methoxymethyl)phenyl, methoxychlorophenyl, carboxyphenyl or cyanophenyl,
- n: is 2, 3 or 4,
and the salts, stereoisomers and the salts of the stereoisomers of these compounds.

In a further preferred embodiment, the invention relates to compounds of formula I, in which
- R1: is hydrogen, chlorine or bromine,
- R2: is methyl or ethyl, methoxy or ethoxy, hydrogen, halomethoxy or haloethoxy,
- A: is benzyl, halobenzyl (e.g. fluorobenzyl, chlorobenzyl or bromobenzyl), methylbenzyl, methoxybenzyl or hydroxybenzyl,
- R4: is ethyl, n-propyl, isopropyl, sec-butyl or cyclopropyl, either
- R5 and R6: are both hydrogen,
or
- R5: is methyl, and
- R6: is hydrogen,
or
- R5: is ethyl, and
- R6: is hydrogen,
or
- R5: is n-propyl, and
- R6: is hydrogen,
or
- R5: is isopropyl, and
- R6: is hydrogen,
or
- R5 and R6: are both methyl,
- B: is phenyl, or R7- and R71-substituted phenyl, in which
- R7: is methyl, ethyl, trifluoromethyl, cyano, methoxy, ethoxy, halogen, carboxyl, methoxymethyl or hydroxymethyl,
- R71: is hydrogen, halogen, methyl or ethyl, or
- R7 and R71: when located in ortho position to each other and taken together are methylenedioxy or ethylenedioxy, such as, for example,
- B: is phenyl, methylphenyl, ethylphenyl, halophenyl, dihalophenyl, methylhalophenyl, (hydroxymethyl)phenyl, halo(trifluoromethyl)phenyl, trifluoromethylphenyl, methoxyphenyl, methylenedioxyphenyl or cyanophenyl, such as, for more detailed example,
- B: is phenyl, 4-methylphenyl, 3-methylphenyl, 4-chlorophenyl, 4-bromophenyl, 4-fluorophenyl, 3-fluoro-4-methylphenyl, 4-trifluoromethylphenyl, 4-methoxyphenyl, 4-trifluoromethylphenyl, 3,4-dichlorophenyl or 2,3-dichlorophenyl,
- n: is 2, 3 or 4,
and the salts, stereoisomers and the salts of the stereoisomers of these compounds.

In a further preferred embodiment, the invention relates to compounds of formula I, in which
- R1: is hydrogen, chlorine or bromine, particularly
- R1: is chlorine or bromine,
- R2: is methyl, methoxy, difluoromethoxy or hydrogen,
- A: is benzyl, fluorobenzyl, chlorobenzyl, bromobenzyl, methylbenzyl, methoxybenzyl or hydroxybenzyl, in particular
- A: is benzyl or fluorobenzyl (e.g. 3-fluorobenzyl or 4-fluorobenzyl),
- R4: is ethyl, n-propyl, isopropyl, sec-butyl or cyclopropyl, either
- R5 and R6: are both hydrogen,
or
- R5: is methyl, and
- R6: is hydrogen,
or
- R5: is ethyl, and
- R6: is hydrogen,
or
- R5 and R6: are both methyl,
- B: is phenyl, or R7- and R71-substituted phenyl, in which
- R7: is methyl, ethyl, trifluoromethyl, fluorine, chlorine or bromine,
- R71: is hydrogen, methyl, fluorine, chlorine or bromine, such as, for example,
- B: is phenyl, methylphenyl, trifluoromethylphenyl, methoxyphenyl, halophenyl, dihalophenyl or methylhalophenyl,
such as, for more detailed example,
- B: is phenyl, methylphenyl, trifluoromethylphenyl, methoxyphenyl, fluorophenyl, chlorophenyl, bromophenyl, dichlorophenyl, difluorophenyl, methylfluorophenyl, methylchlorophenyl or methylbromophenyl,
such as, for yet more detailed example,
- B: is phenyl, 4-methylphenyl, 3-methylphenyl, 4-chlorophenyl, 4-bromophenyl, 4-fluorophenyl, 3-fluoro-4-methylphenyl, 4-trifluoromethylphenyl, 4-methoxyphenyl or 3,4-dichlorophenyl,
- n: is 2 or 3,
and the salts, stereoisomers and the salts of the stereoisomers of these compounds.

In a further preferred embodiment, the invention relates to compounds of formula I, in which
- R1: is chlorine or bromine,
- R2: is methyl, methoxy, difluoromethoxy or hydrogen,
- A: is benzyl or fluorobenzyl (e.g. 3-fluorobenzyl or 4-fluorobenzyl),
- R4: is ethyl, n-propyl, isopropyl, sec-butyl or cyclopropyl, either
- R5 and R6: are both hydrogen,
or
- R5 and R6: are both methyl,
- B: is phenyl, methylphenyl, trifluoromethylphenyl, methoxyphenyl, halophenyl, dihalophenyl or methylhalophenyl,
such as, for example,
- B: is methylphenyl, trifluoromethylphenyl, methoxyphenyl, fluorophenyl, chlorophenyl, bromophenyl, dichlorophenyl, difluorophenyl, methylfluorophenyl, methylchlorophenyl or methylbromophenyl,
such as, for more detailed example,
- B: is 4-methylphenyl, 3-methylphenyl, 4-chlorophenyl, 4-bromophenyl, 4-fluorophenyl, 3-fluoro-4-methylphenyl, 4-trifluoromethylphenyl, 4-methoxyphenyl or 3,4-dichlorophenyl,
- n: is 2 or 3,
and the salts, stereoisomers and the salts of the stereoisomers of these compounds.

In a further preferred embodiment, the invention relates to compounds of formula I, in which
- R1: is chlorine or bromine,
- R2: is methyl, methoxy, difluoromethoxy or hydrogen,
- A: is benzyl, 3-fluorobenzyl or 4-fluorobenzyl,
- R4: is ethyl, n-propyl, isopropyl or cyclopropyl, either
- R5 and R6: are both hydrogen,
or
- R5 and R6: are both methyl,
- B: is methylphenyl, trifluoromethylphenyl, methoxyphenyl, chlorophenyl, dichlorophenyl, bromophenyl, fluorophenyl or methylfluorophenyl,
such as, for example,
- B: is 4-methylphenyl, 3-methylphenyl, 4-trifluoromethylphenyl, 4-methoxyphenyl, 4-chlorophenyl, 4-bromophenyl, 4-fluorophenyl or 3-fluoro-4-methylphenyl,
- n: is 2 or 3,
and the salts, stereoisomers and the salts of the stereoisomers of these compounds.

In a further preferred embodiment, the invention relates to compounds of formula I, in which
- R1: is hydrogen, chlorine or bromine,
- R2: is methyl, methoxy, difluoromethoxy or hydrogen,
- A: is benzyl or fluorobenzyl,
- R4: is ethyl, n-propyl, isopropyl, sec-butyl or cyclopropyl, either
- R5 and R6: are both hydrogen,
or
- R5 and R6: are both methyl,
- B: is phenyl, methylphenyl, trifluoromethylphenyl, methoxyphenyl, halophenyl, dihalophenyl or methylhalophenyl,
- n: is 2 or 3,
and the salts, stereoisomers and the salts of the stereoisomers of these compounds.

In a further preferred embodiment, the invention relates to compounds of formula I, in which
- R1: is chlorine or bromine,
- R2: is methyl, methoxy, difluoromethoxy or hydrogen,
- A: is benzyl,
- R4: is ethyl or isopropyl,
either
- R5 and R6: are both hydrogen,
or
- R5 and R6: are both methyl,
- B: is methylphenyl, chlorophenyl, trifluoromethylphenyl, methoxyphenyl, bromophenyl or fluorophenyl,
such as, for example,
- B: is 4-methylphenyl, 3-methylphenyl, 4-trifluoromethylphenyl, 4-methoxyphenyl, 4-chlorophenyl, 4-bromophenyl or 4-fluorophenyl,
- n: is 3,
and the salts, stereoisomers and the salts of the stereoisomers of these compounds.

In a further preferred embodiment, the invention relates to compounds of formula I, in which
- R1: is chlorine or bromine;
- R2: is methyl, methoxy, difluoromethoxy or hydrogen,;
- A: is benzyl;
- R4: is ethyl or isopropyl;
either
- R5 and R6: are both hydrogen,
or
- R5 and R6: are both methyl;
- B: is 4-methylphenyl, 3-methylphenyl, 4-trifluoromethylphenyl, 4-methoxyphenyl, 4-fluorophenyl, 4-chlorophenyl, 4-bromophenyl or 3-fluoro-4-methylphenyl; and
- n: is 3;
and the salts, stereoisomers and the salts of the stereoisomers of these compounds.

In a further preferred embodiment, the invention relates to compounds of formula I, in which
- R1: is chlorine or bromine,
- R2: is methyl, methoxy, difluoromethoxy or hydrogen,
- A: is benzyl,

- R4: is isopropyl,
- R5 and R6: are both hydrogen,
- B: is methylphenyl, chlorophenyl, trifluoromethylphenyl, methoxyphenyl, bromophenyl or fluorophenyl,
such as, for example,
- B: is 4-methylphenyl, 4-chlorophenyl or 4-bromophenyl,
- n: is 3,
and the salts, stereoisomers and the salts of the stereoisomers of these compounds.

In a further preferred embodiment, the invention relates to compounds of formula I, in which
- R1: is chlorine or bromine;
- R2: is methyl methoxy, difluoromethoxy or hydrogen;
- A: is benzyl;
- R4: is isopropyl;
- R5 and R6: are both hydrogen;
- B: is 4-methylphenyl, 4-chlorophenyl or 4-bromophenyl; and
- n: is 3;
and the salts, stereoisomers and the salts of the stereoisomers of these compounds.

In a further preferred embodiment, the invention relates to compounds of formula I, in which
- R1: is hydrogen, chlorine, bromine or fluorine,
- R2: is methyl, methoxy, difluoromethoxy or hydrogen,
- A: is benzyl,
- R4: is ethyl, isopropyl, sec-butyl, cyclopropyl or cyclobutyl,
either
- R5 and R6: are both hydrogen,
or
- R5 and R6: are both methyl,
- B: is 4-methylphenyl, 3-methylphenyl, 4-fluorophenyl, 4-bromophenyl, 4-chlorophenyl, 4-methoxyphenyl, 4-trifluoromethylphenyl, 3-fluoro-4-methylphenyl, 2- fluoro-4-methylphenyl, 3,4-dichlorophenyl or 2,3-dichlorophenyl,
- n: is 2, 3 or 4,
and the salts, stereoisomers and the salts of the stereoisomers of these compounds.

In a further preferred embodiment, the invention relates to compounds of formula I, in which
- R1: is hydrogen, chlorine or bromine,
- R2: is methyl, methoxy, difluoromethoxy or hydrogen,
- A: is benzyl,
- R4: is ethyl or isopropyl,
either
- R5 and R6: are both hydrogen,
or
- R5 and R6: are both methyl,
- B: is 4-methylphenyl, 3-methylphenyl, 4-fluorophenyl, 4-bromophenyl, 4-chlorophenyl, 4-methoxyphenyl,
- n: is 3,
and the salts, stereoisomers and the salts of the stereoisomers of these compounds.

In a further preferred embodiment, the invention relates to compounds of formula I, in which
- R1: is chlorine, bromine or fluorine,
- R2: is methyl, methoxy, difluoromethoxy or hydrogen,
- A: is benzyl,
- R4: is ethyl, isopropyl, sec-butyl or cyclopropyl,
- R5 and R6: are both hydrogen,
- B: is 4-methylphenyl, 3-methylphenyl, 4-fluorophenyl, 4-bromophenyl, 4-chlorophenyl, 4-methoxyphenyl, 3-fluoro-4-methylphenyl, 2-fluoro-4-methylphenyl or 3,4-dichlorophenyl,
- n: is 3 or 4,
and the salts, stereoisomers and the salts of the stereoisomers of these compounds.

In a further preferred embodiment, the invention relates to compounds of formula I, in which
- R1: is chlorine or hydrogen,
- R2: is methyl, methoxy, difluoromethoxy or hydrogen,
- R5 and R6: are both hydrogen,
- B: is 4-methylphenyl
- n: is 2 or 3,
provided that not both of R1 and R2 are hydrogen, and the salts, stereoisomers and the salts of the stereoisomers of these compounds;
whereas in a more preferred embodiment A is benzyl and R4 is isopropyl.

Salts of the compounds according to the invention include all inorganic and organic acid addition salts and salts with bases, depending on the substitution. Preferred are the pharmaceutically acceptable inorganic and organic acid addition salts and salts with bases, particularly all pharmaceutically acceptable inorganic and organic acid addition salts and salts with bases customarily used in pharmacy. Acids used for the preparation of the acid addition salts include, but are not limited to (1) inorganic acids, (2) carboxylic acids, (a) aliphatic, alicyclic, saturated or unsaturated carboxylic acids, (b) aromatic or heterocyclic carboxylic acids, (c) hydroxylated or carbohydrate-derived carboxylic acids, and (3) sulfonic acids. Suitable salts include water-insoluble and, particularly, water-soluble acid addition salts and salts with bases.

Examples of acid addition salts include, but are not limited to, hydrochlorides, sulfates, phosphates, hydrobromides, nitrates, acetates, trifluoroacetates, succinates, oxalates, maleates, fumarates, butyrates, stearates, laurates, benzoates, embonates, salicylates, sulphosalicylates, 2-(4-hydroxybenzoyl)benzoates, 3-hydroxy-2-naphthoates, citrates, tartarates such as (+)-L-tartarates or (-)-D-tartarates or meso-tartarates, lactates such as D-lactates or L-lactates, malates such as (-)-L-malates or (+)-D-malates, D-gluconates, D-glucuronates, lactobionates (salts of 4-O-beta-D-galactopyranosyl-D-gluconic acid), galactarates, tosilates, mesilates, besilates, laurylsulfonates, and ascorbates.
Of these acid addition salts, hydrochlorides, sulfates, acetates, mesilates, citrates, maleates, oxalates, fumarates and tartarates are preferred. Most preferred are the salts selected from hydrochlorides, mesylates, tartrates and citrates.

In one embodiment of this invention, salts of compounds of formula I include a hydrochloride of a compound of formula I.

In another embodiment of this invention, salts of compounds of formula I include a hydrochloride, phosphate, citrate, tartrate, mesylate, tosylate and sulfate of a compound of formula I.

Examples of salts with bases include, but are not limited to, lithium, sodium, potassium, calcium, aluminium, magnesium, titanium, ammonium, meglumine and guanidinium salts.

The compounds of formula I according to this invention, and the salts, stereoisomers and the salts of the stereoisomers of these compounds, may contain, e.g. when isolated in crystalline form, varying amounts of solvents. Included within the scope of the invention are therefore all solvates of the compounds of formula I, and the salts, the stereoisomers and the salts thereof. Hydrates are a preferred example of said solvates.

The substituents R3 and R31 of compounds of formula I may be attached, unless otherwise noted, in the ortho, meta or para position with respect to the binding position in which the Aryl ring is bonded to the 1-4C-alkyl group, wherein preference is given to the attachment of R3 in the meta or para position. In one embodiment R31 is hydrogen.

The substituents R7 and R71 of compounds of formula I may be attached, unless otherwise noted, in the ortho, meta or para position with respect to the binding position in which the phenyl ring is bonded to the carbonyl group, wherein preference is given to the attachment of R7 in the meta or para position. In one embodiment R71 is hydrogen.

### Numbering:

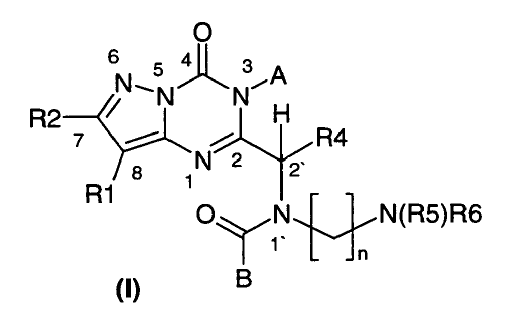

The compounds of formula I are chiral compounds having at least one chiral center in position 2'. Therefore, the compounds according to the invention and the salts thereof include stereoisomers. The stereogenic centers present in said stereoisomers may have the absolute configuration R or the absolute configuration S (according to the rules of Cahn, Ingold and Prelog). Accordingly, the stereoisomers (2'R) and (2'S), wherein the number refers to the atom indicated in formula above, and the salts thereof are part of the invention.

The invention includes all conceivable stereoisomers of compounds of formula I, like e.g. diastereomers and enantiomers. The invention further includes the stereoisomers in pure form as well as all mixtures of the stereoisomers mentioned above independent of the ratio, including the racemates, as well as the salts thereof.

Thus, stereoisomers of the compounds according to this invention, particularly stereoisomers of the following examples, are all part of the present invention and may be obtained according to procedures customary to the skilled person, e.g. by separation of corresponding mixtures, by using stereochemically pure starting materials and/or by stereoselective synthesis, as described in more detail below.

Preferred compounds of the present invention are the enantiomers of formula I and the salts thereof, which have, with respect to the position 2', the same configuration as shown in formula I*:

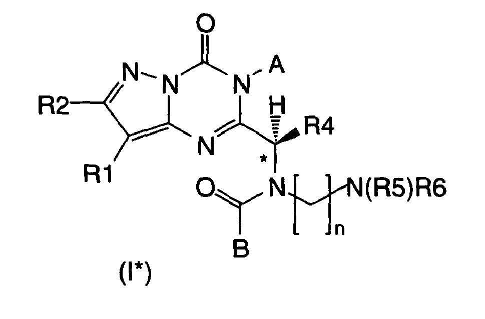

In compounds according to formula I*, in which R4 is methyl, ethyl, n-propyl, isopropyl, sec-butyl or cyclopropyl, the configuration - according to the rules of Cahn, Ingold and Prelog - is R in the 2' position.

Further preferred compounds of the present invention are the enantiomers of formula I and the salts thereof, which have, with respect to the position 2', the same configuration as shown in formula I**:

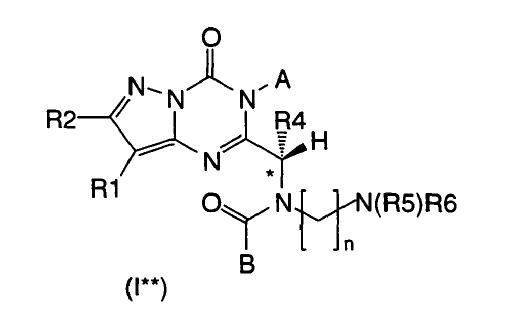

In compounds according to formula I**, in which R4 is methyl, ethyl, n-propyl, isopropyl, sec-butyl or cyclopropyl, the configuration - according to the rules of Cahn, Ingold and Prelog - is S in the position 2'.

A special interest in the compounds according to this invention refers to those compounds of formula I and their salts, stereoisomers and salts of stereoisomers which are included -within the scope of this invention- by one or, when possible, by a combination of more of the following special embodiments:
A special embodiment (embodiment 1) of the compounds of formula I according to this invention refers to those compounds of formula I and their salts, stereoisomers and salts of stereoisomers, in which
   R1 is hydrogen.
A special embodiment (embodiment 2) of the compounds of formula I according to this invention refers to those compounds of formula I and their salts, stereoisomers and salts of stereoisomers, in which
   R1 is chlorine.
A special embodiment (embodiment 3) of the compounds of formula I according to this invention refers to those compounds of formula I and their salts, stereoisomers and salts of stereoisomers, in which
   R1 is bromine.
A special embodiment (embodiment 3a) of the compounds of formula I according to this invention refers to those compounds of formula I and their salts, stereoisomers and salts of stereoisomers, in which
   R1 is fluorine.
A special embodiment (embodiment 4) of the compounds of formula I according to this invention refers to those compounds of formula I and their salts, stereoisomers and salts of stereoisomers, in which
   R2 is methyl, methoxy, difluoromethoxy or hydrogen.
A special embodiment (embodiment 5) of the compounds of formula I according to this invention refers to those compounds of formula I and their salts, stereoisomers and salts of stereoisomers, in which
   R1 is chlorine, and R2 is methyl, methoxy, difluoromethoxy or hydrogen.
A special embodiment (embodiment 6) of the compounds of formula I according to this invention refers to those compounds of formula I and their salts, stereoisomers and salts of stereoisomers, in which
   R1 is bromine, and R2 is methyl, methoxy, difluoromethoxy or hydrogen.
A special embodiment (embodiment 7) of the compounds of formula I according to this invention refers to those compounds of formula I and their salts, stereoisomers and salts of stereoisomers, in which
   A is benzyl.
A special embodiment (embodiment 8) of the compounds of formula I according to this invention refers to those compounds of formula I and their salts, stereoisomers and salts of stereoisomers, in which
   A is fluorobenzyl, such as e.g. 3-fluorobenzyl or 4-fluorobenzyl.
A special embodiment (embodiment 9) of the compounds of formula I according to this invention refers to those compounds of formula I and their salts, stereoisomers and salts of stereoisomers, in which
   A is benzyl, halobenzyl (such as e.g. chlorobenzyl or bromobenzyl), methylbenzyl, hydroxybenzyl or methoxybenzyl.
A special embodiment (embodiment 10) of the compounds of formula I according to this invention refers to those compounds of formula I and their salts, stereoisomers and salts of stereoisomers, in which
   R4 is ethyl.
A special embodiment (embodiment 11) of the compounds of formula I according to this invention refers to those compounds of formula I and their salts, stereoisomers and salts of stereoisomers, in which
   R4 is isopropyl.
A special embodiment (embodiment 11a) of the compounds of formula I according to this invention refers to those compounds of formula I and their salts, stereoisomers and salts of stereoisomers, in which
   R1 is chlorine, and R2 is methyl, methoxy, difluoromethoxy or hydrogen, and
   R4 is isopropyl.
A special embodiment (embodiment 11b) of the compounds of formula I according to this invention refers to those compounds of formula I and their salts, stereoisomers and salts of stereoisomers, in which
   A is benzyl, and
   R4 is isopropyl.
A special embodiment (embodiment 11c) of the compounds of formula I according to this invention refers to those compounds of formula I and their salts, stereoisomers and salts of stereoisomers, in which
   R1 is chlorine, and R2 is methyl, methoxy, difluoromethoxy or hydrogen,and
   A is benzyl, and
   R4 is isopropyl.
A special embodiment (embodiment 12) of the compounds of formula I according to this invention refers to those compounds of formula I and their salts, stereoisomers and salts of stereoisomers, in which
   R4 is n-propyl.
A special embodiment (embodiment 13) of the compounds of formula I according to this invention refers to those compounds of formula I and their salts, stereoisomers and salts of stereoisomers, in which
   R4 is cyclopropyl.
A special embodiment (embodiment 13a) of the compounds of formula I according to this invention refers to those compounds of formula I and their salts, stereoisomers and salts of stereoisomers, in which
   R4 is cyclobutyl.
A special embodiment (embodiment 14) of the compounds of formula I according to this invention refers to those compounds of formula I and their salts, stereoisomers and salts of stereoisomers, in which
   R4 is sec-butyl.
A special embodiment (embodiment 15) of the compounds of formula I according to this invention refers to those compounds of formula I and their salts, stereoisomers and salts of stereoisomers, in which
   n is 2.
A special embodiment (embodiment 16) of the compounds of formula I according to this invention refers to those compounds of formula I and their salts, stereoisomers and salts of stereoisomers, in which
   n is 3.
A special embodiment (embodiment 17) of the compounds of formula I according to this invention refers to those compounds of formula I and their salts, stereoisomers and salts of stereoisomers, in which
   R5 and R6 are the same and selected from hydrogen, methyl and ethyl.
A special embodiment (embodiment 18) of the compounds of formula I according to this invention refers to those compounds of formula I and their salts, stereoisomers and salts of stereoisomers, in which
   R5 is methyl, ethyl, n-propyl or isopropyl, and R6 is hydrogen.
A special embodiment (embodiment 19) of the compounds of formula I according to this invention refers to those compounds of formula I and their salts, stereoisomers and salts of stereoisomers, in which
   R5 is methyl or ethyl, and R6 is hydrogen.
A special embodiment (embodiment 20) of the compounds of formula I according to this invention refers to those compounds of formula I and their salts, stereoisomers and salts of stereoisomers, in which
   R5 and R6 are both hydrogen, and n is 2.
A special embodiment (embodiment 21) of the compounds of formula I according to this invention refers to those compounds of formula I and their salts, stereoisomers and salts of stereoisomers, in which
   R5 and R6 are both methyl, and n is 2.
A special embodiment (embodiment 22) of the compounds of formula I according to this invention refers to those compounds of formula I and their salts, stereoisomers and salts of stereoisomers, in which
   R5 and R6 are both hydrogen, and n is 3.
A special embodiment (embodiment 23) of the compounds of formula I according to this invention refers to those compounds of formula I and their salts, stereoisomers and salts of stereoisomers, in which
   R5 and R6 are both methyl, and n is 3.
A special embodiment (embodiment 24) of the compounds of formula I according to this invention refers to those compounds of formula I and their salts, stereoisomers and salts of stereoisomers, in which
   - B: is phenyl, or R7- and R71-substituted phenyl, in which
   - R7: is fluorine, chlorine, bromine, methyl, trifluoromethyl or ethyl,
   - R71: is hydrogen, fluorine, chlorine or methyl.
A special embodiment (embodiment 25) of the compounds of formula I according to this invention refers to those compounds of formula I and their salts, stereoisomers and salts of stereoisomers, in which
   - B: is phenyl, halophenyl, dihalophenyl, cyanophenyl, halo(trifluoromethyl)phenyl, (hydroxymethyl)phenyl, (methoxymethyl)phenyl, methoxyphenyl, ethoxyphenyl, carboxyphenyl, methylphenyl, ethylphenyl, methylenedioxyphenyl, ethylenedioxyphenyl, methoxychlorophenyl, methylhalophenyl or (trifluoromethyl)phenyl.
A special embodiment (embodiment 26) of the compounds of formula I according to this invention refers to those compounds of formula I and their salts, stereoisomers and salts of stereoisomers, in which
   - B: is phenyl, methylphenyl, halophenyl (e.g. chlorophenyl or bromophenyl), dihalophenyl (e.g. dichlorophenyl), methylhalophenyl (e.g. methylfluorophenyl), (hydroxymethyl)phenyl, halo(trifluoromethyl)phenyl, methylenedioxyphenyl, (trifluoromethyl)phenyl, methoxyphenyl or cyanophenyl.
A special embodiment (embodiment 27) of the compounds of formula I according to this invention refers to those compounds of formula I and their salts, stereoisomers and salts of stereoisomers, in which
   - B: is methylphenyl, chlorophenyl, dichlorophenyl, bromophenyl, fluorophenyl, (trifluoromethyl)phenyl, methoxyphenyl or methylfluorophenyl.
A special embodiment (embodiment 28) of the compounds of formula I according to this invention refers to those compounds of formula I and their salts, stereoisomers and salts of stereoisomers, in which
   - B: is 4-methylphenyl, 3-methylphenyl, 4-trifluoromethylphenyl, 4-methoxyphenyl, 4-fluorophenyl, 4-chlorophenyl, 4-bromophenyl or 3-fluoro-4-methylphenyl.
A special embodiment (embodiment 29) of the compounds of formula I according to this invention refers to those compounds of formula I and their salts, stereoisomers and salts of stereoisomers, in which
   - B: is 4-methylphenyl, 4-chlorophenyl or 4-bromophenyl.
A special embodiment (embodiment 30) of the compounds of formula I according to this invention refers to those compounds of formula I and their salts, stereoisomers and salts of stereoisomers, in which
   - B: is 4-methylphenyl.

A special embodiment (embodiment 31) of the compounds of formula I according to this invention refers to those compounds and their salts which are represented by formula I*:

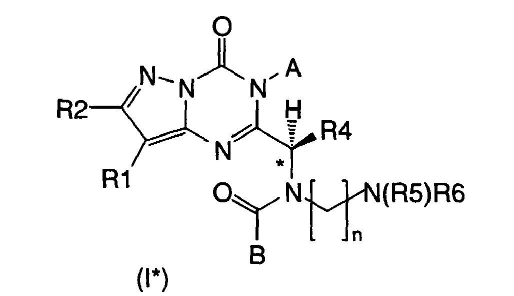

A special embodiment (embodiment 32) of the compounds of formula I according to this invention refers to those compounds and their salts which are represented by formula I**:

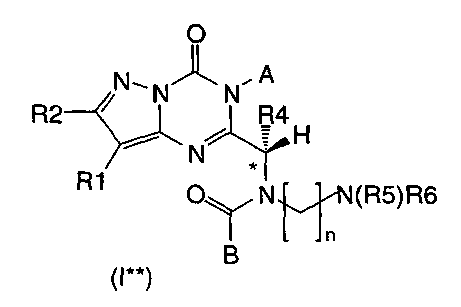

The compounds, or a salt, stereoisomer or a salt of a stereoisomer thereof, according to the invention can be prepared as follows. Preferably, they are prepared in a manner as described by way of example in the following examples. Furthermore, they can be prepared analogously to said preparation procedures or synthesis strategies known to the person skilled in the art.

"Analogously" includes performing a described reaction procedure, while choosing the corresponding reactants to yield the desired product. "Analogous procedures" differ from the described procedure in that reactants are replaced in order to adapt the procedure to represent the synthesis of the desired product.

As shown in the synthesis route outlined in scheme 1 below, the synthesis is based on the aminopyrazole derivative. Aminopyrazoles of formula X, in which R2 has the meanings given above, are known or can be prepared according to known procedures or as described in the following examples, or analogously or similarly thereto. The pyrazolotriazine is build up by an appropriate building block as a formal derivative of acetic acid and treating subsequently with a CO-reactive agent, like diethylcarbonate to obtain the pyrazolotriazinone heterocycle.

Compounds of formula XII are benzylated with compounds of formula A-X, in which A has the meanings given above and X is a suitable leaving group (e.g. halogen or the like), in a standard manner or as described exemplarily in the following examples to give corresponding compounds of formula XIII.

Subsequently compounds of formula XIV, in which R2 and A have the meanings given above and R1 is halogen, in particular fluorine or, in more particular, chlorine or bromine, are prepared by halogenation starting from compounds of formula XIII. Said halogenation reaction can be carried out according to customary procedures or as described in the following examples, or analogously or similarly thereto.

Thus, chlorination or bromination reaction can be carried out using an appropriate electrophilic chlorinating or brominating reagent, e.g. N-chlorosuccinimide (NCS) or N-bromosuccinimide (e.g. NBS / AIBN), respectively, under conditions customary for the skilled person or as described in the following examples.

Fluorination reaction may be carried out using a suitable electrophilic fluorinating reagent, such as e.g. 1-chloromethyl-4-fluoro-1,4-diazoniabicyclo[2.2.2]octane bis-(tetrafluoroborate) (Selectfluor I) or 1-methyl-4-fluoro-1,4-diazoniabicyclo[2.2.2]octane bis-(tetrafluoroborate) (Selectfluor II) or the like, under appropriate conditions.

Using appropriate conditions, said halogenation reaction is carried out largely regioselectively to obtain halogenation on the pyrazole moiety of the pyrazolotriazine scaffold.

The methyl group attached to the triazine moiety of the pyrazolotriazine of compounds of formula VI, in which R1 is halogen and A and R2 have the meanings given above, is oxidized under suitable conditions to obtain corresponding compounds of formula V. Said oxidation is carried out in a manner habitual for the skilled person or as described in the following examples using a suitable oxidation reagent, e.g. selenium dioxide, to give largely regioselectively the 5-formyl derivative of formula XV.

Compounds of formula XV, in which R1 is halogen and A and R2 have the meanings given above, are reacted in a nucleophilic addition reaction with compounds of formula R4-M, in which R4 is different from hydrogen and has the meanings given above and M is a suitable metal or metal complex, to give corresponding compounds of formula XV. Said nucleophilic addition reaction can be obtained using organomagnesium halides (Grignard reagents) of formula R4-Mg-X (X = Halogen) with or without further additives (e.g. LiCl or alkyl lithium reagents). Advantageously said reaction is carried out in the presence of alkyl lithium reagents, e.g. methyl lithium, forming in situ more reactive organomagnesium reagents, e.g. lithium trialkyl magnesiates such as R4Me₂MgLi from 2 equiv. LiMe and 1 equiv. R4-Mg-X. The nucleophilic addition reaction can be carried out in a manner customary for the skilled person or as described in the following examples.

Alcohols of formula XVI, in which R1 is hydrogen, fluorine or chlorine and A, R2 and R4 have the meanings given above, are converted into the corresponding ketones of formula XVII by oxidation reaction using an appropriate oxidation reagent, such as e.g. NMO (N-methylmorpholien-N-oxide) / TPAP (tetrapropylammonium perruthenate) or the like, under conditions known for the skilled person or as described in the following examples.

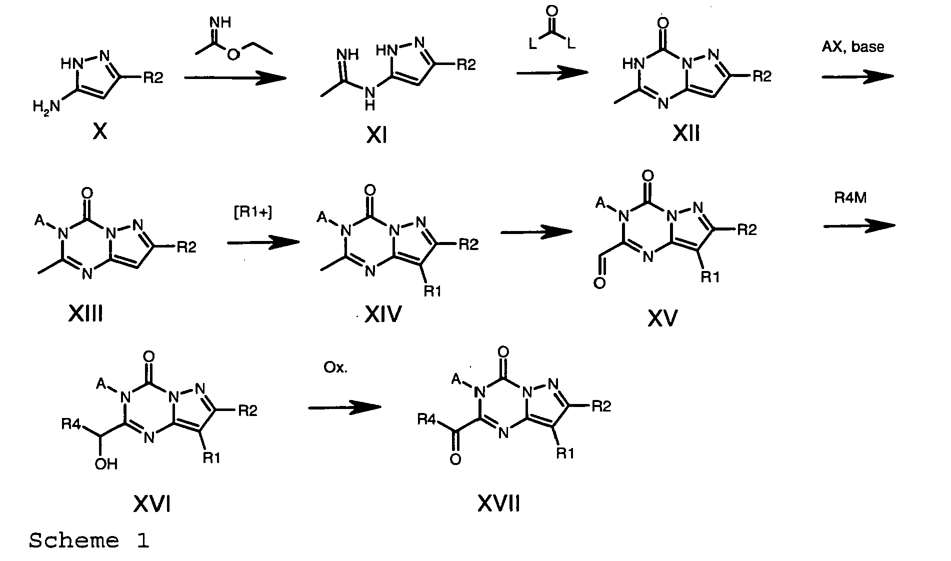

Said reaction sequence can be carried out as it is habitual for the skilled person or as described in the following examples or analogously or similarly thereto, in the presence of a suitable base (e.g. sodium carbonate) in a suitable solvent, for example water, at elevated temperature.

Alternatively the imidoester hydrochloride XVIII in which R4 has the meaning given above can be formed, as shown in the scheme 2 below, by starting either from the corresponding amide derivative and alkylate or treating the corresponding nitril with ethanol and HCl.

XVIII in which R4 has the meaning given above is then reacted with a aminopyrazole with R2 having the meaning above and in particular hydrogen, and an appropriate building block as CO-reactive agent, like diethylcarbonate or carbonyldimidazole to obtain the pyrazolotriazinone heterocycle XX.

Compounds of formula XX in which R2 and R4 have the meaning given above are benzylated with compounds of formula A-X, in which A has the meanings given above and X is a suitable leaving group (e.g. halogen or the like), in a standard manner or as described exemplarily in the following examples to give corresponding compounds of formula XXI.

Subsequently compounds of formula XXII, in which R2, R4 and A have the meanings given above and R1 is halogen, in particular fluorine or, in more particular, chlorine or bromine, are prepared by halogenation starting from compounds of formula XXI. Said halogenation reaction can be carried out according to customary procedures or as described in the following examples, or analogously or similarly thereto.

Thus, chlorination or bromination reaction can be carried out using an appropriate electrophilic chlorinating or brominating reagent, e.g. N-chlorosuccinimide (NCS) or N-bromosuccinimide (e.g. NBS / AIBN), respectively, under conditions customary for the skilled person or as described in the following examples.

Fluorination reaction may be carried out using a suitable electrophilic fluorinating reagent, such as e.g. 1-chloromethyl-4-fluoro-1,4-diazoniabicyclo[2.2.2]octane bis-(tetrafluoroborate) (Selectfluor I) or 1-methyl-4-fluoro-1,4-diazoniabicyclo[2.2.2]octane bis-(tetrafluoroborate) (Selectfluor II) or the like, under appropriate conditions.

Using appropriate conditions, said halogenation reaction is carried out largely regioselectively to obtain halogenation on the pyrazole moiety of the pyrazolotriazine scaffold towards XXII in which R1, R2, R4 and A have the meanings given above. Subsequently, more drastic or elevated chlorination conditions using NCS at higher temperature results in the building block XXIII in which R1, R2, R4 and A have the meanings given above.

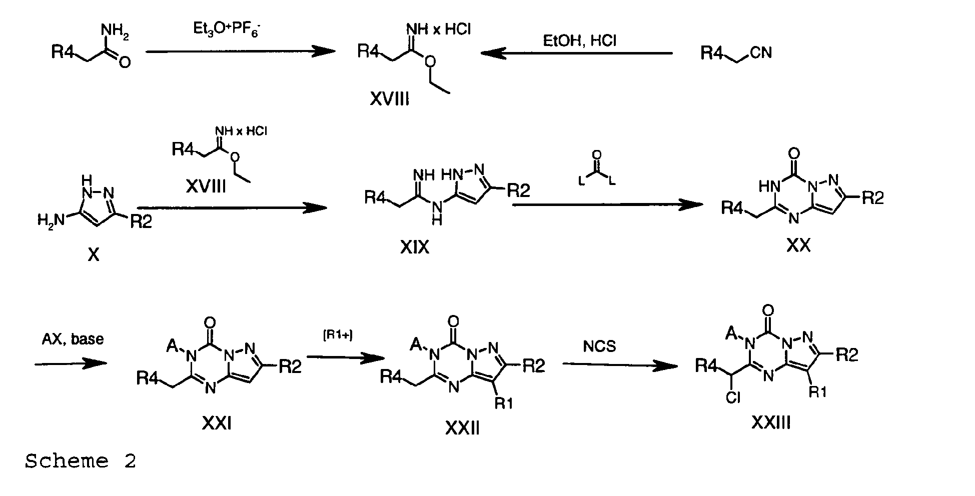

The building block X with R2 having the meaning above and in particular alkoxy and haloalkoxy, is prepared from hydroxyaminopyrazole as starting material, which is protected at the amine position as a phthalimido group. As shown in scheme 3, hydroxyaminopyrazole is protected as phathalimide to compound XXIV. The hydroxy-group of XXIV is subsequently transferred to the compound XXV with R2 is 1-4C-alkoxy or 1-2C-haloalkoxy. This is performed using either 1-4C-alkyl-L or 1-2C-haloalkyl-L with L is a suitable leaving group like halogen, carboxylate, diazo or an alcohol under Mitsunobu conditions (conditions with a phosphine as triphenylphosphine and an azodicarboxylate, like diethyl azodicarboxylate).

Deprotection of the phthalimido protection group is performed with hydrazine solution, hydrazine hydrate or other conditions which are habitual for the skilled person or as described in the following examples or analogously or similarly thereto.

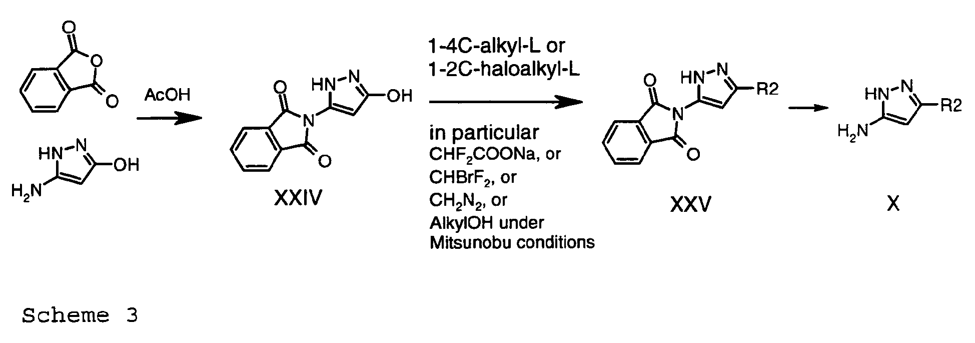

Alternatively the pyrazolotriazine heterocyclic core structure is prepared according to scheme 4. Starting from an appropriate aldehyde, the corresponding cyanhydrine XXVI in which R4 has the meaning given above is prepared under appropriate conditions with cyanide and weak base. Subsequently, the corresponding imidoester hydrochloride XXVII is obtained by reaction analogously to above with HCl in ethanol. XXVII is then treated with the substituted aminopyrazole X under basic conditions to the compound XXVIII in which R2 and R4 have the meanings given above. The cyclization reaction is performed with CO-reactive agent, like diethylcarbonate or carbonyldimidazole to obtain the pyrazolotriazinone heterocycle XXIX in which R2 and R4 have the meanings given above.

Compounds of formula XXIX are benzylated with compounds of formula A-X, in which A has the meanings given above and X is a suitable leaving group (e.g. halogen or the like), in a standard manner or as described exemplarily in the following examples to give corresponding compounds of formula XXX in which R2, R4 and A have the meanings given above. The hydroxy group is then transferred to a bromide leaving group by typical methods, like treating with CBr4 and triphenylphsophine towards XXXI in which R2, R4 and A have the meanings given above. Similarly, compounds XVI can be transformed to XXXII in which R1, R2, R4 and A have the meanings given above using typical methods, like CBr4 and triphenylphsophine.

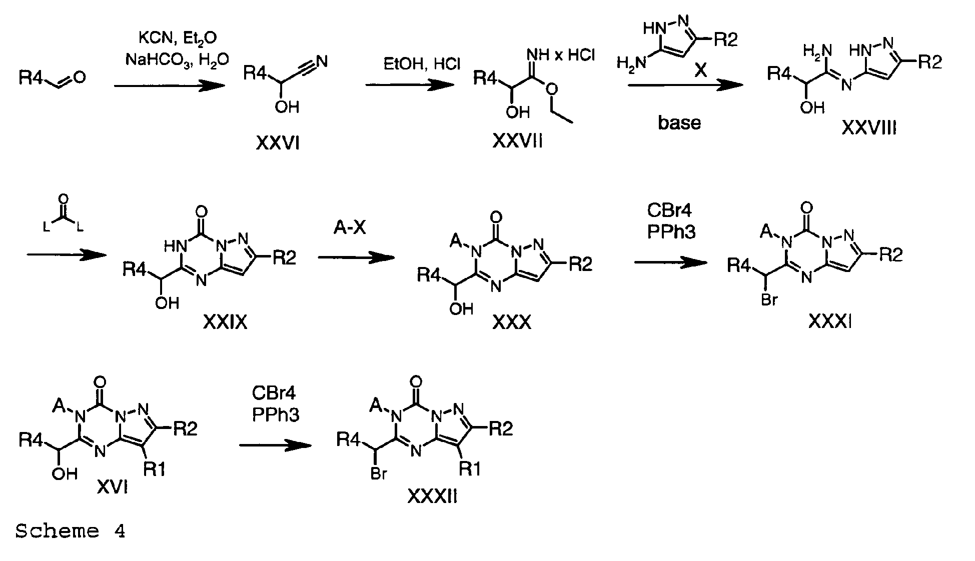

According to scheme 5 compounds of formula XVII, in which R1, A, R2 and R4 have the meanings given above, are subjected to a reductive amination reaction with amines of formula H₂N-(CH₂)ₙ-N(R5)R6, in which n has the meanings indicated above and R5 and R6 stand for a suitable temporary protective group PG1 or for the meanings given above with the exception that R5 and R6 are not hydrogen, to afford corresponding compounds of formula XXXIII in a manner which is habitual for the skilled person or which is described in the following examples. In more detail, in a first step of the reductive amination reaction the reaction partners are reacted in the presence of a suitable Lewis acid, e.g. a suitable titanium reagent like titanium(IV)isopropoxide, to give the corresponding imine compounds, which are reduced in a second step, such as with the aid of a suitable reducing reagent, e.g. a suitable hydride like a borohydride (e.g. NaCNBH₃, NaBH₄ or Na(OAc)₃BH) or the like, Raney-Ni or in the presence of hydrogen/transition metal catalyst (e.g. H₂/Pd).

PG1 stands for a suitable temporary protective group for the amino group, for example tert-butoxycarbonyl, benzyloxycarbonyl or 4-methoxy-benzyloxycarbonyl or one of those art-known protective groups mentioned in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

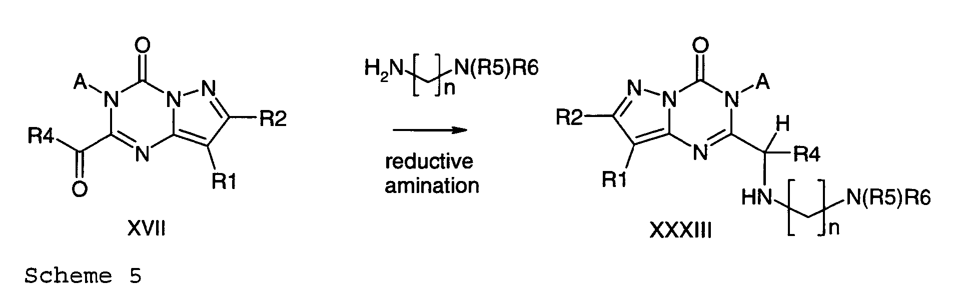

Furthermore compounds of formula XXXIII in which R1, A, R2, R5, R6 and R4 have the meanings given above can be prepared according to scheme 6 starting from XXIII or XXXII by treating with an azide salt like sodium azide in an aprotic polar solvent like DMF. Subsequently, the azido-derivative XXXIV is reduced under reductive conditions, like Staudinger conditions with phosphine and water or with Raney-Ni or in the presence of hydrogen/transition metal catalyst (e.g. H₂/Pd).

The resulting amine XXXV in which R1, A, R2 and R4 have the meanings given above, is subjected to a reductive amination reaction with an aldehyde of formula CHO-(CH₂)ₙ-N(R5)R6, in which n has the meanings indicated above and R5 and R6 stand for a suitable temporary protective group PG1 or for the meanings given above with the exception that R5 and R6 are not hydrogen, to afford corresponding compounds of formula XXXIII in a manner which is habitual for the skilled person or which is described in the following examples. In more detail, in a first step of the reductive amination reaction the reaction partners are reacted in the presence of a suitable Lewis acid, e.g. a suitable titanium reagent like titanium(IV)isopropoxide, to give the corresponding imine compounds, which are reduced in a second step, such as with the aid of a suitable reducing reagent, e.g. a suitable hydride like a borohydride (e.g. NaCNBH₃, NaBH₄ or Na(OAc)₃BH) or the like, Raney-Ni or in the presence of hydrogen/transition metal catalyst (e.g. H₂/Pd).

PG1 stands for a suitable temporary protective group for the amino group, for example tert-butoxycarbonyl, benzyloxycarbonyl or 4-methoxy-benzyloxycarbonyl or one of those art-known protective groups.

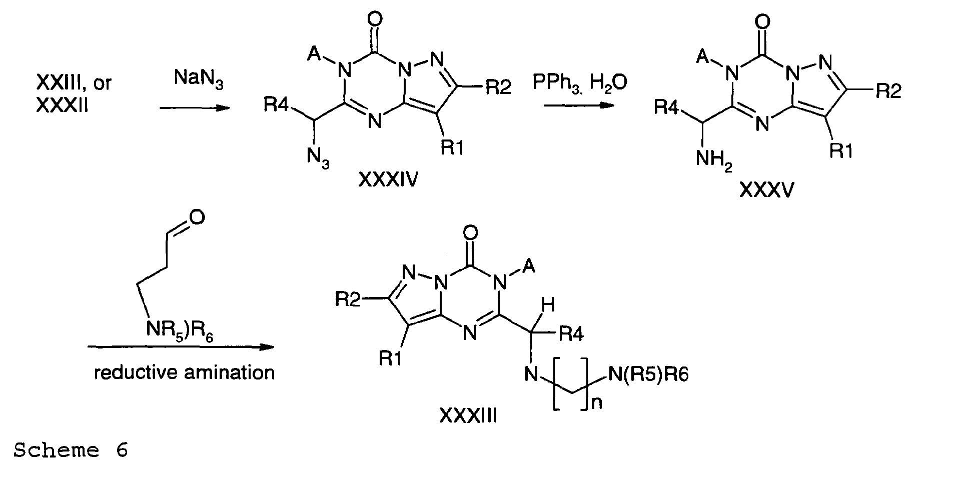

According to scheme 7 compounds of formula XXXIII, in which R1, A, n, R2 and R4 have the meanings given above and R5 and R6 stand for a suitable temporary protective group PG1 as defined above or for the meanings given above with the exception that R5 and R6 are not hydrogen, are benzoylated with compounds of formula B-C(O)-X2, in which B has the meanings given above and X2 is a suitable leaving group (e.g. chlorine), to give corresponding compounds of formula I.

Compounds of formula I, in which R1, A, n, B, R2 and R4 have the meanings given above and R5 and R6 stand for a suitable temporary protective group PG1 as defined above or for the meanings given above with the exception that R5 and R6 are not hydrogen, can be halogenated, particularly brominated, using a suitable halogenating reagent (for bromination e.g. NBS) under appropriate conditions to obtain corresponding further compounds of formula I, in which R1 is halogen. Said halogenation reaction can be carried out according to customary procedures, such as those mentioned above, or as described in the following examples, or analogously or similarly thereto.

Compounds of formula I, in which R1, A, n, B, R2 and R4 have the meanings given above and R5 and R6 stand for a suitable temporary protective group PG1 as defined above, are deprotected by removal of said protecting group PG1 in a manner known to the person skilled in the art or as described in the following examples to give corresponding deprotected compounds of formula I.

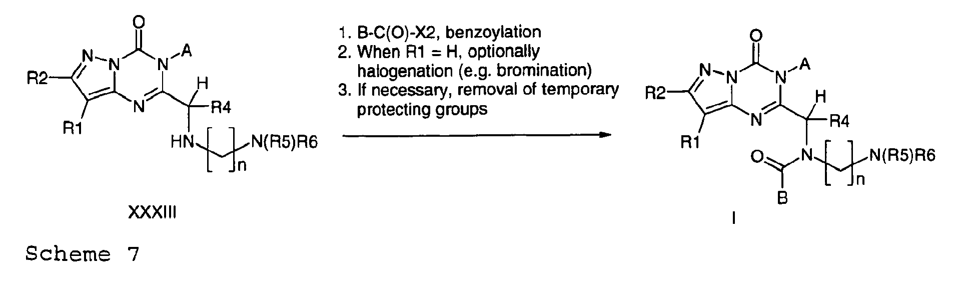

Further derivatives falling under the scope of the compounds of formula I according to this invention may also be prepared by the above processes and/or by processes known to the person skilled in the art.

Pure diastereomers and pure enantiomers of the compounds and salts according to the invention may be prepared by processes known to the person skilled in the art. Preferably, they are obtained by asymmetric synthesis, by using chiral starting compounds in synthesis or by splitting up enantiomeric and diastereomeric mixtures obtained in synthesis. In asymmetric synthesis, chiral synthons or chiral reagents are used as starting material.

Diastereomeric and/or enantiomeric compounds may be separated at an appropriate stage in the preparation, e.g. at the stage of an intermediate.

Enantiomeric and diastereomeric mixtures can be split up into the pure enantiomers and pure diastereomers by methods known to a person skilled in the art.

Preferably, diastereomeric mixtures are separated by crystallization, in particular fractional crystallization, or chromatography. A preferred method for the separation of diastereomeric mixtures is the crystallization. Another preferred method for the separation of diastereomeric mixtures is the separation using chromatography.

Enantiomeric mixtures can preferably be separated e.g. by forming diastereomers with a chiral auxiliary agent, resolving the diastereomers obtained and removing the chiral auxiliary agent. A preferred method of separation is the diastereomeric salt formation of the racemic compounds with optically active auxiliary agents. For example, enatiomer separation may be carried out at the stage of the compounds of formula I or at the stage of the starting compounds having a protonatable group, e.g. a free amino group, such as the starting compounds of formula XXXIII. As chiral auxiliary agents, for example, chiral acids (such as e.g. those mentioned below) can be used to separate enantiomeric bases and chiral bases can be used to separate enantiomeric acids via formation of diastereomeric salts. Preferred chiral acids are mandelic acid, tartaric acid, O,O'-dibenzoyltartaric acid, camphoric acid, quinic acid, glutamic acid, pyroglutamic acid, malic acid, camphorsulfonic acid, 3-bromocamphorsulfonic acid, α-methoxyphenylacetic acid, α-methoxy-α-trifluoromethylphenylacetic acid and 2-phenylpropionic acid.

Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures.

Another suitable method for the isolation of enantiomers is the enzymatic separation, e.g. using a suitable lipase. Other methods include the kinetic resolution of a racemate, enantioselective (preferential) crystallization (or crystallization by entrainment) from a conglomerate of enantiomorphous crystals under suitable conditions; or by (fractional) crystallization from a suitable solvent in the presence of a chiral auxiliary.

A preferred method for the separation of enantiomeric mixtures is the crystallization or the separation using chiral separating columns in chromatography.

Enantiomerically pure starting compounds according to the invention may be obtained as described above for the synthesis or separation of enantiomers and diastereomers of the compounds of formula I.

When one of the final steps or purification is carried out under the presence of an inorganic or organic acid (e.g. hydrochloric, trifluoroacetic, acetic or formic acid or the like), the compounds of formula I may be obtained - depending on their individual chemical nature and the individual nature of the acid used - as free base or containing said acid in an stoechiometric or non-stoechiometric quantity. The amount of the acid contained can be determined according to art-known procedures, e.g. by titration or NMR.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The substances according to the invention are isolated and purified in a manner known per se, for example by distilling off the solvent under reduced pressure and recrystallizing the residue obtained from a suitable solvent or subjecting it to one of the customary purification methods, such as, for example, column chromatography on a suitable support material.

Optionally, compounds of the formula I can be converted into their salts, or, optionally, salts of the compounds of the formula I can be converted into the free compounds.

Salts of the compounds of formula I according to the invention can be obtained by dissolving the free compound in a suitable solvent (for example a ketone such as acetone, methylethylketone or methylisobutylketone, an ether such as diethyl ether, diisopropyl ether, tetrahydrofuran or dioxane, a chlorinated hydrocarbon such as methylene chloride or chloroform, a low molecular weight aliphatic alcohol such as methanol, ethanol or isopropanol, or an ester, such as ethyl acetate) which contains the desired acid or base, or to which the desired acid or base is then added, if necessary upon heating.

The acid or base can be employed in salt preparation, depending on whether a mono- or polybasic acid or base is concerned and depending on which salt is desired, in an equimolar quantitative ratio or one differing therefrom.

The salts are obtained for example by evaporating the solvent, by re-precipitating or by precipitating upon cooling or by precipitating with a non-solvent for the salt and separation, for example by filtration, of the salt after precipitation.

Salts obtained can be converted into the free compounds which, in turn, can be converted into salts. In this manner, pharmaceutically unacceptable salts, which can be obtained, for example, as process products in the production of the compounds, or a salt, stereoisomer or a salt of a stereoisomer thereof, according to the invention on an industrial scale or in the isolation or purification of compounds of formula I, can be converted into pharmaceutically acceptable salts by processes known to the person skilled in the art.

In the salt preparation, the acids or bases are employed in an equimolar ratio or in a ratio differing therefrom, depending on the acid or base concerned, e.g. whether the acid is a mono- or polybasic acid, and on which salt is desired.

Thus, the present invention also relates to processes disclosed herein for preparing compounds according to this invention, which processes comprise one or more steps of converting and/or reacting the mentioned intermediates with the appropriate reaction partners under conditions as disclosed herein. The present invention also relates to intermediates (including their salts, stereoisomers and salts of these stereoisomers), methods and processes, which are disclosed herein and which are useful in synthesizing compounds according to this invention.

Further special embodiments (embodiments 33 to 92) of the compounds of formula I according to this invention refer to those compounds of formula I and their salts, stereoisomers and salts of stereoisomers, in which
R1 is bromine,
R2 is methyl, methoxy, difluoromethoxy or hydrogen, and
A, R4, n, R5 and R6 have the respective meanings indicated in Table 1 given below.

**Table 1:**

| **embodiment** | **A** | **R4** | **n** | **R5** | **R6** |
|---|---|---|---|---|---|
| 33 | benzyl | ethyl | 2 | H | H |
| 34 | 3-fluorobenzyl | ethyl | 2 | H | H |
| 35 | 4-fluorobenzyl | ethyl | 2 | H | H |
| 36 | benzyl | isopropyl | 2 | H | H |
| 37 | 3-fluorobenzyl | isopropyl | 2 | H | H |
| 38 | 4-fluorobenzyl | isopropyl | 2 | H | H |
| 39 | benzyl | sec-butyl | 2 | H | H |
| 40 | 3-fluorobenzyl | sec-butyl | 2 | H | H |
| 41 | 4-fluorobenzyl | sec-butyl | 2 | H | H |
| 42 | benzyl | cyclopropyl | 2 | H | H |
| 43 | 3-fluorobenzyl | cyclopropyl | 2 | H | H |
| 44 | 4-fluorobenzyl | cyclopropyl | 2 | H | H |
| 45 | benzyl | n-propyl | 2 | H | H |
| 46 | 3-fluorobenzyl | n-propyl | 2 | H | H |
| 47 | 4-fluorobenzyl | n-propyl | 2 | H | H |
| 48 | benzyl | ethyl | 3 | H | H |
| 49 | 3-fluorobenzyl | ethyl | 3 | H | H |
| 50 | 4-fluorobenzyl | ethyl | 3 | H | H |
| 51 | benzyl | isopropyl | 3 | H | H |
| 52 | 3-fluorobenzyl | isopropyl | 3 | H | H |
| 53 | 4-fluorobenzyl | isopropyl | 3 | H | H |
| 54 | benzyl | sec-butyl | 3 | H | H |
| 55 | 3-fluorobenzyl | sec-butyl | 3 | H | H |
| 56 | 4-fluorobenzyl | sec-butyl | 3 | H | H |
| 57 | benzyl | cyclopropyl | 3 | H | H |
| 58 | 3-fluorobenzyl | cyclopropyl | 3 | H | H |
| 59 | 4-fluorobenzyl | cyclopropyl | 3 | H | H |
| 60 | benzyl | n-propyl | 3 | H | H |
| 61 | 3-fluorobenzyl | n-propyl | 3 | H | H |
| 62 | 4-fluorobenzyl | n-propyl | 3 | H | H |
| 63 | benzyl | ethyl | 2 | methyl | methyl |
| 64 | 3-fluorobenzyl | ethyl | 2 | methyl | methyl |
| 65 | 4-fluorobenzyl | ethyl | 2 | methyl | methyl |
| 66 | benzyl | isopropyl | 2 | methyl | methyl |
| 67 | 3-fluorobenzyl | isopropyl | 2 | methyl | methyl |
| 68 | 4-fluorobenzyl | isopropyl | 2 | methyl | methyl |
| 69 | benzyl | sec-butyl | 2 | methyl | methyl |
| 70 | 3-fluorobenzyl | sec-butyl | 2 | methyl | methyl |
| 71 | 4-fluorobenzyl | sec-butyl | 2 | methyl | methyl |
| 72 | benzyl | cyclopropyl | 2 | methyl | methyl |
| 73 | 3-fluorobenzyl | cyclopropyl | 2 | methyl | methyl |
| 74 | 4-fluorobenzyl | cyclopropyl | 2 | methyl | methyl |
| 75 | benzyl | n-propyl | 2 | methyl | methyl |
| 76 | 3-fluorobenzyl | n-propyl | 2 | methyl | methyl |
| 77 | 4-fluorobenzyl | n-propyl | 2 | methyl | methyl |
| 78 | benzyl | ethyl | 3 | methyl | methyl |
| 79 | 3-fluorobenzyl | ethyl | 3 | methyl | methyl |
| 80 | 4-fluorobenzyl | ethyl | 3 | methyl | methyl |
| 81 | benzyl | isopropyl | 3 | methyl | methyl |
| 82 | 3-fluorobenzyl | isopropyl | 3 | methyl | methyl |
| 83 | 4-fluorobenzyl | isopropyl | 3 | methyl | methyl |
| 84 | benzyl | sec-butyl | 3 | methyl | methyl |
| 85 | 3-fluorobenzyl | sec-butyl | 3 | methyl | methyl |
| 86 | 4-fluorobenzyl | sec-butyl | 3 | methyl | methyl |
| 87 | benzyl | cyclopropyl | 3 | methyl | methyl |
| 88 | 3-fluorobenzyl | cyclopropyl | 3 | methyl | methyl |
| 89 | 4-fluorobenzyl | cyclopropyl | 3 | methyl | methyl |
| 90 | benzyl | n-propyl | 3 | methyl | methyl |
| 91 | 3-fluorobenzyl | n-propyl | 3 | methyl | methyl |
| 92 | 4-fluorobenzyl | n-propyl | 3 | methyl | methyl |

Additional further special embodiments (embodiments 93 to 152) of the compounds of formula I according to this invention refer to those compounds of formula I and their salts, stereoisomers and salts of stereoisomers, in which
R1 is chlorine,
R2 is methyl, methoxy, difluoromethoxy or hydrogen, and
A, R4, n, R5 and R6 have the respective meanings indicated in Table 2 given below.

**Table 2:**

| **embodiment** | **A** | **R4** | **n** | **R5** | **R6** |
|---|---|---|---|---|---|
| 93 | benzyl | ethyl | 2 | H | H |
| 94 | 3-fluorobenzyl | ethyl | 2 | H | H |
| 95 | 4-fluorobenzyl | ethyl | 2 | H | H |
| 96 | benzyl | isopropyl | 2 | H | H |
| 97 | 3-fluorobenzyl | isopropyl | 2 | H | H |
| 98 | 4-fluorobenzyl | isopropyl | 2 | H | H |
| 99 | benzyl | sec-butyl | 2 | H | H |
| 100 | 3-fluorobenzyl | sec-butyl | 2 | H | H |
| 101 | 4-fluorobenzyl | sec-butyl | 2 | H | H |
| 102 | benzyl | cyclopropyl | 2 | H | H |
| 103 | 3-fluorobenzyl | cyclopropyl | 2 | H | H |
| 104 | 4-fluorobenzyl | cyclopropyl | 2 | H | H |
| 105 | benzyl | n-propyl | 2 | H | H |
| 106 | 3-fluorobenzyl | n-propyl | 2 | H | H |
| 107 | 4-fluorobenzyl | n-propyl | 2 | H | H |
| 108 | benzyl | ethyl | 3 | H | H |
| 109 | 3-fluorobenzyl | ethyl | 3 | H | H |
| 110 | 4-fluorobenzyl | ethyl | 3 | H | H |
| 111 | benzyl | isopropyl | 3 | H | H |
| 112 | 3-fluorobenzyl | isopropyl | 3 | H | H |
| 113 | 4-fluorobenzyl | isopropyl | 3 | H | H |
| 114 | benzyl | sec-butyl | 3 | H | H |
| 115 | 3-fluorobenzyl | sec-butyl | 3 | H | H |
| 116 | 4-fluorobenzyl | sec-butyl | 3 | H | H |
| 117 | benzyl | cyclopropyl | 3 | H | H |
| 118 | 3-fluorobenzyl | cyclopropyl | 3 | H | H |
| 119 | 4-fluorobenzyl | cyclopropyl | 3 | H | H |
| 120 | benzyl | n-propyl | 3 | H | H |
| 121 | 3-fluorobenzyl | n-propyl | 3 | H | H |
| 122 | 4-fluorobenzyl | n-propyl | 3 | H | H |
| 123 | benzyl | ethyl | 2 | methyl | methyl |
| 124 | 3-fluorobenzyl | ethyl | 2 | methyl | methyl |
| 125 | 4-fluorobenzyl | ethyl | 2 | methyl | methyl |
| 126 | benzyl | isopropyl | 2 | methyl | methyl |
| 127 | 3-fluorobenzyl | isopropyl | 2 | methyl | methyl |
| 128 | 4-fluorobenzyl | isopropyl | 2 | methyl | methyl |
| 129 | benzyl | sec-butyl | 2 | methyl | methyl |
| 130 | 3-fluorobenzyl | sec-butyl | 2 | methyl | methyl |
| 131 | 4-fluorobenzyl | sec-butyl | 2 | methyl | methyl |
| 132 | benzyl | cyclopropyl | 2 | methyl | methyl |
| 133 | 3-fluorobenzyl | cyclopropyl | 2 | methyl | methyl |
| 134 | 4-fluorobenzyl | cyclopropyl | 2 | methyl | methyl |
| 135 | benzyl | n-propyl | 2 | methyl | methyl |
| 136 | 3-fluorobenzyl | n-propyl | 2 | methyl | methyl |
| 137 | 4-fluorobenzyl | n-propyl | 2 | methyl | methyl |
| 138 | benzyl | ethyl | 3 | methyl | methyl |
| 139 | 3-fluorobenzyl | ethyl | 3 | methyl | methyl |
| 140 | 4-fluorobenzyl | ethyl | 3 | methyl | methyl |
| 141 | benzyl | isopropyl | 3 | methyl | methyl |
| 142 | 3-fluorobenzyl | isopropyl | 3 | methyl | methyl |
| 143 | 4-fluorobenzyl | isopropyl | 3 | methyl | methyl |
| 144 | benzyl | sec-butyl | 3 | methyl | methyl |
| 145 | 3-fluorobenzyl | sec-butyl | 3 | methyl | methyl |
| 146 | 4-fluorobenzyl | sec-butyl | 3 | methyl | methyl |
| 147 | benzyl | cyclopropyl | 3 | methyl | methyl |
| 148 | 3-fluorobenzyl | cyclopropyl | 3 | methyl | methyl |
| 149 | 4-fluorobenzyl | cyclopropyl | 3 | methyl | methyl |
| 150 | benzyl | n-propyl | 3 | methyl | methyl |
| 151 | 3-fluorobenzyl | n-propyl | 3 | methyl | methyl |
| 152 | 4-fluorobenzyl | n-propyl | 3 | methyl | methyl |

It is to be understood that the present invention includes any or all possible combinations and subsets of the special embodiments defined hereinabove.

Exemplary compounds according to this invention are the following compounds of formula I* and their salts,
in which
R1 is bromine
R2 is methyl, methoxy, difluoromethoxy or hydrogen, and
A, R4, n, R5, R6 and B have the respective meanings indicated in Table 3 given below.

Further exemplary compounds according to this invention are the following compounds of formula I* and their salts,
in which
R1 is chlorine,
R2 is methyl, methoxy, difluoromethoxy or hydrogen, and
A, R4, n, R5, R6 and B have the respective meanings indicated in Table 3 given below.

Further exemplary compounds according to this invention are the following compounds of formula I* and their salts,
in which
R1 is hydrogen,
R2 is methyl, methoxy, difluoromethoxy or hydrogen, and
A, R4, n, R5, R6 and B have the respective meanings indicated in Table 3 given below.

Further exemplary compounds according to this invention are the following compounds of formula I** and their salts,
in which
R1 is bromine
R2 is methyl, methoxy, difluoromethoxy or hydrogen, and
A, R4, n, R5, R6 and B have the respective meanings indicated in Table 3 given below.

Further exemplary compounds according to this invention are the following compounds of formula I** and their salts,
in which
R1 is chlorine,
R2 is methyl, methoxy, difluoromethoxy or hydrogen, and
A, R4, n, R5, R6 and B have the respective meanings indicated in Table 3 given below.

Further exemplary compounds according to this invention are the following compounds of formula I** and their salts,
in which
R1 is hydrogen,
R2 is methyl, methoxy, difluoromethoxy or hydrogen, and
A, R4, n, R5, R6 and B have the respective meanings indicated in Table 3 given below.

**Table 3:**

| | **A** | **R4** | **n** | **R5** | **R6** | **B** |
|---|---|---|---|---|---|---|
| 1) | benzyl | ethyl | 2 | H | H | 4-methylphenyl |
| 2) | benzyl | ethyl | 2 | H | H | 4-bromophenyl |
| 3) | benzyl | ethyl | 2 | H | H | 4-chlorophenyl |
| 4) | benzyl | ethyl | 2 | H | H | 4-methyl-3-fluorophenyl |
| 5) | benzyl | ethyl | 2 | H | H | 4-fluorophenyl |
| 6) | benzyl | ethyl | 2 | H | H | 4-trifluoromethylphenyl |
| 7) | benzyl | ethyl | 2 | H | H | 4-methoxyphenyl |
| 8) | benzyl | ethyl | 3 | H | H | 4-methylphenyl |
| 9) | benzyl | ethyl | 3 | H | H | 4-bromophenyl |
| 10) | benzyl | ethyl | 3 | H | H | 4-chlorophenyl |
| 11) | benzyl | ethyl | 3 | H | H | 4-methyl-3-fluorophenyl |
| 12) | benzyl | ethyl | 3 | H | H | 4-fluorophenyl |
| 13) | benzyl | ethyl | 3 | H | H | 4-trifluoromethylphenyl |
| 14) | benzyl | ethyl | 3 | H | H | 4-methoxyphenyl |
| 15) | benzyl | ethyl | 2 | methyl | methyl | 4-methylphenyl |
| 16) | benzyl | ethyl | 2 | methyl | methyl | 4-bromophenyl |
| 17) | benzyl | ethyl | 2 | methyl | methyl | 4-chlorophenyl |
| 18) | benzyl | ethyl | 2 | methyl | methyl | 4-methyl-3-fluorophenyl |
| 19) | benzyl | ethyl | 2 | methyl | methyl | 4-fluorophenyl |
| 20) | benzyl | ethyl | 2 | methyl | methyl | 4-trifluoromethylphenyl |
| 21) | benzyl | ethyl | 2 | methyl | methyl | 4-methoxyphenyl |
| 22) | benzyl | ethyl | 3 | methyl | methyl | 4-methylphenyl |
| 23) | benzyl | ethyl | 3 | methyl | methyl | 4-bromophenyl |
| 24) | benzyl | ethyl | 3 | methyl | methyl | 4-chlorophenyl |
| 25) | benzyl | ethyl | 3 | methyl | methyl | 4-methyl-3-fluorophenyl |
| 26) | benzyl | ethyl | 3 | methyl | methyl | 4-fluorophenyl |
| 27) | benzyl | ethyl | 3 | methyl | methyl | 4-trifluoromethylphenyl |
| 28) | benzyl | ethyl | 3 | methyl | methyl | 4-methoxyphenyl |
| 29) | 3-fluorobenzyl | ethyl | 2 | H | H | 4-methylphenyl |
| 30) | 3-fluorobenzyl | ethyl | 2 | H | H | 4-bromophenyl |
| 31) | 3-fluorobenzyl | ethyl | 2 | H | H | 4-chlorophenyl |
| 32) | 3-fluorobenzyl | ethyl | 2 | H | H | 4-methyl-3-fluorophenyl |
| 33) | 3-fluorobenzyl | ethyl | 2 | H | H | 4-fluorophenyl |
| 34) | 3-fluorobenzyl | ethyl | 2 | H | H | 4-trifluoromethylphenyl |
| 35) | 3-fluorobenzyl | ethyl | 2 | H | H | 4-methoxyphenyl |
| 36) | 3-fluorobenzyl | ethyl | 3 | H | H | 4-methylphenyl |
| 37) | 3-fluorobenzyl | ethyl | 3 | H | H | 4-bromophenyl |
| 38) | 3-fluorobenzyl | ethyl | 3 | H | H | 4-chlorophenyl |
| 39) | 3-fluorobenzyl | ethyl | 3 | H | H | 4-methyl-3-fluorophenyl |
| 40) | 3-fluorobenzyl | ethyl | 3 | H | H | 4-fluorophenyl |
| 41) | 3-fluorobenzyl | ethyl | 3 | H | H | 4-trifluoromethylphenyl |
| 42) | 3-fluorobenzyl | ethyl | 3 | H | H | 4-methoxyphenyl |
| 43) | 3-fluorobenzyl | ethyl | 2 | methyl | methyl | 4-methylphenyl |
| 44) | 3-fluorobenzyl | ethyl | 2 | methyl | methyl | 4-bromophenyl |
| 45) | 3-fluorobenzyl | ethyl | 2 | methyl | methyl | 4-chlorophenyl |
| 46) | 3-fluorobenzyl | ethyl | 2 | methyl | methyl | 4-methyl-3-fluorophenyl |
| 47) | 3-fluorobenzyl | ethyl | 2 | methyl | methyl | 4-fluorophenyl |
| 48) | 3-fluorobenzyl | ethyl | 2 | methyl | methyl | 4-trifluoromethylphenyl |
| 49) | 3-fluorobenzyl | ethyl | 2 | methyl | methyl | 4-methoxyphenyl |
| 50) | 3-fluorobenzyl | ethyl | 3 | methyl | methyl | 4-methylphenyl |
| 51) | 3-fluorobenzyl | ethyl | 3 | methyl | methyl | 4-bromophenyl |
| 52) | 3-fluorobenzyl | ethyl | 3 | methyl | methyl | 4-chlorophenyl |
| 53) | 3-fluorobenzyl | ethyl | 3 | methyl | methyl | 4-methyl-3-fluorophenyl |
| 54) | 3-fluorobenzyl | ethyl | 3 | methyl | methyl | 4-fluorophenyl |
| 55) | 3-fluorobenzyl | ethyl | 3 | methyl | methyl | 4-trifluoromethylphenyl |
| 56) | 3-fluorobenzyl | ethyl | 3 | methyl | methyl | 4-methoxyphenyl |
| 57) | 4-fluorobenzyl | ethyl | 2 | H | H | 4-methylphenyl |
| 58) | 4-fluorobenzyl | ethyl | 2 | H | H | 4-bromophenyl |
| 59) | 4-fluorobenzyl | ethyl | 2 | H | H | 4-chlorophenyl |
| 60) | 4-fluorobenzyl | ethyl | 2 | H | H | 4-methyl-3-fluorophenyl |
| 61) | 4-fluorobenzyl | ethyl | 2 | H | H | 4-fluorophenyl |
| 62) | 4-fluorobenzyl | ethyl | 2 | H | H | 4-trifluoromethylphenyl |
| 63) | 4-fluorobenzyl | ethyl | 2 | H | H | 4-methoxyphenyl |
| 64) | 4-fluorobenzyl | ethyl | 3 | H | H | 4-methylphenyl |
| 65) | 4-fluorobenzyl | ethyl | 3 | H | H | 4-bromophenyl |
| 66) | 4-fluorobenzyl | ethyl | 3 | H | H | 4-chlorophenyl |
| 67) | 4-fluorobenzyl | ethyl | 3 | H | H | 4-methyl-3-fluorophenyl |
| 68) | 4-fluorobenzyl | ethyl | 3 | H | H | 4-fluorophenyl |
| 69) | 4-fluorobenzyl | ethyl | 3 | H | H | 4-trifluoromethylphenyl |
| 70) | 4-fluorobenzyl | ethyl | 3 | H | H | 4-methoxyphenyl |
| 71) | 4-fluorobenzyl | ethyl | 2 | methyl | methyl | 4-methylphenyl |
| 72) | 4-fluorobenzyl | ethyl | 2 | methyl | methyl | 4-bromophenyl |
| 73) | 4-fluorobenzyl | ethyl | 2 | methyl | methyl | 4-chlorophenyl |
| 74) | 4-fluorobenzyl | ethyl | 2 | methyl | methyl | 4-methyl-3-fluorophenyl |
| 75) | 4-fluorobenzyl | ethyl | 2 | methyl | methyl | 4-fluorophenyl |
| 76) | 4-fluorobenzyl | ethyl | 2 | methyl | methyl | 4-trifluoromethylphenyl |
| 77) | 4-fluorobenzyl | ethyl | 2 | methyl | methyl | 4-methoxyphenyl |
| 78) | 4-fluorobenzyl | ethyl | 3 | methyl | methyl | 4-methylphenyl |
| 79) | 4-fluorobenzyl | ethyl | 3 | methyl | methyl | 4-bromophenyl |
| 80) | 4-fluorobenzyl | ethyl | 3 | methyl | methyl | 4-chlorophenyl |
| 81) | 4-fluorobenzyl | ethyl | 3 | methyl | methyl | 4-methyl-3-fluorophenyl |
| 82) | 4-fluorobenzyl | ethyl | 3 | methyl | methyl | 4-fluorophenyl |
| 83) | 4-fluorobenzyl | ethyl | 3 | methyl | methyl | 4-trifluoromethylphenyl |
| 84) | 4-fluorobenzyl | ethyl | 3 | methyl | methyl | 4-methoxyphenyl |
| 85) | benzyl | isopropyl | 2 | H | H | 4-methylphenyl |
| 86) | benzyl | isopropyl | 2 | H | H | 4-bromophenyl |
| 87) | benzyl | isopropyl | 2 | H | H | 4-chlorophenyl |
| 88) | benzyl | isopropyl | 2 | H | H | 4-methyl-3-fluorophenyl |
| 89) | benzyl | isopropyl | 2 | H | H | 4-fluorophenyl |
| 90) | benzyl | isopropyl | 2 | H | H | 4-trifluoromethylphenyl |
| 91) | benzyl | isopropyl | 2 | H | H | 4-methoxyphenyl |
| 92) | benzyl | isopropyl | 3 | H | H | 4-methylphenyl |
| 93) | benzyl | isopropyl | 3 | H | H | 4-bromophenyl |
| 94) | benzyl | isopropyl | 3 | H | H | 4-chlorophenyl |
| 95) | benzyl | isopropyl | 3 | H | H | 4-methyl-3-fluorophenyl |
| 96) | benzyl | isopropyl | 3 | H | H | 4-fluorophenyl |
| 97) | benzyl | isopropyl | 3 | H | H | 4-trifluoromethylphenyl |
| 98) | benzyl | isopropyl | 3 | H | H | 4-methoxyphenyl |
| 99) | benzyl | isopropyl | 2 | methyl | methyl | 4-methylphenyl |
| 100) | benzyl | isopropyl | 2 | methyl | methyl | 4-bromophenyl |
| 101) | benzyl | isopropyl | 2 | methyl | methyl | 4-chlorophenyl |
| 102) | benzyl | isopropyl | 2 | methyl | methyl | 4-methyl-3-fluorophenyl |
| 103) | benzyl | isopropyl | 2 | methyl | methyl | 4-fluorophenyl |
| 104) | benzyl | isopropyl | 2 | methyl | methyl | 4-trifluoromethylphenyl |
| 105) | benzyl | isopropyl | 2 | methyl | methyl | 4-methoxyphenyl |
| 106) | benzyl | isopropyl | 3 | methyl | methyl | 4-methylphenyl |
| 107) | benzyl | isopropyl | 3 | methyl | methyl | 4-bromophenyl |
| 108) | benzyl | isopropyl | 3 | methyl | methyl | 4-chlorophenyl |
| 109) | benzyl | isopropyl | 3 | methyl | methyl | 4-methyl-3-fluorophenyl |
| 110) | benzyl | isopropyl | 3 | methyl | methyl | 4-fluorophenyl |
| 111) | benzyl | isopropyl | 3 | methyl | methyl | 4-trifluoromethylphenyl |
| 112) | benzyl | isopropyl | 3 | methyl | methyl | 4-methoxyphenyl |
| 113) | 3-fluorobenzyl | isopropyl | 2 | H | H | 4-methylphenyl |
| 114) | 3-fluorobenzyl | isopropyl | 2 | H | H | 4-bromophenyl |
| 115) | 3-fluorobenzyl | isopropyl | 2 | H | H | 4-chlorophenyl |
| 116) | 3-fluorobenzyl | isopropyl | 2 | H | H | 4-methyl-3-fluorophenyl |
| 117) | 3-fluorobenzyl | isopropyl | 2 | H | H | 4-fluorophenyl |
| 118) | 3-fluorobenzyl | isopropyl | 2 | H | H | 4-trifluoromethylphenyl |
| 119) | 3-fluorobenzyl | isopropyl | 2 | H | H | 4-methoxyphenyl |
| 120) | 3-fluorobenzyl | isopropyl | 3 | H | H | 4-methylphenyl |
| 121) | 3-fluorobenzyl | isopropyl | 3 | H | H | 4-bromophenyl |
| 122) | 3-fluorobenzyl | isopropyl | 3 | H | H | 4-chlorophenyl |
| 123) | 3-fluorobenzyl | isopropyl | 3 | H | H | 4-methyl-3-fluorophenyl |
| 124) | 3-fluorobenzyl | isopropyl | 3 | H | H | 4-fluorophenyl |
| 125) | 3-fluorobenzyl | isopropyl | 3 | H | H | 4-trifluoromethylphenyl |
| 126) | 3-fluorobenzyl | isopropyl | 3 | H | H | 4-methoxyphenyl |
| 127) | 3-fluorobenzyl | isopropyl | 2 | methyl | methyl | 4-methylphenyl |
| 128) | 3-fluorobenzyl | isopropyl | 2 | methyl | methyl | 4-bromophenyl |
| 129) | 3-fluorobenzyl | isopropyl | 2 | methyl | methyl | 4-chlorophenyl |
| 130) | 3-fluorobenzyl | isopropyl | 2 | methyl | methyl | 4-methyl-3-fluorophenyl |
| 131) | 3-fluorobenzyl | isopropyl | 2 | methyl | methyl | 4-fluorophenyl |
| 132) | 3-fluorobenzyl | isopropyl | 2 | methyl | methyl | 4-trifluoromethylphenyl |
| 133) | 3-fluorobenzyl | isopropyl | 2 | methyl | methyl | 4-methoxyphenyl |
| 134) | 3-fluorobenzyl | isopropyl | 3 | methyl | methyl | 4-methylphenyl |
| 135) | 3-fluorobenzyl | isopropyl | 3 | methyl | methyl | 4-bromophenyl |
| 136) | 3-fluorobenzyl | isopropyl | 3 | methyl | methyl | 4-chlorophenyl |
| 137) | 3-fluorobenzyl | isopropyl | 3 | methyl | methyl | 4-methyl-3-fluorophenyl |
| 138) | 3-fluorobenzyl | isopropyl | 3 | methyl | methyl | 4-fluorophenyl |
| 139) | 3-fluorobenzyl | isopropyl | 3 | methyl | methyl | 4-trifluoromethylphenyl |
| 140) | 3-fluorobenzyl | isopropyl | 3 | methyl | methyl | 4-methoxyphenyl |
| 141) | 4-fluorobenzyl | isopropyl | 2 | H | H | 4-methylphenyl |
| 142) | 4-fluorobenzyl | isopropyl | 2 | H | H | 4-bromophenyl |
| 143) | 4-fluorobenzyl | isopropyl | 2 | H | H | 4-chlorophenyl |
| 144) | 4-fluorobenzyl | isopropyl | 2 | H | H | 4-methyl-3-fluorophenyl |
| 145) | 4-fluorobenzyl | isopropyl | 2 | H | H | 4-fluorophenyl |
| 146) | 4-fluorobenzyl | isopropyl | 2 | H | H | 4-trifluoromethylphenyl |
| 147) | 4-fluorobenzyl | isopropyl | 2 | H | H | 4-methoxyphenyl |
| 148) | 4-fluorobenzyl | isopropyl | 3 | H | H | 4-methylphenyl |
| 149) | 4-fluorobenzyl | isopropyl | 3 | H | H | 4-bromophenyl |
| 150) | 4-fluorobenzyl | isopropyl | 3 | H | H | 4-chlorophenyl |
| 151) | 4-fluorobenzyl | isopropyl | 3 | H | H | 4-methyl-3-fluorophenyl |
| 152) | 4-fluorobenzyl | isopropyl | 3 | H | H | 4-fluorophenyl |
| 153) | 4-fluorobenzyl | isopropyl | 3 | H | H | 4-trifluoromethylphenyl |
| 154) | 4-fluorobenzyl | isopropyl | 3 | H | H | 4-methoxyphenyl |
| 155) | 4-fluorobenzyl | isopropyl | 2 | methyl | methyl | 4-methylphenyl |
| 156) | 4-fluorobenzyl | isopropyl | 2 | methyl | methyl | 4-bromophenyl |
| 157) | 4-fluorobenzyl | isopropyl | 2 | methyl | methyl | 4-chlorophenyl |
| 158) | 4-fluorobenzyl | isopropyl | 2 | methyl | methyl | 4-methyl-3-fluorophenyl |
| 159) | 4-fluorobenzyl | isopropyl | 2 | methyl | methyl | 4-fluorophenyl |
| 160) | 4-fluorobenzyl | isopropyl | 2 | methyl | methyl | 4-trifluoromethylphenyl |
| 161) | 4-fluorobenzyl | isopropyl | 2 | methyl | methyl | 4-methoxyphenyl |
| 162) | 4-fluorobenzyl | isopropyl | 3 | methyl | methyl | 4-methylphenyl |
| 163) | 4-fluorobenzyl | isopropyl | 3 | methyl | methyl | 4-bromophenyl |
| 164) | 4-fluorobenzyl | isopropyl | 3 | methyl | methyl | 4-chlorophenyl |
| 165) | 4-fluorobenzyl | isopropyl | 3 | methyl | methyl | 4-methyl-3-fluorophenyl |
| 166) | 4-fluorobenzyl | isopropyl | 3 | methyl | methyl | 4-fluorophenyl |
| 167) | 4-fluorobenzyl | isopropyl | 3 | methyl | methyl | 4-trifluoromethylphenyl |
| 168) | 4-fluorobenzyl | isopropyl | 3 | methyl | methyl | 4-methoxyphenyl |
| 169) | benzyl | cyclopropyl | 2 | H | H | 4-methylphenyl |
| 170) | benzyl | cyclopropyl | 2 | H | H | 4-bromophenyl |
| 171) | benzyl | cyclopropyl | 2 | H | H | 4-chlorophenyl |
| 172) | benzyl | cyclopropyl | 2 | H | H | 4-methyl-3-fluorophenyl |
| 173) | benzyl | cyclopropyl | 2 | H | H | 4-fluorophenyl |
| 174) | benzyl | cyclopropyl | 2 | H | H | 4-trifluoromethylphenyl |
| 175) | benzyl | cyclopropyl | 2 | H | H | 4-methoxyphenyl |
| 176) | benzyl | cyclopropyl | 3 | H | H | 4-methylphenyl |
| 177) | benzyl | cyclopropyl | 3 | H | H | 4-bromophenyl |
| 178) | benzyl | cyclopropyl | 3 | H | H | 4-chlorophenyl |
| 179) | benzyl | cyclopropyl | 3 | H | H | 4-methyl-3-fluorophenyl |
| 180) | benzyl | cyclopropyl | 3 | H | H | 4-fluorophenyl |
| 181) | benzyl | cyclopropyl | 3 | H | H | 4-trifluoromethylphenyl |
| 182) | benzyl | cyclopropyl | 3 | H | H | 4-methoxyphenyl |
| 183) | benzyl | cyclopropyl | 2 | methyl | methyl | 4-methylphenyl |
| 184) | benzyl | cyclopropyl | 2 | methyl | methyl | 4-bromophenyl |
| 185) | benzyl | cyclopropyl | 2 | methyl | methyl | 4-chlorophenyl |
| 186) | benzyl | cyclopropyl | 2 | methyl | methyl | 4-methyl-3-fluorophenyl |
| 187) | benzyl | cyclopropyl | 2 | methyl | methyl | 4-fluorophenyl |
| 188) | benzyl | cyclopropyl | 2 | methyl | methyl | 4-trifluoromethylphenyl |
| 189) | benzyl | cyclopropyl | 2 | methyl | methyl | 4-methoxyphenyl |
| 190) | benzyl | cyclopropyl | 3 | methyl | methyl | 4-methylphenyl |
| 191) | benzyl | cyclopropyl | 3 | methyl | methyl | 4-bromophenyl |
| 192) | benzyl | cyclopropyl | 3 | methyl | methyl | 4-chlorophenyl |
| 193) | benzyl | cyclopropyl | 3 | methyl | methyl | 4-methyl-3-fluorophenyl |
| 194) | benzyl | cyclopropyl | 3 | methyl | methyl | 4-fluorophenyl |
| 195) | benzyl | cyclopropyl | 3 | methyl | methyl | 4-trifluoromethylphenyl |
| 196) | benzyl | cyclopropyl | 3 | methyl | methyl | 4-methoxyphenyl |
| 197) | 3-fluorobenzyl | cyclopropyl | 2 | H | H | 4-methylphenyl |
| 198) | 3-fluorobenzyl | cyclopropyl | 2 | H | H | 4-bromophenyl |
| 199) | 3-fluorobenzyl | cyclopropyl | 2 | H | H | 4-chlorophenyl |
| 200) | 3-fluorobenzyl | cyclopropyl | 2 | H | H | 4-methyl-3-fluorophenyl |
| 201) | 3-fluorobenzyl | cyclopropyl | 2 | H | H | 4-fluorophenyl |
| 202) | 3-fluorobenzyl | cyclopropyl | 2 | H | H | 4-trifluoromethylphenyl |
| 203) | 3-fluorobenzyl | cyclopropyl | 2 | H | H | 4-methoxyphenyl |
| 204) | 3-fluorobenzyl | cyclopropyl | 3 | H | H | 4-methylphenyl |
| 205) | 3-fluorobenzyl | cyclopropyl | 3 | H | H | 4-bromophenyl |
| 206) | 3-fluorobenzyl | cyclopropyl | 3 | H | H | 4-chlorophenyl |
| 207) | 3-fluorobenzyl | cyclopropyl | 3 | H | H | 4-methyl-3-fluorophenyl |
| 208) | 3-fluorobenzyl | cyclopropyl | 3 | H | H | 4-fluorophenyl |
| 209) | 3-fluorobenzyl | cyclopropyl | 3 | H | H | 4-trifluoromethylphenyl |
| 210) | 3-fluorobenzyl | cyclopropyl | 3 | H | H | 4-methoxyphenyl |
| 211) | 3-fluorobenzyl | cyclopropyl | 2 | methyl | methyl | 4-methylphenyl |
| 212) | 3-fluorobenzyl | cyclopropyl | 2 | methyl | methyl | 4-bromophenyl |
| 213) | 3-fluorobenzyl | cyclopropyl | 2 | methyl | methyl | 4-chlorophenyl |
| 214) | 3-fluorobenzyl | cyclopropyl | 2 | methyl | methyl | 4-methyl-3-fluorophenyl |
| 215) | 3-fluorobenzyl | cyclopropyl | 2 | methyl | methyl | 4-fluorophenyl |
| 216) | 3-fluorobenzyl | cyclopropyl | 2 | methyl | methyl | 4-trifluoromethylphenyl |
| 217) | 3-fluorobenzyl | cyclopropyl | 2 | methyl | methyl | 4-methoxyphenyl |
| 218) | 3-fluorobenzyl | cyclopropyl | 3 | methyl | methyl | 4-methylphenyl |
| 219) | 3-fluorobenzyl | cyclopropyl | 3 | methyl | methyl | 4-bromophenyl |
| 220) | 3-fluorobenzyl | cyclopropyl | 3 | methyl | methyl | 4-chlorophenyl |
| 221) | 3-fluorobenzyl | cyclopropyl | 3 | methyl | methyl | 4-methyl-3-fluorophenyl |
| 222) | 3-fluorobenzyl | cyclopropyl | 3 | methyl | methyl | 4-fluorophenyl |
| 223) | 3-fluorobenzyl | cyclopropyl | 3 | methyl | methyl | 4-trifluoromethylphenyl |
| 224) | 3-fluorobenzyl | cyclopropyl | 3 | methyl | methyl | 4-methoxyphenyl |
| 225) | 4-fluorobenzyl | cyclopropyl | 2 | H | H | 4-methylphenyl |
| 226) | 4-fluorobenzyl | cyclopropyl | 2 | H | H | 4-bromophenyl |
| 227) | 4-fluorobenzyl | cyclopropyl | 2 | H | H | 4-chlorophenyl |
| 228) | 4-fluorobenzyl | cyclopropyl | 2 | H | H | 4-methyl-3-fluorophenyl |
| 229) | 4-fluorobenzyl | cyclopropyl | 2 | H | H | 4-fluorophenyl |
| 230) | 4-fluorobenzyl | cyclopropyl | 2 | H | H | 4-trifluoromethylphenyl |
| 231) | 4-fluorobenzyl | cyclopropyl | 2 | H | H | 4-methoxyphenyl |
| 232) | 4-fluorobenzyl | cyclopropyl | 3 | H | H | 4-methylphenyl |
| 233) | 4-fluorobenzyl | cyclopropyl | 3 | H | H | 4-bromophenyl |
| 234) | 4-fluorobenzyl | cyclopropyl | 3 | H | H | 4-chlorophenyl |
| 235) | 4-fluorobenzyl | cyclopropyl | 3 | H | H | 4-methyl-3-fluorophenyl |
| 236) | 4-fluorobenzyl | cyclopropyl | 3 | H | H | 4-fluorophenyl |
| 237) | 4-fluorobenzyl | cyclopropyl | 3 | H | H | 4-trifluoromethylphenyl |
| 238) | 4-fluorobenzyl | cyclopropyl | 3 | H | H | 4-methoxyphenyl |
| 239) | 4-fluorobenzyl | cyclopropyl | 2 | methyl | methyl | 4-methylphenyl |
| 240) | 4-fluorobenzyl | cyclopropyl | 2 | methyl | methyl | 4-bromophenyl |
| 241) | 4-fluorobenzyl | cyclopropyl | 2 | methyl | methyl | 4-chlorophenyl |
| 242) | 4-fluorobenzyl | cyclopropyl | 2 | methyl | methyl | 4-methyl-3-fluorophenyl |
| 243) | 4-fluorobenzyl | cyclopropyl | 2 | methyl | methyl | 4-fluorophenyl |
| 244) | 4-fluorobenzyl | cyclopropyl | 2 | methyl | methyl | 4-trifluoromethylphenyl |
| 245) | 4-fluorobenzyl | cyclopropyl | 2 | methyl | methyl | 4-methoxyphenyl |
| 246) | 4-fluorobenzyl | cyclopropyl | 3 | methyl | methyl | 4-methylphenyl |
| 247) | 4-fluorobenzyl | cyclopropyl | 3 | methyl | methyl | 4-bromophenyl |
| 248) | 4-fluorobenzyl | cyclopropyl | 3 | methyl | methyl | 4-chlorophenyl |
| 249) | 4-fluorobenzyl | cyclopropyl | 3 | methyl | methyl | 4-methyl-3-fluorophenyl |
| 250) | 4-fluorobenzyl | cyclopropyl | 3 | methyl | methyl | 4-fluorophenyl |
| 251) | 4-fluorobenzyl | cyclopropyl | 3 | methyl | methyl | 4-trifluoromethylphenyl |
| 252) | 4-fluorobenzyl | cyclopropyl | 3 | methyl | methyl | 4-methoxyphenyl |
| 253) | benzyl | sec-butyl | 2 | H | H | 4-methylphenyl |
| 254) | benzyl | sec-butyl | 2 | H | H | 4-bromophenyl |
| 255) | benzyl | sec-butyl | 2 | H | H | 4-chlorophenyl |
| 256) | benzyl | sec-butyl | 2 | H | H | 4-methyl-3-fluorophenyl |
| 257) | benzyl | sec-butyl | 2 | H | H | 4-fluorophenyl |
| 258) | benzyl | sec-butyl | 2 | H | H | 4-trifluoromethylphenyl |
| 259) | benzyl | sec-butyl | 2 | H | H | 4-methoxyphenyl |
| 260) | benzyl | sec-butyl | 3 | H | H | 4-methylphenyl |
| 261) | benzyl | sec-butyl | 3 | H | H | 4-bromophenyl |
| 262) | benzyl | sec-butyl | 3 | H | H | 4-chlorophenyl |
| 263) | benzyl | sec-butyl | 3 | H | H | 4-methyl-3-fluorophenyl |
| 264) | benzyl | sec-butyl | 3 | H | H | 4-fluorophenyl |
| 265) | benzyl | sec-butyl | 3 | H | H | 4-trifluoromethylphenyl |
| 266) | benzyl | sec-butyl | 3 | H | H | 4-methoxyphenyl |
| 267) | benzyl | sec-butyl | 2 | methyl | methyl | 4-methylphenyl |
| 268) | benzyl | sec-butyl | 2 | methyl | methyl | 4-bromophenyl |
| 269) | benzyl | sec-butyl | 2 | methyl | methyl | 4-chlorophenyl |
| 270) | benzyl | sec-butyl | 2 | methyl | methyl | 4-methyl-3-fluorophenyl |
| 271) | benzyl | sec-butyl | 2 | methyl | methyl | 4-fluorophenyl |
| 272) | benzyl | sec-butyl | 2 | methyl | methyl | 4-trifluoromethylphenyl |
| 273) | benzyl | sec-butyl | 2 | methyl | methyl | 4-methoxyphenyl |
| 274) | benzyl | sec-butyl | 3 | methyl | methyl | 4-methylphenyl |
| 275) | benzyl | sec-butyl | 3 | methyl | methyl | 4-bromophenyl |
| 276) | benzyl | sec-butyl | 3 | methyl | methyl | 4-chlorophenyl |
| 277) | benzyl | sec-butyl | 3 | methyl | methyl | 4-methyl-3-fluorophenyl |
| 278) | benzyl | sec-butyl | 3 | methyl | methyl | 4-fluorophenyl |
| 279) | benzyl | sec-butyl | 3 | methyl | methyl | 4-trifluoromethylphenyl |
| 280) | benzyl | sec-butyl | 3 | methyl | methyl | 4-methoxyphenyl |
| 281) | 3-fluorobenzyl | sec-butyl | 2 | H | H | 4-methylphenyl |
| 282) | 3-fluorobenzyl | sec-butyl | 2 | H | H | 4-bromophenyl |
| 283) | 3-fluorobenzyl | sec-butyl | 2 | H | H | 4-chlorophenyl |
| 284) | 3-fluorobenzyl | sec-butyl | 2 | H | H | 4-methyl-3-fluorophenyl |
| 285) | 3-fluorobenzyl | sec-butyl | 2 | H | H | 4-fluorophenyl |
| 286) | 3-fluorobenzyl | sec-butyl | 2 | H | H | 4-trifluoromethylphenyl |
| 287) | 3-fluorobenzyl | sec-butyl | 2 | H | H | 4-methoxyphenyl |
| 288) | 3-fluorobenzyl | sec-butyl | 3 | H | H | 4-methylphenyl |
| 289) | 3-fluorobenzyl | sec-butyl | 3 | H | H | 4-bromophenyl |
| 290) | 3-fluorobenzyl | sec-butyl | 3 | H | H | 4-chlorophenyl |
| 291) | 3-fluorobenzyl | sec-butyl | 3 | H | H | 4-methyl-3-fluorophenyl |
| 292) | 3-fluorobenzyl | sec-butyl | 3 | H | H | 4-fluorophenyl |
| 293) | 3-fluorobenzyl | sec-butyl | 3 | H | H | 4-trifluoromethylphenyl |
| 294) | 3-fluorobenzyl | sec-butyl | 3 | H | H | 4-methoxyphenyl |
| 295) | 3-fluorobenzyl | sec-butyl | 2 | methyl | methyl | 4-methylphenyl |
| 296) | 3-fluorobenzyl | sec-butyl | 2 | methyl | methyl | 4-bromophenyl |
| 297) | 3-fluorobenzyl | sec-butyl | 2 | methyl | methyl | 4-chlorophenyl |
| 298) | 3-fluorobenzyl | sec-butyl | 2 | methyl | methyl | 4-methyl-3-fluorophenyl |
| 299) | 3-fluorobenzyl | sec-butyl | 2 | methyl | methyl | 4-fluorophenyl |
| 300) | 3-fluorobenzyl | sec-butyl | 2 | methyl | methyl | 4-trifluoromethylphenyl |
| 301) | 3-fluorobenzyl | sec-butyl | 2 | methyl | methyl | 4-methoxyphenyl |
| 302) | 3-fluorobenzyl | sec-butyl | 3 | methyl | methyl | 4-methylphenyl |
| 303) | 3-fluorobenzyl | sec-butyl | 3 | methyl | methyl | 4-bromophenyl |
| 304) | 3-fluorobenzyl | sec-butyl | 3 | methyl | methyl | 4-chlorophenyl |
| 305) | 3-fluorobenzyl | sec-butyl | 3 | methyl | methyl | 4-methyl-3-fluorophenyl |
| 306) | 3-fluorobenzyl | sec-butyl | 3 | methyl | methyl | 4-fluorophenyl |
| 307) | 3-fluorobenzyl | sec-butyl | 3 | methyl | methyl | 4-trifluoromethylphenyl |
| 308) | 3-fluorobenzyl | sec-butyl | 3 | methyl | methyl | 4-methoxyphenyl |
| 309) | 4-fluorobenzyl | sec-butyl | 2 | H | H | 4-methylphenyl |
| 310) | 4-fluorobenzyl | sec-butyl | 2 | H | H | 4-bromophenyl |
| 311) | 4-fluorobenzyl | sec-butyl | 2 | H | H | 4-chlorophenyl |
| 312) | 4-fluorobenzyl | sec-butyl | 2 | H | H | 4-methyl-3-fluorophenyl |
| 313) | 4-fluorobenzyl | sec-butyl | 2 | H | H | 4-fluorophenyl |
| 314) | 4-fluorobenzyl | sec-butyl | 2 | H | H | 4-trifluoromethylphenyl |
| 315) | 4-fluorobenzyl | sec-butyl | 2 | H | H | 4-methoxyphenyl |
| 316) | 4-fluorobenzyl | sec-butyl | 3 | H | H | 4-methylphenyl |
| 317) | 4-fluorobenzyl | sec-butyl | 3 | H | H | 4-bromophenyl |
| 318) | 4-fluorobenzyl | sec-butyl | 3 | H | H | 4-chlorophenyl |
| 319) | 4-fluorobenzyl | sec-butyl | 3 | H | H | 4-methyl-3-fluorophenyl |
| 320) | 4-fluorobenzyl | sec-butyl | 3 | H | H | 4-fluorophenyl |
| 321) | 4-fluorobenzyl | sec-butyl | 3 | H | H | 4-trifluoromethylphenyl |
| 322) | 4-fluorobenzyl | sec-butyl | 3 | H | H | 4-methoxyphenyl |
| 323) | 4-fluorobenzyl | sec-butyl | 2 | methyl | methyl | 4-methylphenyl |
| 324) | 4-fluorobenzyl | sec-butyl | 2 | methyl | methyl | 4-bromophenyl |
| 325) | 4-fluorobenzyl | sec-butyl | 2 | methyl | methyl | 4-chlorophenyl |
| 326) | 4-fluorobenzyl | sec-butyl | 2 | methyl | methyl | 4-methyl-3-fluorophenyl |
| 327) | 4-fluorobenzyl | sec-butyl | 2 | methyl | methyl | 4-fluorophenyl |
| 328) | 4-fluorobenzyl | sec-butyl | 2 | methyl | methyl | 4-trifluoromethylphenyl |
| 329) | 4-fluorobenzyl | sec-butyl | 2 | methyl | methyl | 4-methoxyphenyl |
| 330) | 4-fluorobenzyl | sec-butyl | 3 | methyl | methyl | 4-methylphenyl |
| 331) | 4-fluorobenzyl | sec-butyl | 3 | methyl | methyl | 4-bromophenyl |
| 332) | 4-fluorobenzyl | sec-butyl | 3 | methyl | methyl | 4-chlorophenyl |
| 333) | 4-fluorobenzyl | sec-butyl | 3 | methyl | methyl | 4-methyl-3-fluorophenyl |
| 334) | 4-fluorobenzyl | sec-butyl | 3 | methyl | methyl | 4-fluorophenyl |
| 335) | 4-fluorobenzyl | sec-butyl | 3 | methyl | methyl | 4-trifluoromethylphenyl |
| 336) | 4-fluorobenzyl | sec-butyl | 3 | methyl | methyl | 4-methoxyphenyl |

The following examples illustrate the invention in greater detail, without restricting it. Further compounds according to this invention, of which the preparation is not explicitly described, can be prepared in an analogous way.

The compounds, which are mentioned in the examples, and the salts thereof, their stereoisomers and salts thereof, represent preferred embodiments of the invention.

In the examples, m.p. stands for melting point, h for hour(s), min for minutes, v:v or v:v:v or v:v:v:v for ratio of volumes, conc. for concentrated, M for molar concentration, THF for tetrahydrofurane, calc. for calculated, fnd. for found, EF for elemental formula, MS for mass spectrometry, LCMS for liquid chromatography mass spectrometry, HPLC for high pressure liquid chromatography, M⁺ for molecular ion in mass spectrometry, MH⁺ for the proton adduct ion of the molecule with the mass M, m/z for observed mass peak (mass per charge), NMR for nuclear magnetic resonance, and other abbreviations have their meanings customary per se to the skilled person.
Room temperature is a temperature between 20 and 25°C.

The names of the compounds have been generated using Autonom in ISIS, Autonom Engine Version 4.0, Copyright (1998) Beilstein Institut Frankfurt am Main.

Further on, according to common practice in stereochemistry, the symbol RS is used to denote the specific configuration of the indicated chiral centers of a racemate. In more detail, the term "(RS)" stands for a racemate comprising the one enantiomer having the configuration (R) and the other enantiomer having the configuration (S). Each of these enantiomers and their salts in pure form as well as their mixtures including the racemic mixtures is part of this invention.

### Examples

### Final compounds

**1. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide hydrochloride**
   A solution of 230 mg (1.0 eq) {3-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-p-tolyl-methanoyl)-amino]-n-propyl}-carbamic acid tert-butyl ester (compound A1) in 10 mL diethylether is treated with 5 mL HCl in dioxane (4.4 M). This mixture is stirred for 16 h at ambient temperature. Evaporation of the solvents in vacuo yielded 235 mg (quant.) of the title compound as colorless solid.
   MS: m/z (MH⁺) = 521.0, 523.1.
**1.a N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide**
   A solution of 336 mg (1.0 eq) {3-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-p-tolyl-methanoyl)-amino]-n-propyl}-carbamic acid tert-butyl ester (compound A1) in 8 mL dichloromethane is treated with 2 mL trifluoro acetic acid and stirred for 1 d at ambient temperature. The solvent is removed in vacuo and the residue is partitioned between dichloromethane and a saturated sodium hydrogen carbonate solution. The phases are separated and the organic phase is extracted three times with dichloromethane. The combined organic phase is dried with magnesiumsulfate and the solvent is evaporated in vacuo to yield the title compound as colorless solid.
   M.p.: 87-102 °C.
   MS: m/z (MH⁺) = 521.1, 523.0.
**2. N-(2-Amino-ethyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide hydrochloride**
   A solution of 196 mg (1.0 eq) {2-[[(RS)-1-(3-Benzyl-8-chloro-7-methyl-4-oxo-3,4-dihydro-pyrazolo[1,5-a][1,3,5]triazin-2-yl)-2-methyl-n-propyl]-(l-p-tolyl-methanoyl)-amino]-ethyl}-carbamic acid tert-butyl ester (compound A2) in 4 mL dichloromethane is treated with 1.3 mL HCl in dioxane (4 M). This mixture is stirred overnight at ambient temperature. After evaporation of the solvent, the residue is treated with diethylether, filtered and washed three times with diethylether. This yielded 141 mg (81%) of the title compound as colorless solid.
   M.p.: sinter starts at 167 °C, 189-205 °C (decomposition). MS: m/z (MH⁺) = 506.9, 509.0.
**3. N-[(RS)-(3-Benzyl-8-chloro-7-methyl-4-oxo-3,4-dihydro-pyrazolo[1,5-a][1,3,5]triazin-2-yl)-methyl-n-propyl]-N-(2-dimethylamino-ethyl)-4-methyl-benzamide**
   75 mg (1.0 eq) of N-(2-Amino-ethyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide hydrochloride (compound 2) are dissolved in 10 mL dichloromethane and treated with a saturated sodium hydrogen carbonate solution. After phase separation, extraction of the aqueous layer with dichloromethane, combination of the organic phase, drying over magnesiumsulfate and evaporation of the solvent yielded the free amine. This amine is dissolved in 1 mL formic acid, treated with 33 µL (3.0 eq) formaline and heated for 4 h at reflux. The solvent is removed in vacuo and the residue is purified by silica gel flash chromatography using ethyl acetate as eluent. This yielded 56 mg (76%) of the title compound as colorless foam. M.p.: sinter 73-78 °C, melting 80-84 °C.
   MS: m/z (MH⁺) = 535.0, 537.0.
**4. N-(3-Amino-n-propyl)-N-[(RS)-1-(3-benzyl-8-chloro-4-oxo-3,4-dihydro-pyrazolo[1,5-a][1,3,5]triazin-2-yl)-2-methyl-n-propyl]-4-methyl-benzamide hydrochloride**
   The title compound is prepared analogously to the procedure described for example 1. 125 mg of {3-[[(RS)-1-(3-Benzyl-8-chloro-4-oxo-3,4-dihydro-pyrazolo[1,5-a][1,3,5]triazin-2-yl)-2-methyl-n-propyl]-(1-p-tolyl-methanoyl)-amino]-n-propyl}-carbamic acid tert-butyl ester (compound A3, contaminated by acetic acid) yielded 70 mg (quant.) of the title compound as colorless solid.
   MS: m/z (MH⁺) = 507.1, 509.0.
**5. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-chloro-benzamide hydrochloride**
   The title compound is prepared analogously to the procedure described for example 2. 128 mg of (3-{[(RS)-1-(3-Benzyl-8-chloro-7-methyl-4-oxo-3,4-dihydro-pyrazolo[1,5-a] [1,3,5]triazin-2-yl)-2-methyl-n-propyl]-[1-(4-chlorophenyl)-methanoyl]-amino}-n-propyl)-carbamic acid tert-butyl ester (compound A4) yielded 90 mg (78%) of the title compound as colorless solid.
   M.p.: sinter 187-205 °C, melting 205-220 °C.
   MS: m/z (MH⁺) = 541.0, 543.0, 544.9.
**6. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-fluoro-benzamide hydrochloride**
   The title compound is prepared analogously to the procedure described for example 2. 108 mg of (3-{[(RS)-1-(3-Benzyl-8-chloro-7-methyl-4-oxo-3,4-dihydro-pyrazolo[1,5-a] [1,3,5]triazin-2-yl)-2-methyl-n-propyl]- [1-(4-fluorophenyl)-methanoyl]-amino}-n-propyl)-carbamic acid tert-butyl ester (compound A5) yielded 66 mg (69%) of the title compound as colorless solid.
   M.p.: 260-263 °C.
   MS: m/z (MH⁺) = 525.0, 526.9.
**7. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3-methyl-benzamide hydrochloride**
   The title compound is prepared analogously to the procedure described for example 2. 103 mg of {3-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-m-tolyl-methanoyl)-amino]-n-propyl}-carbamic acid tert-butyl ester (compound A6) yielded 40 mg (42%) of the title compound as colorless solid.
   M.p.: sinter 161-170 °C, melting 170-182 °C.
   MS: m/z (MH⁺) = 521.0, MH⁺+2.
**8. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-bromo-benzamide hydrochloride**
   The title compound is prepared analogously to the procedure described for example 2. 84 mg of (3-{[(RS)-1-(3-Benzyl-8-chloro-7-methyl-4-oxo-3,4-dihydro-pyrazolo[1,5-a] [1,3,5]triazin-2-yl)-2-methyl-n-propyl]-[1-(4-bromophenyl)-methanoyl]-amino}-n-propyl)-carbamic acid tert-butyl ester (compound A7) yielded 67 mg (90%) of the title compound as colorless solid.
   M.p.: sinter 179-193 °C, melting 193-206 °C.
   MS: m/z (MH⁺) = 584.8, 586.8, 588.9.
**9. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-trifluoromethyl-benzamide hydrochloride**
   The title compound is prepared analogously to the procedure described for example 2. 67 mg of (3-{[(RS)-1-(Benzyl-chloromethyl-oxo-3,4-dihydro-pyrazolo[1,5-a][1,3,5]triazin-2-yl)-2-methyl-n-propyl]-[1-(4-trifluoromethyl-phenyl)-methanoyl]-amino}-n-propyl)-carbamic acid tert-butyl ester (compound A8) yielded 38 mg (63%) of the title compound as colorless solid. M.p.: sinter 179-193 °C, melting 193-206 °C.
   MS: m/z (MH⁺) = 574.9, 577.0.
**10. N-(3-Amino-n-propyl)-N-{(RS)-1-[3-benzyl-7-(1,1-difluoro-methoxy)-4-oxo-3,4-dihydro-pyrazolo[1,5-a] [1,3,5]triazin-2-yl]-2-methyl-n-propyl}-4-methyl-benzamide hydrochloride**
   The title compound is prepared analogously to the procedure described for example 2. 198 mg of {3-[{(RS)-1-[3-Benzyl-7-(1,1-difluoro-methoxy)-4-oxo-3,4-dihydro-pyrazolo[1,5-a][1,3,5]triazin-2-yl]-2-methyl-n-propyl}-(1-p-tolyl-methanoyl)-amino]-n-propyl}-carbamic acid tert-butyl ester (compound A9) yielded 151 mg (85%) of the title compound as colorless solid.
   MS: m/z (MH⁺) = 539.0, 540.0.
**11. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3,4-dichloro-benzamide hydrocloride**
   The title compound is prepared analogously to the procedure described for example 2. 119 mg of (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(3,4-dichlorophenyl)-methanoyl]-amino}-n-propyl)-carbamic acid tert-butyl ester (compound A10) yielded 91 mg (82%) of the title compound as colorless solid.
   M.p.: sinter 181-196 °C, melting 196-224 °C.
   MS: m/z (MH⁺) = 574.9, 576.9, 578.8, MH⁺+6.
**12. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-2-fluoro-4-methyl-benzamide hydrocloride**
   The title compound is prepared analogously to the procedure described for example 2. 129 mg of (3-{[(RS)-1-(3-Benzyl-8-chloro-7-methyl-4-oxo-3,4-dihydro-pyrazolo[1,5-a][1,3,5] triazin-2-yl)-2-methyl-n-propyl]-[1-(2-fluoro-4-methyl-phenyl)-methanoyl]-amino}-n-propyl)-carbamie acid tert-butyl ester (compound A11) yielded 61 mg (53%) of the title compound as colorless solid.
   M.p.: sinter 177-204 °C, melting 204-212 °C.
   MS: m/z (MH⁺) = 539.0, 540.9.
**13. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3-fluoro-4-methyl-benzamide hydrocloride**
   The title compound is prepared analogously to the procedure described for example 2. 136 mg of (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(3-fluoro-4-methyl-phenyl)-methanoyl]-amino}-n-propyl)-carbamic acid tert-butyl ester (compound A12) yielded 76 mg (63%) of the title compound as colorless solid.
   M.p.: sinter 164-188 °C, melting 188-205 °C.
   MS: m/z (MH⁺) = 539.0, MH⁺+2.
**14. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-2,3-dichloro-benzamide hydrochloride**
   The title compound is prepared analogously to the procedure described for example 2. 139 mg of (3-{[(RS)-1-(3-Benzyl-8-chloro-7-methyl-4-oxo-3,4-dihydro-pyrazolo[1,5-a] [1,3,5]triazin-2-yl)-2-methyl-n-propyl]-[1-(2,3-dichlorophenyl)-methanoyl]-amino}-n-propyl)-carbamic acid tert-butyl ester (compound A13) yielded 101 mg (79%) of the title compound as colorless solid.
   M.p.: 200-210 °C.
   MS: m/z (MH⁺) = 574.9, 576.9, 578.9, 580.9.
**15. N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methoxy-benzamide hydrochloride**
   The title compound is prepared analogously to the procedure described for example 2. 103 mg of (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(4-methoxyphenyl)-methanoyl]-amino}-n-propyl)-carbamic acid tert-butyl ester (compound A14) yielded 76 mg (83%) of the title compound as colorless solid.
   M.p.: sinter 114-131 °C, melting 131-149 °C.
   MS: m/z (MH⁺) = 536.9, 539.0.
**16. N-(4-Amino-n-butyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide hydrocloride**
   The title compound is prepared analogously to the procedure described for example 2. 96 mg of {4-[[(RS)-1-(3-Benzyl-8-chloro-7-methyl-4-oxo-3,4-dihydro-pyrazolo[1,5-a] [1,3,5]triazin-2-yl)-2-methyl-n-propyl]-(1-p-tolyl-methanoyl)-amino]-n-butyl}-carbamic acid tert-butyl ester (compound A15) yielded 66 mg (77%) of the title compound as colorless solid.
   M.p.: 168-185 °C.
   MS: m/z (MH⁺) = 535.2, 537.2
**17. N-(3-Amino-n-propyl)-N-{(RS)-1-[3-benzyl-8-chloro-7-(1,1-difluoro-methoxy)-4-oxo-3,4-dihydro-pyrazolo[1,5-a] [1,3,5]triazin-2-yl]-2-methyl-n-propyl}-4-methyl-benzamide** hydrochloride
   434 mg (0.64 mmol) of {3-[[1-(3-Benzyl-8-chloro-7-difluoromethoxy-4-oxo-3,4-dihydro-pyrazolo[1,5a][1,3,5]triazin-2-yl) -2-methyl-n-propyl] - (4-methyl-benzoyl) -amino]-n-propyl}-carbamic acid tert-butyl ester is dissolved in 4 ml dioxane. 6 ml ( 24 mmol) of 4 M HCl in dioxane are added and the reaction is stirred for 4 h at ambient temperature. The precipitated solid is filtered, washed with diethylether and dried.
   By this method 344 mg ( 93 % ) of the title compound are obtained.
   M.p.: 220-222 °C.
**18. N-[(RS)-(3-Benzyl-8-chloro-7-methyl-4-oxo-3,4-dihydro-pyrazolo[1,5-a][1,3,5]triazin-2-yl)-methyl-n-propyl]-N-(4-dimethylamino-n-butyl)-4-methyl-benzamide**
   30 mg (1.0 eq) of N-(4-Amino-n-butyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide hydrocloride (compound 16) are dissolved in dichloromethane and treated with a saturated sodium hydrogen carbonate solution. After phase separation, extraction of the aqueous layer with dichloromethane, combination of the organic phase, drying over magnesiumsulfate and evaporation of the solvent yielded the free amine. This amine is dissolved in 1 mL formic acid, treated with 12 µL (3.0 eq) formaline and heated for 1 day at reflux. The solvent is removed in vacuo and the residue is purified by silica gel flash chromatography (dichloromethane/methanol = 100:0 to 90:10 + 1% triethylamine). This yielded 6 mg (22%) of the title compound as colorless viscous oil.
   MS: m/z (MH⁺) = 563.2, 565.0.
**19. N-[(RS)-(3-Benzyl-8-chloro-7-methyl-4-oxo-3,4-dihydro-pyrazolo[1,5-a][1,3,5]triazin-2-yl)-methyl-n-propyl]-N-(3-dimethylamino-n-propyl)-4-methyl-benzamide**
   100 mg (1.0 eq) of N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide (compound 1a) are dissolved in 1 mL formic acid, treated with 146 µL (9.6 eq) formaline and heated for 7 hours at reflux. The solvent is removed in vacuo and the residue is partitioned between water and dichloromethane. After basification with a 1M aqueous solution of sodium hydroxide the phases are separated. The aqueous layer is extracted with dichloromethane. The combined organic phase is dried over magnesium sulfate. After evaporation of the solvent in vacuo the residue is purified by silica gel flash chromatography (dichloromethane/methanol = 100:0 to 90:10 + 1% triethylamine). This yielded 50 mg (48%) of the title compound as slightly yellow foam.
   M.p.: sinter starts at 66 °C, melting at 89 °C.
   MS: m/z (MH⁺) = 549.1, 551.1.
**20. N-(3-Amino-n-propyl)-N-[(RS)-1-(3-benzyl-7-methoxy-4-oxo-3,4-dihydro-pyrazolo[1,5-a][1,3,5]triazin-2-yl)-2-methyl-n-propyl]-4-methyl-benzamide hydrochlorid**
   220 mg of {3-[[1-(3-Benzyl-7-methoxy-4-oxo-3,4-dihydro-pyrazolo[1,5-a][1,3,5]triazin-2-yl)-2-methyl-n-propyl]-(4-methyl-benzoyl)-amino]-n-propyl}-carbamic acid tert-butyl ester are dissolved in 3 ml Dioxane and 3ml of a 4N HCl in Dioxane are added. The mixture is stirred overnight. The resulting solid is filtered dried on air. 150 mg (83 %) of the title compound are obtained.
   M.p.: 220-235°C.
**21. N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-methyl-benzamide hydrochloride**
   238 mg {3-[[1-(3-benzyl-8-chloro-7-methoxy-4-oxo-3,4-dihydro-pyrazolo[1,5-a][1,3,5]triazin-2-yl)-2-methyl-n-propyl]-(4-methyl-benzoyl)-amino]-n-propyl}-carbamic acid tert-butyl ester are dissolved in 3 ml dioxane and treated with 3 ml 4N HCl in dioxane. After 2h at ambient temperature, the solution is evaported and the oily residue treated with diethylether. The resulting solid is collected. 140mg of the title compound are obtained.
   MS: m/z (MH⁺) = 537.2, 539.1.
**22. N-(3-Amino-n-propyl)-N-[(RS)-1-(3-benzyl-8-chloro-4-oxo-3,4-dihydro-pyrazolo[1,5-a][1,3,5]triazin-2-yl)-n-propyl]-4-methyl-benzamide hydrochloride**
   70 mg of {3-[[1-(3-Benzyl-8-chloro-4-oxo-3,4-dihydro-pyrazolo[1,5-a][1,3,5]triazin-2-yl)-n-propyl]-(4-methyl-benzoyl)-amino]-n-propyl}-carbamic acid tert-butyl esterare dissolved in 4 ml dioxane and the mixture is treated with 3ml of a 8.8 N HCl in dioxane and stirred overnight. The solvents are removed. 77 mg of the title compound are obtained as colorless solid.
   MS: m/z (MH⁺) = 493.2, 496.2.
**23. N-(3-Amino-n-propyl)-N-[(RS)-1-(3-benzyl-8-chloro-4-oxo-3,4-dihydro-pyrazolo[1,5-a][1,3,5]triazin-2-yl)-n-propyl]-4-chloro-benzamide hydrochloride**
   56 mg of {3-[[1-(3-Benzyl-8-chloro-4-oxo-3,4-dihydro-pyrazolo[1,5-a] [1,3,5]triazin-2-yl)-n-propyl]-(4-chloro-benzoyl)-amino]-n-propyl}-carbamic acid tert-butyl ester are dissolved in 3ml dioxane and treated with 2ml 8.8 N HCl in dioxane. The mixture is stirred overnight at ambient temperature. The sovents are removed in vacuo and 74 mg of the title compound are obtained as colorless solid.
   MS: m/z (MH⁺) = 517.1.
**24. N-(3-Amino-a-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-2-fluoro-4-methyl-benzamide hydrochloride**
   188 mg of {3-[[1-(3-benzyl-8-chloro-7-methoxy-4-oxo-3,4-dihydro-pyrazolo[1,5-a][1,3,5]triazin-2-yl)-2-methyl-n-propyl]-(2-fluoro-4-methyl-benzoyl)-amino]-n-propyl}-carbamic acid tert-butyl ester, 0.29 mmol are dissolved in 9ml dioxane and treated with 6ml 8.8 N HCl in dioxaneand stirred overnight at ambient temperature. The solvents are removed and the residue is treated wth diethyl ether. 174 mg of the title compound are obtained as yellowish solid.
**25. N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-3-fluoro-4-methyl-benzamide hydrochloride**
   In a similar way, from {3-[[1-(3-Benzyl-8-chloro-7-methoxy-4-oxo-3,4-dihydro-pyrazolo[1,5-a][1,3,5]triazin-2-yl)-2-methyl-n-propyl]-(3-fluoro-4-methyl-benzoyl)-amino]-n-propyl}-carbamic acid tert-butyl ester the title compound is obtained as yellowish solid.
**26. N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-methoxy-benzamide hydrochloride**
   In a similar way from {3-[[1-(3-Benzyl-8-chloro-7-methoxy-4-oxo-3,4-dihydro-pyrazolo[1,5-a][1,3,5]triazin-2-yl)-2-methyl-n-propyl]-(4-methoxy-benzoyl)-amino]-n-propyl}-carbamic acid tert-butyl ester the title compound is obtained as brownish solid.
**27. N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-chloro-benzamide hydrochloride**
   In a similar way, 96 mg of a yellowish solid are abtained from coresponding carbamic acid tert-butyl ester.

### Starting materials:

**A1. {3-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-p-tolyl-methanoyl)-amino]-n-propyl}-carbamic acid tert-butyl ester**
   A solution of 381 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 150 µL (1.5 eq) p-toluoyl chloride and 316 µL (3.0 eq) triethylamine in 5 mL dichloromethane is stirred for 1 day at ambient temperature. The reaction mixture is diluted with dichloromethane and treated with a saturated sodium hydrogen carbonate solution. The phases are separated and the aqueous layer is extracted two times with dichloromethane. The combined organic phase is dried over magnesiumsulfate, the solvent is evaporated and the residue is purified by silica gel flash chromatography using a gradient od ethyl acetate and n-hexane from 0:100 to 50:50. 351 mg (74%) of the title compound are isolated as colorless solid.
   M.p.: 177-182 °C
   MS: m/z (MH⁺) = 620.9, 622.8.
**A2. {2-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-p-tolyl-methanoyl)-amino]-ethyl}-carbamic acid tert-butyl ester**
   The title compound is prepared analogously to the procedure described for example A1. 233 mg (1.0 eq) {2-[(RS)-1-(3-Benzyl-8-chloro-7-methyl-4-oxo-3,4-dihydro-pyrazolo[1,5-a][1,3,5]triazin-2-yl)-2-methyl-n-propylamino]-ethyl}-carbamic acid tert-butyl ester (compound B2), 94 µL (1.5 eq) p-toluoyl chloride and 200 µL (3.0 eq) triethylamine yielded after three days stirring at ambient temperature, workup and silica gel flash chromatography 236 mg (81%) of the title compound as a slightly yellow foam.
   M.p.: 93-99 °C.
   MS: m/z (MH⁺) = 606.8, 608.8.
**A3. {3-[[(RS)-1-(3-Benzyl-8-chloro-4-oxo-3,4-dihydro-pyrazolo[1,5-a][1,3,5]triazin-2-yl)-2-methyl-n-propyl]-(1-p-tolyl-methanoyl)-amino]-n-propyl}-carbamic acid tert-butyl ester**
   The title compound is prepared analogously to the procedure described for example A1. 165 mg (1.0 eq) {3-[(RS)-1-(3-Benzyl-8-chloro-4-oxo-3,4-dihydro-pyrazolo[1,5-a][1,3,5]triazin-2-yl)-2-methyl-n-propylamino]-n-propyl}-carbamic acid tert-butyl ester (compound B3), 75 µL (1.5 eq) p-toluoyl chloride and 138 µL (3.0 eq) triethylamine yielded after two days stirring at ambient temperature, workup and silica gel flash chromatography (using cyclohexane / ethylacetate in a ratio of 2:1 + 1% acetic acid as eluent) 125 mg (51%) of the title compound as colorless solid.
**A4. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(4-chloro-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 57 µL (1.5 eq) 4-chloro-benzoyl chloride and 125 µL (3.0 eq) triethylamine yielded after workup and silica gel flash chromatography 157 mg (82%) of the title compound as a colorless foam.
   M.p.: 95-98 °C.
   MS: m/z (MH⁺) = 640.7, 642.8, 644.5.
**A5. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(4-fluoro-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 88 µL (2.5 eq) 4-fluoro-benzoyl chloride and 167 µL (4.0 eq) triethylamine yielded after 1 days stirring at ambient temperature, two hours at 50 °C, workup and silica gel flash chromatography 152 mg (81%) of the title compound as a colorless foam.
   M.p.: 85-90 °C.
   MS: m/z (MH⁺) = 624.8, MH⁺+2.
**A6. {3-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-m-tolyl-methanoyl)-amino]-n-propyl}-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 59 µL (1.5 eq) m-toluoyl chloride and 125 µL (3.0 eq) triethylamine yielded after workup and silica gel flash chromatography 138 mg (74%) of the title compound as a colorless foam.
   M.p.: sinter 82-90 °C, melting 90-95 °C.
   MS: m/z (MH⁺) = 620.8, 622.8.
**A7. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(4-bromo-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 165 mg (2.5 eq) 4-bromo-benzoyl chloride and 167 µL (4.0 eq) triethylamine yielded after 1 days stirring at ambient temperature, two hours at 50 °C, workup and silica gel flash chromatography 109 mg (53%) of the title compound as a colorless foam.
   M.p.: 215-220 °C.
   MS: m/z (MH⁺) = (684.7), 688.6, 688.7.
**A8. (3-{[(RS)-1-(Benzyl-chloro-methyl-oxo-3,4-dihydro-pyrazolo[1,5-a][1,3,5]triazin-2-yl)-2-methyl-n-propyl]-[1-(4-trifluoromethyl-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl)-carbamic acid tert-butyl ester (compound B1), 157 mg (2.5 eq) 4-(trifluoromethyl)-benzoyl chloride and 167 µL (4.0 eq) triethylamine yielded after 1 days stirring at ambient temperature, two hours at 50 °C, workup and silica gel flash chromatography 100 mg (50%) of the title compound as a colorless solid..
   M.p.: 215-217 °C.
   MS: m/z (MH⁺) = 674.7, 676.6.
**A9. {3-[{(RS)-l-[3-Benzyl-7-(1,1-difluoro-methoxy)-4-oxo-3,4-dihydro-pyrazolo[1,5-a][1,3,5]triazin-2-yl]-2-methyl-n-propyl}-(1-p-tolyl-methanoyl)-amino]-n-propyl}-carbamic acid tert-butyl ester**
   A solution of 220 mg (1.0 eq) (3-{(RS)-1-[3-Benzyl-7-(1,1-difluoro-methoxy)-4-oxo-3,4-dihydro-pyrazolo[1,5-a][1,3,5}triazin-2-yl]-2-methyl-n-propylamino}-n-propyl)-carbamic acid tert-butyl ester (compound B4), 84 µL (1.5 eq) p-toluoyl chloride and 175 µL (3.0 eq) triethylamine in 5 mL tetrahydrofurane is stirred for 3 days at ambient temperature. The solvent is evaporated and the residue is purified by silica gel flash chromatography using ethyl acetate and cyclohexane in a ratio of 1:3 as eluent. 220 mg (82%) of the title compound are isolated as colorless foam.
**A10. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(3,4-dichloro-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 94 mg (1.5 eq) 3,4-dichloro-benzoyl chloride and 125 µL (3.0 eq) triethylamine yielded after workup and silica gel flash chromatography 150 mg (74%) of the title compound as a colorless foam.
   M.p.: sinter 93-95 °C, melting 95-101 °C.
   MS: m/z (MH⁺) = 674.9, 676.5, 678.6, MH⁺+6.
**A11. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(2-fluoro-4-methyl-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 130 mg (2.5 eq) 2-fluoro-4-methyl-benzoyl chloride and 167 µL (4.0 eq) triethylamine yielded after two days stirring at ambient temperature, three hours at 50 °C, workup and silica gel flash chromatography 164 mg (86%) of the title compound as a colorless foam.
   M.p.: sinter 68-78 °C, melting 78-80 °C.
   MS: m/z (MH⁺) = 638.7, 640.8.
**A12. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(3-fluoro-4-methyl-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 130 mg (2.5 eq) 3-fluoro-4-methyl-benzoyl chloride and 167 µL (4.0 eq) triethylamine yielded after one day stirring at ambient temperature, three hours at 50 °C, workup and silica gel flash chromatography 174 mg (91%) of the title compound as a colorless foam.
   M.p.: sinter 75-80 °C, melting 80-85 °C.
   MS: m/z (MH⁺) = 638.7, 640.8.
**A13. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(2,3-dichloro-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 157 mg (2.5 eq) 2,3-dichloro-benzoyl chloride and 167 µL (4.0 eq) triethylamine yielded after two days stirring at ambient temperature, three hours at 50 °C, workup and silica gel flash chromatography 179 mg (88%) of the title compound as a colorless foam.
   M.p.: sinter 68-78 °C, melting 78-80 °C.
   MS: m/z (MH⁺) = (674.8), 676.8, 678.5.
**A14. (3-{[(RS)-1-(3-Benzyl-8-chloro-7-methyl-4-oxo-3,4-dihydro-pyrazolo[1,5-a][1,3,5]triazin-2-yl)-2-methyl-n-propyl]-[1-(4-methoxy-phenyl)-methanoyl]-amino}-n-propyl)-carbamic acid tert-butyl ester**
   The title compound is prepared analogously to the procedure described for example A1. 150 mg (1.0 eq) {3-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-propyl}-carbamic acid tert-butyl ester (compound B1), 128 mg (2.5 eq) p-anisoyl chloride and 167 µL (4.0 eq) triethylamine yielded after two days stirring at ambient temperature, workup and silica gel flash chromatography 134 mg (70%) of the title compound as a colorless foam.
   M.p.: sinter 85-89 °C, melting 89-93 °C.
   MS: m/z (MH⁺) = 636.9, 638.9.
**A15. {4-[[(RS)-1-(3-Benzyl-8-chloro-7-methyl-4-oxo-3,4-dihydro-pyrazolo[1,5-a][1,3,5]triazin-2-yl)-2-methyl-n-propyl]-(1-p-tolyl-methanoyl)-amino]-n-butyl}-carbamic acid tert-butyl ester**
   The title compound is prepared analogously to the procedure described for example A1. 167 mg (1.0 eq) {4-[(RS)-1-(3-Benzyl-8-chloro-7-methyl-4-oxo-3,4-dihydro-pyrazolo[1,5-a] [1,3,5]triazin-2-yl)-2-methyl-n-propylamino]-n-butyl}-carbamic acid tert-butyl ester (compound B5), 64 µL (1.5 eq) p-toluoyl chloride and 133 µL (4.0 eq) triethylamine yielded after four days stirring at ambient temperature, workup and silica gel flash chromatography 125 mg (61%) of the title compound as viscous oil.
   MS: m/z (MH⁺) = 634.7, 636.7.
**B1. {3-[(RS)-1-(3-Benzyl-8-chloro-7-methyl-4-oxo-3,4-dihydro-pyrazolo[1,5-a][1,3.,5]triazin-2-yl)-2-methyl-n-propylamino]-n-propyl}-carbamic acid tert-butyl ester**
   A solution of 1.71 g (1.0 eq) 2-((RS)-1-Amino-2-methyl-n-propyl)-3-benzyl-8-chloro-7-methyl-3H-pyrazolo[1,5-a][1,3,5]triazin-4-one (compound C1), 0.88 g (1.0 eq) (3-oxon-propyl)-carbamic acid tert-butyl ester (compound C4), 1.44 g (1.4 eq) sodium triacetoxyborohydride and 0.42 mL (1.4 eq) glacial acetic acid in 30 mL dichloromethane is stirred for half an hour at ambient temperature. The reaction mixture is diluted with dichloromethane and washed subsequently with saturated sodium hydrogen carbonate solution and brine. The organic layer is dried over magnesium sulfate, the solvent is evaporated under reduced pressure and the residue is purified by silica gel flash chromatography using a gradient of n-hexane and ethylacetate from 100:0 to 50:50. This yielded 2.16 g (88%) of the title compound as colorless solid.
   M.p.: 58-60 °C.
   MS: m/z (MH⁺) = 503.0, MH⁺+2.
**B2. {2-[(RS)-1-(3-Benzyl-8-chloro-7-methyl-4-oxo-3,4-dihydro-pyrazolo[1,5-a][1,3,5]triazin-2-yl)-2-methyl-n-propylamino]-ethyl}-carbamic acid tert-butyl ester**
   The title compound is prepared analogously to the procedure described for example B1. 200 mg (1.0 eq) 2-((RS)-1-Amino-2-methyl-n-propyl)-3-benzyl-8-chloro-7-methyl-3H-pyrazolo[1,5-a] [1,3,5]triazin-4-one (compound C1), 110 mg (1.2 eq) (3-oxoethyl)-carbamic acid tert-butyl ester, 172 mg (1.4 eq) sodium triacetoxyborohydride and 46 µL (1.4 eq) glacial acetic acid in 4 mL dichloromethane yielded after stirring for 2 hours at ambient temperature, workup and silica gel flash chromatography 267 mg (94%) of the title compound as colorless foam.
   M.p.: sinter starts at 58 °C, melting 67-73 °C.
   MS: m/z (MH⁺) = 488.9, MH⁺+2.
**B3. {3-[(RS)-1-(3-Benzyl-8-chloro-4-oxo-3,4-dihydro-pyrazolo[1,5-a][1,3,5]triazin-2-yl)-2-methyl-n-propylamino]-n-propyl}-carbamic acid tert-butyl ester**
   A solution of 230 mg (1.0 eq) 2-((RS)-1-Amino-2-methyl-n-propyl)-3-benzyl-8-chloro-3H-pyrazolo[1,5-a][1,3,5]triazin-4-one (compound C2), 180 mg (1.5 eq) (3-oxo-n-propyl)-carbamic acid tert-butyl ester (compound C4) and molecular sieves (4A) is stirred for 3 days at ambient temperature. After cooling to 0 °C 3 mL ethanol and 174 mg (4.0 eq) sodium cyanoborohydride are added. The reaction mixture is stirred for an additional 3 hours at ambient temperature. After filtration and washing with ethanol, the solvent is removed under reduced pressure. The residue is purified using silica gel flash chromatography (cyclohexane/ethylacetate = 2:1) to yield 165 mg (47%) of the title compound as colorless foam.
**B4. (3-{(RS)-1-[3-Benzyl-7-(1,1-difluoro-methoxy)-4-oxo-3,4-dihydro-pyrazolo[1,5-a][1,3,5}triazin-2-yl]-2-methyl-n-propylamino}-n-propyl)-carbamic acid tert-butyl ester**
   A solution of 230 mg (1.0 eq) 2-((RS)-1-Amino-2-methyl-n-propyl)-3-benzyl-7-(1,1-difluoro-methoxy)-3H-pyrazolo[1,5-a][1,3,5]triazin-4-one (compound C3), 168 mg (1.6 eq) (3-oxon-propyl)-carbamic acid tert-butyl ester (compound C4) and 1.0 g molecular sieves (4A) is stirred for 16 hours at ambient temperature. After cooling to 5 °C 5 mL ethanol and 159 mg (4.0 eq) sodium cyanoborohydride are added. The reaction mixture is stirred for an additional 3 hours at ambient temperature. The solvent is removed under reduced pressure and the residue is purified using silica gel flash chromatography (cyclohexane/ethylacetate = 3:2) to yield 250 mg (76%) of the title compound as slightly yellow oil.
**B5. {4-[(RS)-1-(3-Benzyl-8-chloro-7-methyl-4-oxo-3,4-dihydro-pyrazolo[1,5-a][1,3,5]triazin-2-yl)-2-methyl-n-propylamino]-n-butyl}-carbamic acid tert-butyl ester**
   The title compound is prepared analogously to the procedure described for example B1. 140 mg (1.0 eq) 2-((RS)-1-Amino-2-methyl-n-propyl)-3-benzyl-8-chloro-7-methyl-3H-pyrazolo[1,5-a][1,3,5]triazin-4-one (compound C1), 447 mg (6.0 eq) (3-oxon-butyl)-carbamic acid tert-butyl ester (compound C5), 122 mg (1.4 eq) sodium triacetoxyborohydride and 33 µL (1.4 eq) glacial acetic acid in 5 mL dichloromethane yielded after stirring for 16 hours at ambient temperature, workup and silica gel flash chromatography 175 mg (83%) of the title compound as viscous oil.
   MS: m/z (MH⁺) = 517.0, 519.0.
**C1. 2-((RS)-1-Amino-2-methyl-n-propyl)-3-benzyl-8-chloro-7-methyl-3H-pyrazolo[1,5-a][1,3,5]triazin-4-one**
   2.8 g (1.0 eq) of 2-((RS)-1-Azido-2-methyl-n-propyl)-3-benzyl-8-chloro-7-methyl-3H-pyrazolo[1,5-a][1,3,5]triazin-4-one (crude material, compound D1) are dissolved in 150 mL tetrahydrofurane. After addition of 3.0 g (1.5 eq) triphenylphosphine and 18 mL water the reaction mixture is heated for 16 hours at 50 °C. The solvents are removed under reduced pressure and the residue is treated with ethylacetate and water. The phases are separated and the aqueous phase is extracted three times with ethylacetate. The combined organic layer is dried over magnesium sulfate to afford the crude product. Silica gel flash chromatography with a gradient of hexane and ethylacetate from 100:0 to 30:70 yielded 1.8 g (68%) of the title compound as white foam.
   MS: m/z (MH⁺) = 345.9, 348.0.
**C2. 2-((RS)-1-Amino-2-methyl-n-propyl)-3-benzyl-8-chloro-3H-pyrazolo[1,5-a][1,3,5]triazin-4-one**
   655 mg (1.0 eq) of 2-((RS)-1-Azido-2-methyl-n-propyl)-3-benzyl-8-chloro-3H-pyrazolo[1,5-a][1,3,5]triazin-4-one (crude material, compound D2) are dissolved in 30 mL tetrahydrofurane. After addition of 640 mg (1.5 eq) triphenylphosphine and 10 drops of water the reaction mixture is stirred for 16 hours at ambient temperature. The solvents are removed under reduced pressure and the residue is purified using silica gel flash chromatography (cyclohexane/ethylacetate = 1:2) to yield 230 mg (35%) of the title compound as yellow oil. There are slight impurities of triphenylphosphine oxide.
**C3. 2-((RS)-1-Amino-2-methyl-n-propyl)-3-benzyl-7-(1,1-difluoro-methoxy)-3H-pyrazolo[1,5-a][1,3,5]triazin-4-one**
   320 mg (1.0 eq) of 2-((RS)-1-Azido-2-methyl-n-propyl)-3-benzyl-7-(1,1-difluoro-methoxy)-3H-pyrazolo[1,5-a][1,3,5]triazin-4-one (crude material, compound D3) are dissolved in 12 mL tetrahydrofurane. After addition of 323 mg (1.5 eq) triphenylphosphine and 0.2 mL water the reaction mixture is heated for 24 hours at 50 °C. The solvents are removed under reduced pressure and the residue is purified using silica gel flash chromatography (chloroform/tert-butyl methyl ether = 3:2) to yield 250 mg (84%) of the title compound as solidified oil.
**C4. (3-oxo-n-propyl)-carbamic acid tert-butyl ester**
   A solution of 9.00 g (1.0 eq) tert-butyl N-(3-hydroxy-n-propyl)carbamate, 1.67 g (0.1 eq) tetra-n-butylammonium bromide, 0.81 g (0.1 eq) 2,2,6,6-tetramethylpiperidinyloxy (TEMPO), 10.30 g (1.5 eq) N-chlorosuccinimide, 1.24 g (0.17 eq) potassium carbonate and 7.56 g (1.7 eq) sodium hydrogen carbonate in 180 mL warter and 180 mL chloroform is stirred vigorously for 150 min at ambient temperature. The phases are separated and the organic layer is dried over magnesiumsulfate. After evaporation of the solvent the crude product is purified by silica gel flash chromatography (n-hexane/ethylacetate = 100:0 to 50:50) to afford 6.05 g (68%) of the title compound as colorless oil.
**C5. (3-oxo-n-butyl)-carbamic acid tert-butyl ester**
   A solution of 5.12 g (1.0 eq) tert-butyl N-(3-hydroxy-n-butyl)carbamate, 0.87 g (0.1 eq) tetra-n-butylammonium bromide, 0.42 g (0.1 eq) 2,2,6,6-tetramethylpiperidinyloxy (TEMPO), 5.43 g (1.5 eq) N-chlorosuccinimide, 0.65 g (0.17 eq) potassium carbonate and 4.00 g (1.7 eq) sodium hydrogen carbonate in 95 mL warter and 95 mL chloroform is stirred for 18 hours at ambient temperature. The phases are separated and the organic layer is dried over magnesiumsulfate. After evaporation of the solvent the crude product is purified by silica gel flash chromatography (n-hexane/ethylacetate = 100:0 to 50:50) to afford 4.47 g (88%) of the title compound as colorless oil.
**D1. 2-((RS)-1-Azido-2-methyl-n-propyl)-3-benzyl-8-chloro-7-methyl-3H-pyrazolo[1,5-a][1,3,5]triazin-4-one**
   A solution of 3.10 g (1.0 eq) 3-Benzyl-2-((RS)-1-bromo-2-methyl-n-propyl)-8-chloro-7-methyl-3H-pyrazolo[1,5-a][1,3,5]triazin-4-one (compound E1) and 0.72 g (1.5 eq) sodium azide in 50 mL dimethylformamide is stirred for 3 days at ambient temperature. After evaporation of the solvent under reduced pressure the residue is partitioned between water and dichloromethane. The phases are separated and the aqueous phase is extracted three times with dichloromethane. The combined organic phase is dried over magnesiumsulfate and the solvent is evaporated in vacuo. This yielded in 2.94 g (quant.) of the crude product that is directly reduced to the corresponding amine (example C1).
   MS: m/z (MH⁺) = 371.9, 373.9.
**D2. 2-((RS)-1-Azido-2-methyl-n-propyl)-3-benzyl-8-chloro-3H-pyrazolo[1,5-a][1,3,5]triazin-4-one**
   A solution of 0.82 g (1.0 eq) 3-Benzyl-8-chloro-2-((RS)-1-chloro-2-methyl-n-propyl)-3H-pyrazolo[1,5-a][1,3,5]triazin-4-one (compound E2) and 0.30 g (2.0 eq) sodium azide in 20 mL dimethylformamide is stirred for 1 day at ambient temperature. The reaction mixture is diluted with water and extracted with diethylether. The combined organic phase is dried over magnesiumsulfate and the solvent is evaporated in vacuo. This yielded in 0.66 g (78%) of the crude product that is directly reduced to the corresponding amine (example C2).
**D3. 2-((RS)-1-Azido-2-methyl-n-propyl)-3-benzyl-7-(1,1-difluoro-methoxy)-3H-pyrazolo[1,5-a][1,3,5]triazin-4-one**
   A solution of 2.87 g (1.0 eq) 3-Benzyl-2-((RS)-1-bromo-2-methyl-n-propyl)-7-(1,1-difluoro-methoxy)-3H-pyrazolo[1,5-a][1,3,5]triazin-4-one (compound E3) and 0.66 g (1.5 eq) sodium azide in 40 mL dimethylformamide is stirred for 16 hours at ambient temperature. The residue is dissolved in ethylacetate and washed once with water. The organic phase is dried over sodiumsulfate and the solvent is evaporated in vacuo. This yielded in the crude product that is directly reduced to the corresponding amine (example C3).
**E1. -3-Benzyl-2-((RS)-1-bromo-2-methyl-n-propyl)-8-chloro-7 methyl-3H-pyrazolo[1,5-a][1,3,5]triazin-4-one**
   A solution of 3.48 g (1.0 eq) 3-Benzyl-8-chloro-2-isobutyl-7-methyl-3H-pyrazolo[1,5-a][1,3,5]triazin-one (compound F1) in 50 mL tetrachloromethane is treated with 1.87 g (1.0 eq) N-bromosuccinimide and a few milligrams of 2,2'-Azobis(isobutyronitrile). This mixture is heated at reflux for 14 hours. After filtration, the solution is washed with saturated aqueous hydrogen carbonate solution and dried over magnesium sulfate. The solvent is evaporated under reduced pressure to afford the title compound as crude product. Silica gel flash chromatography (n-hexane/ethylacetate = 100:0 to 70:30) afforded 3.10 g (72%) of the title compound as colorless solid.
   M.p.: sinter 107-120 °C, melting 120-124 °C.
   MS: m/z (MH⁺) = 408.9, 410.9, 412.9.
**E2. 3-Benzyl-8-chloro-2-((RS)-1-chloro-2-methyl-n-propyl)-3H-pyrazolo[1,5-a][1,3,5]triazin-4-one**
   A solution of 880 mg (1.0 eq) 3-Benzyl-8-chloro-2-isobutyl-3H-pyrazolo[1,5-a][1,3,5]triazin-4-one (compound F2) in 15 mL dimethylformamide is treated with 618 mg (1.2 eq) N-chlorosuccinimide. This mixture is heated at 80 °C for 3 hours. The mixture is diluted with diethylether and water. The phases are separated and the aqueous layer is extracted with diethylether. The combined organic phase is dried over magnesiumsulfate. The solvent is evaporated under reduced pressure to afford the title compound as crude product. Silica gel flash chromatography (cyclohexane/ethylacetate = 3:1) afforded 620 mg (58%, two steps) of the title compound as colorless solid.
**E3. 3-Benzyl-2-((RS)-1-bromo-2-methyl-n-propyl)-7-(1,1-difluoro-methoxy)-3H-pyrazolo[1,5-a][1,3,5]triazin-4-one**
   A solution of 2.94 g (1.0 eq) 3-Benzyl-7-(1,1-difluoromethoxy)-2-((RS)-1-hydroxy-2-methyl-n-propyl)-3H-pyrazolo[1,5-a][1,3,5]triazin-4-one (compound F3) in 110 mL dichloromethane is treated with 6.69 g (2.5 eq) tetrabromomethane. To this mixture 5.29 g (2.5 eq) triphenylphosphine in 40 mL dichloromethane are added dropwise at 5 °C. After complete addition the reaction mixture is stirred for 16 hours at ambient temperature. After evaporation of the solvent under reduced pressure the residue is purified using silica gel flash chromatography (n-heptan/tert-butylmethylether = 7:3) to afford 2.89 g (84%) of the title compound as colorless oil.
**F1. 3-Benzyl-8-chloro-2-isobutyl-7-methyl-3H-pyrazolo[1,5-a] [1,3,5]triazin-4-one**
   A suspension of 2.05 g (1.0 eq) 3-Benzyl-2-isobutyl-7-methyl-3H-pyrazolo[1,5-a][1,3,5]triazin-4-one (compound G1) in 23 mL tetrachloromethane is treated with 1.02 g (1.1 eq) N-chlorosuccinimide. This mixture is heated at reflux for 90 minutes. After filtration, the solution is washed with saturated aqueous hydrogen carbonate solution and dried over magnesium sulfate. The solvent is evaporated under reduced pressure to afford the title compound as crude product. Silica gel flash chromatography (n-hexane/ethylacetate = 100:0 to 50:50) afforded 1.93 g (84%) of the title compound as viscous oil.
   MS: m/z (MH⁺) = 330.9, 332.9.
**F2. 3-Benzyl-8-chloro-2-isobutyl-3H-pyrazolo[1,5-a] [1,3,5]triazin-4-one**
   A solution of 880 mg (1.0 eq) 3-Benzyl-2-isobutyl-3H-pyrazolo[1,5-a][1,3,5]triazin-4-one (compound G2) in 15 mL dimethylformamide is treated with 618 mg (1.2 eq) N-chlorosuccinimide. This mixture is heated at 60 °C for 30 minutes. The mixture is diluted with diethylether and water. The phases are separated and the aqueous layer is extracted three times with diethylether. The combined organic phase is dried over magnesiumsulfate. The solvent is evaporated under reduced pressure to afford the title compound as crude product. This is directly converted in example E2 to the corresponding dichloride.
**F3. 3-Benzyl-7-(1,1-difluoro-methoxy)-2-((RS)-1-hydroxy-2-methyl-n-propyl)-3H-pyrazolo[1,5-a][1,3,5]triazin-4-one**
   A solution of 6.40 g (1.0 eq) 7-(1,1-Difluor-methoxy)-2-((S)-1-hoydroxy-2-methyl-n-propyl)-3H-pyrazolo[1,5a][1,3,5]triazin-4-one (compound G3), 8.06 g (2.5 eq) potassium carbonate and 4.44 mL (1.6 eq) benzylbromide in 136 mL dimethylformamide is stirred for 3 days at ambient temperature. The solvents are removed under reduced pressure. The residue is dissolved in chloroform, washed with water and the organic layer is then dried over sodiumsulfate. After evaporation of the solvent in vacuo the crude product is crytallized from a mixture of 40 mL chloroform and 90 mL n-heptane to afford 2.64 g colorless crystals. The mother liquor is purified by silica gel flash chromatography (n-heptane/tert-butylmethylether = 1:1) to afford together with the crystals 3.51 g (41%) of the title compound.
**G1. 3-Benzyl-2-isobutyl-7-methyl-3H-pyrazolo[1,5-a] [1,3,5]triazin-4-one**
   A solution of 1.89 g (1.0 eq) 2-Isobutyl-7-methyl-3H-pyrazolo[1,5-a][1,3,5]triazin-4-one (compound Hl), 1.46 g (1.5 eq) potassium carbonate and 1.31 mL (1.2 eq) benzylbromide in 23 mL dimethylformamide is stirred for 60 hours at ambient temperature. After addition of water and dichloromethane the phases are separated and the aqueous layer is extracted with dichloromethane. The combined organic phase is dried over sodium sulfate and the solvent is evaporated under reduced pressure. The residue is purified by silica gel flash chromatography (cyclohexane/ethylacetate = 1:1) to yield 2.05 g (76%) of the title compound as viscous oil.
   MS: m/z (MH⁺) = 297.0.
**G2. 3-Benzyl-2-isobutyl-3H-pyrazolo[1,5-a][1,3,5]triazin-4-one**
   A solution of 3.40 g (1.0 eq) 2-Isobutyl-3H-pyrazolo[1,5-a][1,3,5]triazin-4-one (compound H2), 3.66 g (1.5 eq) potassium carbonate and 2.50 mL (1.2 eq) benzylbromide in 50 mL dimethylformamide is stirred for 3 days at ambient temperature. After addition of water and dichloromethane the phases are separated and the aqueous layer is extracted with dichloromethane. The combined organic phase is dried over sodium sulfate and the solvent is evaporated under reduced pressure. The residue is purified by silica gel flash chromatography (first: chloroform, second: cyclohexane/ethylacetate = 2:1) to yield 880 mg (18%) of the title compound as colorless solid. (The mixed fractions of the column chromatography are washed with diethylether).
**G3. 7-(1,1-Difluor-methoxy)-2-((S)-1-hoydroxy-2-methyl-n-propyl)-3H-pyrazolo[1,5a][1,3,5]triazin-4-one**
   To a mixture of 7.4 g (1.0 eq) 5-(1,1-Difluoro-methoxy)-2H-pyrazol-3-ylamine (compound H3) and 10.3 g (2.5 eq) sodium acetate in 110 mL acetonitrile 13.6 g (1.5 eq) (RS)-2-Hydroxy-3-methyl-butyrimidic acid ethyl ester (compound H4) are added in some portions. This mixture is stirred for 16 hours at ambient temperature. The solvent is evaporated in vacuo. The residue is dissolved in chloroform, washed with a 16% aqueous sodium carbonate solution and the organic layer is dried over sodiumsulfate. This yielded a foam that is dissolved in 240 mL acetonitrile. Subsequent addition of 14.0 mL (2.0 eq) triethylamine and carbonyldiimidazole (9.7 g, 1.2 eq, CDI) converted the amidine intermediate in 24 hours stirring at ambient temperature to the title compound. The crude product is purified using silica gel flash chromatorgraphy (chloroform/methanol = 20:1 + 2% ACOH). This afforded 10.0 g (73%) of the title compound.
**H1. 2-Isobutyl-7-methyl-3H-pyrazolo[1,5-a][1,3,5]triazin-4-one**
   5.70 g (10.0 eq) sodium are dissolved in 120 ml refluxing ethanol. After this solution is cooled to ambient temperature 5.4 g (1.0 eq) 3-Methyl-N-(5-methyl-2H-pyrazol-3-yl)-butyramidine (compound I1) in 50 mL ethanol and 30 mL (10.0 eq) diethylcarbonate are added. The reaction mixture is heated for 5 hours at 110 °C and for 14 hours at 80 °C. After evaporation of the solvents under reduced pressure, the residue is dissolved in water and acidified with glacial actetic acid. The aqueous layer is extracted several times using dichloromethane. The combined organic phase is dried over sodium sulfate and the solvent is removed in vacuo. The resulting oil is crystallized from diethylether to yield a colorless solid.
**H2. 2-Isobutyl-3H-pyrazolo[1,5-a][1,3,5]triazin-4-one**
   A solution of 7.25 g (1.0 eq) 3-Methyl-N-(2H-pyrazol-3-yl)-butyramidine (compound I2) and 6.76 g (1.0 eq) carbonyldiimidazole in 120 mL acetonitrile is stirred for 72 hours art ambient temperature. The solvent is removed in vacuo and the residue is purified using silica gel flash chromatography (pure ethylacetate) to yield 3.40 g (25%, two steps) of the title compound as colorless solid.
**H3. 5-(1,1-Difluoro-methoxy)-2H-pyrazol-3-ylamine**
   A mixture of 4.70 g (1.0 eq) 2-[5-(1,1-Difluoro-methoxy)-2H-pyrazol-3-yl]-isoindole-1,3-dione (compound I3), 2.36 mL (0.1 eq) triethylamine and 4.09 mL (0.5 eq) hydrazine hydrate (99%) in 275 mL tetrahydrofurane is stirred for 20 hours at ambient temperature. The precipitate is filtered. The solvent of the filtrate is evaporated in vacuo and the residue is purified by silica gel flash chromatography (chloroform/methanol = 9/1) to afford 2.0 g (80%) of the title compound as colorless oil.
**H4. (RS)-2-Hydroxy-3-methyl-butyrimidic acid ethyl ester**
   Gaseous HCl (∼20 g) is dissolved at -10 °C in 30 mL ethanol. To this solution 22.6 g of (RS)-2-Hydroxy-3-methylbutyronitrile (compound I4) are added. The temperature was always below 0 °C. This solution is stirred overnight and then treated with 500 mL diethylether to precipitate the title compound as colorless solid. After drying in an exsiccator over calciumchloride 38.2 g (92%) of the title compound are isolated as a colorless solid.
**I1. 3-Methyl-N-(5-methyl-2H-pyrazol-3-yl)-butyramidine**
   To a solution of 2.48 g (1.0 eq) 3-Amino-5-methylpyrazole in 14 mL acetonitrile 5.0 g (1.5 eq) 3-Methyl-butyrimidic acid ethyl ester (compound J1) in 30 mL dichloromethane are added. Addition of 1.7 mL (0.7 eq) glacial acetic acid and stirring for 15 hours at ambient temperature yielded after evaporation of the solvents the title compound as a yellow viscous oil that is directly converted in example H1 to the corresponding pyrazolotriazine.
**I2. 3-Methyl-N-(2H-pyrazol-3-yl)-butyramidine**
   To a solution of 6.0 g (1.0 eq) 3-amino-pyrazole in 120 mL acetonitrile are added 15.1 g (1.2 eq) 3-Methyl-butyrimidic acid ethyl ester hydrochloride (compound J2) and 23.6 g (4.0 eq) sodium acetate. After stirring overnight at ambient temperature the solvent is evaporated under reduced pressure. The residue is dissolved in chloroform and washed with saturated aqueous sodium carbonate solution. The aqueous layer is extracted once with chloroform. The combined organic phase Is dried over sodium sulfate and the solvent is evaporated in vacuo. This yielded 7.25 g (60%) of the title compound as crude product that is directly converted to the pyrazolotriazine (compound H2).
**I3. 2-[5-(1,1-Difluoro-methoxy)-2H-pyrazol-3-yl]-isoindole-1,3-dione**
   14.3 g (2.0 eq) gaseous bromodifluoromethane are added to a solution of 12.5 g (1.0 eq) 2-(5-Hydroxy-2H-pyrazol-3-yl)-isoindole-1,3-dione in 63 mL dimethylformamide. 31.3 mL (3.3 eq) ethyldiisopropylamine are added and the resulting mixture is stirred for 24 hours at ambient temperature. After evaporation of the solvent under reduced pressure, the residue is suspended in tetrahydrofurane. The suspension is filtered. Solvent evaporation of the filtrate in vacuo resulted in the crude product that is purified using silica gel flash chromatography (n-heptane/ethylacetate = 1:1). This afforded 2.74 g (18%) of the title compound
   M.p.: 96-154 °C.
**I4. (RS)-2-Hydroxy-3-methyl-butyronitrile**
   A mixture of 20.0 g (1.0 eq) isobutyraldehyde, 19.9 g (1.1 eq) potassium cyanide and 50.5 g (2.2 eq) sodium hydrogen carbonate in 335 mL diethylether and 200 mL water is stirred overnight at ambient temperature. The phases are separated and the aqueous layer is extracted three times with diethylether. The combined organic phase is dried over sodiumsulfate and the solvent is evaporated under reduced pressure. The crude product is kugelrohr distilled after addition of 280 mg chloro acetic acid (150 °C, 8-9 mbar). This afforded 22.8 g (83%) of the title compound.
**J1. 3-Methyl-butyrimidic acid ethyl ester**
   A mixture of 5.00 g (1.0 eq) isovaleramide and 11.9 g (1.1 eq) triethyloxonium hexafluorophosphate in 110 mL dichlroromethane is stirred for 36 hours at ambient temperature. Another 4.00 g (0.3 eq) triethyloxonium hexafluorophosphate are added and the mixture is stirred for another 14 hours. Washing with saturated aqueous sodium hydrogen carbonate solution, drying of the organic layer over sodium sulfate and evaporation of the solvent under reduced pressure (temperature of water bath: 20 °C) afforded 6.3 g of the title compound as colorless oil.
**J2. 3-Methyl-butyrimidic acid ethyl ester hydrochloride**
   Gaseous HCl (∼10 g) is dissolved at -10 °C in 20 mL ethanol. To this solution 5.00 g of isovaleronitrile are added. This solution is stirred for 2 hours at 0 °C before the solvent is evaporated under reduced pressure. The residue is treated with 100 mL diethylether to precipitate the title compound as colorless solid. After drying in an exsiccator over calciumchloride 7.03 g (71%) of the title compound are isolated as a colorless solid.
**J3. 2-(5-Hydroxy-2H-pyrazol-3-yl)-isoindole-1,3-dione**
   To a solution of 10.0 g (1.0 eq) 3-amino-5-hydroxy-pyrazole in 250 mL glacial acetic acid 15.0 g (1.0 eq) phtalic anhydride is added in some portions. The temperature rised and a precipitate is formed. The suspension is heated for 1 hour at 130 °C. By cooling to ambient temperature, 19.0 g (82%) of the title compound precipitated.
**K1. 5-Methoxy-2H-pyrazol-3-ylamine**
   To a solution of 6.74 g (29.4mmol) 2-(5-Hydroxy-2H-pyrazol-3-yl)-isoindole-1,3-dione in 110 ml dry tetrahydrofurane, 1.04ml (32mmol) methanole and 7.71 g triphenylphosphine is added. The solution is stirred 16 h at ambient temperature after adding 6.54 g (32mmol) azodicarboxylate at < 10°C. 7.15ml (147mmol) 99% hydrazinhydrate is added dropwise. The solution is stirred at ambient temperature for 1 hour, filtrated and the solvent is evaporated under reduced pressure. The residue is purified by silica gel flash chromatography ( CHCl₃/MeOH 9:1). By this method 1.1g (33%) of the title compound is obtained.
**K2. 2-(1-Hydroxy-2-methyl-n-propyl)-7-methoxy-3H-pyrazolo[1,5-a][1,3,5]triazin-4-one**
   To a solution of 4.28g (38 mmol) 5-methoxy-2H-pyrazol-3-ylamine in 83 ml acetonitrile 10.3 g(57mmol) of 2-hydroxy-3-methyl-butyrimidic acid ethyl ester hydrochloride and 7.79g ( 95 mmol) sodiumacetate are added bit by bit. The reaction is stirred 32h at ambient temperature. Solvents are evaporated under reduced pressure. The residue is solved in chloroform, the organic phase is washed with 16% sodiumcarbonate solution and dried over sodium sulfate.
   The precipitated solid is solved in tetrahydrofurane. The solution is filtered to remove the sodium sulfate. The solvents are evaporated under reduced pressure and the residue is solved in 180 ml of dry acetonitrile. After adding 10.6 ml (76mmol) of triethylamine, 7.36 g (45mmol) CDI is added. The reaction is stirred 3 d at ambient temperature. The solvent is evaporated under reduced pressure and the residue is purified by silica gel flash chromatography ( CHCl₃/MeOH 20:1 + 2 % AcOH).
   By this method 3.5 g ( 39 %) of the title compound are obtained.
**K3. 3-Benzyl-2-(1-hydroxy-2-methyl-n-propyl)-7-methoxy-3H-pyrazolo[1,5-a][1,3,5]triazin-4-one**
   3.5 g (14.7 mmol) 2-(1-Hydroxy-2-methyl-n-propyl)-7-methoxy-3H-pyrazolo[1,5-a][1,3,5]triazin-4-one, 5.08 (37mmol) potassium carbonate and 3.62 ml (22mmol) benzylbromide are solved in 50 ml dimethylformamide. The solution is stirred 2 days at ambient temperature. The solution is filtrated and the solvent is evaporated under reduced pressure. The residue is solved in chloroform. The organic phase is washed with water and dried with sodium sulfate. The solvent is evaporated and the residue is purified by silica gel flash chromatography (Heptan/tert-Butylmethylether1:4).
   By this method 1.55 g (32%) of the title compound are obtained.
**K4. 3-Benzyl-2-(1-bromo-2-methyl-n-propyl)-7-methoxy-3H-pyrazolo[1,5-a][1,3,5]triazin-4-one**
   1.55 g (4.72mmol) of 3-Benzyl-2-(1-hydroxy-2-methyl-n-propyl)-7-methoxy-3H-pyrazolo[1,5-a][1,3,5]triazin-4-one are solved in 67 ml of dichloromethane. 3.15 g (9.51 mmol) tetrabromomethan are added. The solution is stirred and a solution of 2.49 g (9.51 mmol) triphenylphosphin in 10 ml dichloromethane is added dropwise. The reaction is stirred over night at ambient temperature. The solvent is evaporated under reduced pressure and the residue is purified by silica gel flash chromatography (Heptan / tert-butylmethylether 1:1).
   By this method 1.22 g (66 %) of the title compound are obtained as colorless foam.
**K5. 2-(1-Azido-2-methyl-n-propyl)-3-benzyl-7-methoxy-3H-pyrazolo[1,5-a][1,3,5]triazin-4-one**
   1.22 g ( 3.12mmol) of 3-Benzyl-2-(1-bromo-2-methyl-n-propyl)-7-methoxy-3H-pyrazolo[1,5-a][1,3,5]triazin-4-one are dissolved in 19 ml dimethylformamide. 0.304 g ( 4.68 mmol) sodiumazide is added and the reaction is stirred 90 min at ambient temperature. Sovents are evaporated under reduced pressure and the residue is extracted between water and ethylacetate. The organic phase is dried with sodium sulfate. The solvent is evaporated under reduced pressure.
   By this method 1.17g of the title compound are obtained as oil.
**K6. 2-(1-Amino-2-methyl-n-propyl)-3-benzyl-7-methoxy-3H-pyrazolo[1,5-a][1,3,5]triazin-4-one**
   1.07 g (3.03mmol) 2-(1-Azido-2-methyl-n-propyl)-3-benzyl-7-methoxy-3H-pyrazolo[1,5-a][1,3,5]triazin-4-one and 1.19 g (4.45 mmol) triphenylphosphine are suspended in 45 ml tetrahydrofurane. Water (0.2 ml) is added and the reaction is heated for 2 h at 50 °C. The solvent is evaporated under reduced pressure and the residue is purified by silica gel flash chromatography (CHCl₃/tert-butyl-methylether 3:2). And (CHCl₃/MeOH 20:1) .
   By this method 973 mg (98%) of the title compound are obtained.
**K7. {3-[1-(3-Benzyl-7-methoxy-4-oxo-3,4-dihydro-pyrazolo[1,5-a][1,3,5]triazin-2-yl)-2-methyl-n-propylamino]-n-propyl}-carbamic acid tert-butyl ester**
   0.300 g (0.916 mmol) of 2-(1-Amino-2-methyl-n-propyl)-3-benzyl-7-methoxy-3H-pyrazolo[1,5-a][1,3,5]triazin-4-one are dissolved in 4 ml tetrahydrofurane. (3-Oxo-n-propyl)-carbamic acid tert-butyl ester (0.26g, 1.20 mmol) and 1 g molecular sieve 4 Å is added and the reaction is stirred for 3 d at ambient temperature. The reaction is cooled to 0°C and 4 ml ethanole and 0.230 mg( 3.7 mmol) sodium cyanoborhydride are added and the reaction is stirred 3 hours at ambient temperature. Solvents are evaporated under reduced pressure and the residue is purified by silica gel flash chromatography ( CHCl₃/MTB 8.5 : 1.5 ).
   By this method 186 mg ( 42 %) of the title compound are obtained.
**K8. {3-[[1-(3-Benzyl-7-methoxy-4-oxo-3,4-dihydro-pyrazolo[1,5-a][1,3,5]triazin-2-yl)-2-methyl-n-propyl]-(4-methyl-benzoyl)-amino]-n-propyl}-carbamic** acid **tert-butyl ester**
   0.586 g (1.21 mmol) of {3-[1-(3-Benzyl-7-methoxy-4-oxo-3,4-dihydro-pyrazolo[1,5-a][1,3,5]triazin-2-yl)-2-methyl-n-propylamino]-n-propyl}-carbamic acid tert-butyl ester is suspended in 12 ml tetrahydrofurane. 0.367 g ( 3.63 mmol) triethylamine and 0.28g (1.81 mmol) 4-methylbanzoylchloride are added and the reaction is stirred for 16 h at ambient temperature. The solvent is evaporated under reduced pressure and the residue is purified by silica gel flash chromatograpy ( Heptan/MTB 3:7).
   By this method 627 mg ( 86 %) of the title compound are obtained as a white foam.
**K9. N-(3-Amino-n-propyl)-N-[1-(3-benzyl-7-methoxy-4-oxo-3,4-dihydro-pyrazolo[1,5a][1,3,5]triazin-2-yl]-2-methyl-n-propyl]-4-methyl-benzamide hydrochloride**
   202 mg ( 0.335mmol) {3-[[1-(3-Benzyl-7-methoxy-4-Oxo-3,4-dihydro-pyrazolo[1,5-a][1,3,5]triazin-2-yl)-2-methyl-n-propyl]-(4-methyl-benzoyl)-amino]-n-propyl}-carbamic acid tert-butyl ester are solved in 3 ml Dioxan . 3 ml 4 M HCl in Dioxan are added and the reaction is stirred over night at ambient temperature. The prepicitated solid is filtered, washed with diethylether and dried.
   By this method 150 mg of the title compound is obtained.
**K10. N-(3-Amino-n-propyl)-N-[1-(3-benzyl-8-chloro-7-methoxy-4-oxo-3,4-dihydro-pyrazolo[1,5-a][1,3,5]triazin-2-yl)-2-methyl-n-propyl]-4-methyl-benzamide hydrochloride**
   238mg ( 0.374mmol) {3-[[1-(3-Benzyl-8-chloro-7-methoxy-4-oxo-3,4-dihydro-pyrazolo [1, 5-a] [1,3,5] triazin - 2 - yl) -2-methyl-n-propyl]-(1-p-tolyl-methanoyl)-amino]-n-propyl}-carbamic acid tert-butyl ester are solved in 3 ml Dioxan . 3 ml 4 M HCl in Dioxan are added and the reaction is stirred 2 h at ambient temperature. The solvent is evaporated under reduced pressure and 10 ml of diethyleter are added. The prepicitated solid is filtered, washed with diethylether and dried.
   By this method 140 mg ( 70 %) of a white solid is obtained.
**K11. 3-Benzyl-2-{(RS)-1-[3-(tert-butyl-dimethyl-silanyloxy)-n-propylamino]-2-methyl-n-propyl}-8-chloro-7-methyl-3H-pyrazolo[1,5-a][1,3,5]triazin-4-one**
   100 mg ( 0.29 mmol ) 2-((RS)-1-Amino-2-methyl-n-propyl)-3-benzyl-8-chloro-7-methyl-3H-pyrazolo[1,5-a][1,3,5]triazin-4-one, 66 µl ( 0.35 mmol) 3-(tert-butyl-dimethyl-silanyloxy)-propionaldehyde and 86 mg (0.41 mmol) sodium triacetoxyborohydride are dissolved in 3 ml dichloromethane + 24 µl acetic acid. The reaction is stirred for 1 h at ambient temperature. Dichloromethane is added and the organic layer is washed with NaHCO₃ -solution and with saturated sodium chloride solution. The organic layer is dried with sodium sulfate, filtered and the solvent is evaporated under reduced pressure.
   By this method 147 mg (98%) of a colorless oil are obtained.
**K12. N-[(RS)-1-(Benzyl-chloro-methyl-oxo-3,4-dihydro-pyrazolo [1,5-a][1,3,5]triazin-2-yl)-2-methyl-n-propyl]-N-[3-(tert-butyl-dimethyl-silanyloxy)-n-propyl]-4-methyl-benzamide**
   147 mg ( 0.28 mmo) 3-Benzyl-2-{(RS)-1-[3-(tert-butyldimethyl-silanyloxy)-n-propylamino]-2-methyl-n-propyl}-8-chloro-7-methyl-3H-pyrazolo[1,5-a][1,3,5] triazin-4-one are dissolved in dichloromethane. 56 µl (0.43 mmol) of 4-methyl benzoylchloride and 116 µl ( 0.84 mmol) of triethylamine are added and the reaction is stirred at ambient temperature. To complete the reaction 74 µl of 4-methylbenzoylchoride and 153 µl triethylamine are added after 5 days. The organic layer is washed with NaHCO₃ solution. Solvents are evaporated under reduced pressure and the crude product is purified by silica gel flash chromatography.
   By this method 132 mg (74%) of the title compound are obtained.
**K13. N-[(RS)-1-(3-Benzyl-8-chloro-7-methyl-4-oxo-3,4-dihydro-pyrazolo[1,5-a][1,3,5]triazin-2-yl)-2-methyl-n-propyl]-N-(3-hydroxy-n-propyl)-4-methyl-benzamide**
   120 mg (0.19 mmol) of N-[(RS)-1-(Benzyl-chloro-methyl-oxo-3,4-dihydro-pyrazolo[1,5- a][1,3,5] triazin-2-yl)-2-methyl-n-propyl]-N-[3-(tert-butyl-dimethyl-silanyloxy)-n-propyl] -4-methyl-benzamide and 82 µl ( 0.23 mmol) tetra-n-butylammoniumfluoride are dissolved in 2 ml THF. The reaction is stirred at ambient temperature. Solvent is evaporated under reduced pressure. The residue is extracted between water and dichloromethane. Solvent is evaporated and the residue is purified by silica gel flash chromatography.
   By this method 36 mg (36 %) of the title compound is obtained.
**K14. Butyrimidic acid ethyl ester hydrochloride**
   21.87 ml (250 mmol) butyronitrile are dissolved in 40 ml ethanole. The reaction is cooled to 0°C and HCl gas is passed through the solution. The temperature of the reaction is kept under 20 ° C.
   The reaction is stirred afterwards 2 days at ambient temperature. The solvent is evaporated under reduced pressure. The residue is suspended in ether and the precipitate is filtered and dried.
   By this method 33.92 g ( 89%) of the title compound are obtained.
**K15. N-(2H-Pyrazol-3-yl)-butyramidine**
   10 g (120.4 mmol) 2H-pyrazol-3-ylamine and 24.7 g (301 mmol) sodium acetate are dissolved in acetonitrile. 27.4 g (180.5 mmol) butyrimidic acid ethyl ester hydrochloride is added bit by bit . The reaction is stirred at ambient temperature over night. The solvent is evaporated under reduced pressure and the residue is dissolved in dichloromethane. The organic layer is washed with NaHCO₃-solution, dried with magnesium sulfate and the solvent is evaporated under reduced pressure. By this method 3.47 g ( 19 % ) of a yellow oil are obtained.
**K16. 2-n-propyl-3H-pyrazolo[1,5-a][1,3,5]triazin-4-one**
   3.45 g ( 22.7 mmol) of N-(2H-Pyrazol-3-yl)-butyramidine are dissolved in Acetonitril. 6.28 ml (45.3 mmol) of triethylamin are added.
   1,1'-Carbonyldiimidazole (4.42 g ; 27.2 mmol) are added bit by bit. The reaction is stirred over night by ambient temperature. Solvents are evaporated and the residue is solved in dichloromethane. The organic layer is extracted with NH₄Cl -solution, and then dried with magnesium sulfate. Solvents are evaporated under reduced pressure and the residue is purified by silica gel flash chromatography (DCM/MeOH 10:1 → 9:1).
   By this method 139 mg of the title compound are obtained.
**K17. 3-Benzyl-2-n-propyl-3H-pyrazolo[1,5-a][1,3,5]triazin-4-one**
   139 mg ( 0.78 mmol) of 2-n-propyl-3H-pyrazolo[1,5-a][1,3,5]triazin-4-one and 140 mg caliumcarbonat are dissolved in 2 ml dimethylformamide and stirred for 10 minutes at ambient temperature. 112µl ( 0.94 mmol) of benzylbromide are added and the reaction is stirred for 1 d at ambient temperature.
   Dichloromethan and water is added. After the extraction the organic layer is dried with magnesium sulfate, and the solvent is evaporated under reduced pressure. The residue is purified by silica gel flash chromatography (hexan/ethylacetat 10:0 → 0:10).
   By this method 72.7 mg of white crystals are obtained.
**K18. 2-((S)-1-Amino-2-methyl-n-propyl)-3-benzyl-8-chloro-7-methyl-3H-pyrazolo[1,5-a][1,3,5]triazin-4-one**
   Separation of the enantiomers at Chiral Technologies Europe. First eluting enantiomer of 2-((RS)-1-Amino-2-methyl-n-propyl)-3-benzyl-8-chloro-7-methyl-3H-pyrazolo[1,5-a] [1,3,5]triazin-4-one
**K19. 2-((R)-1-Amino-2-methyl-n-propyl)-3-benzyl-8-chloro-7-methyl-3H-pyrazolo[1,5-a][1,3,5]triazin-4-one**
   Separation of the enantiomers at Chiral Technologies Europe. Second eluting enantiomer of 2-((RS)-1-Amino-2-methyl-n-propyl)-3-benzyl-8-chloro-7-methyl-3H-pyrazolo[1,5-a] [1,3,5]triazin-4-one
**K20. {3-[[1-(3-Benzyl-8-chloro-7-difluoromethoxy-4-oxo-3,4-dihydro-pyrazolo[1,5a][1,3,5]triazin-2-yl)-2-methyl-n-propyl]-(4-methyl-benzoyl)-amino]-n-propyl}-carbamic acid tert-butyl ester**
   0.5 g (0.8 mmol) of {3-[{1-[3-Benzyl-7-(1,1-difluoromethoxy)-4-oxo-3,4-dihydro-pyrazolo[1,5-a][1,3,5]triazin-2-yl]-2-methyl-n-propyl}-(1-p-tolyl-methanoyl)-amino]-n-propyl}-carbamic acid tert-butyl ester is disolved in tetrachloromethane, 115 mg (0.9 mmol) of N-chlorosuccinimide are added and the reaction is stirred 3 h at 80 °C. After 3 h 52 mg ( 0.4 mmol) of N-chlorosuccinimide are added and the reaction is stirred for 8 h at 80°C. The solvent is evaporated under reduced pressure and the residue is purified by silica gel flash chromatography ( Cyclohexan/MTB 3:2).
   By this method 0.434 g ( 82%) of the title compound are obtained.
**K21. {3-[[1-(3-Benzyl-7-methoxy-4-oxo-3,4-dihydro-pyrazolo[1,5-a][1,3,5]triazin-2-yl)-2-methyl-n-propyl]-(4-methyl-benzoyl)-amino]-n-propyl}-carbamic acid tert-butyl ester**
   (0.586 g of {3-[1-(3-Benzyl-7-methoxy-4-oxo-3,4-dihydro-pyrazolo[1,5-a][1,3,5]triazin-2-yl)-2-methyl-n-propylamino]-n-propyl}-carbamic acid tert-butyl ester are dissolved in 12 ml THF, 0.367 g triethylamine and 0.280 g 4-methylbenzoylchloride are added and the addiert and the mixture is stirred for 6 h at ambient temperature. The resulting mixture is evaporated and the residue is purified by silica gel chromatography. 627 mg of the title compound are obtained as colorless foam.
**K22. {3-[1-(3-Benzyl-7-methoxy-4-oxo-3,4-dihydro-pyrazolo[1,5-a][1,3,5]triazin-2-yl)-2-methyl-n-propylamino]-n-propyl}-carbamic acid tert-butyl ester**
   0.586g of 2-(1-amino-2-methyl-n-propyl)-3-benzyl-7-methoxy-3H-pyrazolo[1,5-a][1,3,5]triazin-4-one are dissolved in THF (12 mL), 0.367 g triethylamine and 0.280 g 4-methylbenzoylchloride are added and the residue is stirred for 16h at ambient temperature. The solution is evaporated and the residue is purified by silica gel chromatography. 627 mg of the title compound as a colorless foam are obtained.
**K23. 2-(1-Amino-2-methyl-n-propyl)-3-benzyl-7-methoxy-3*H-*pyrazolo[1,5-a][1,3,5]triazin-4-one**
   To a mixture of 1.07 g of 2-(1-azido-2-methyl-n-propyl)-3-benzyl-7-methoxy-3H-pyrazolo[1,5-a][1,3,5]triazin-4-.one, 1.19 g triphenylphosphine and 45 ml THF are added 0.2 ml water. The mixture is stirred for 2 days at 50°C. The resulting solution is evaporated and the residue is purified by silica gel chromatography. 973mg of a foam are obtained.
**K24. 2-(1-Azido-2-methyl-n-propyl)-3-benzyl-7-methoxy-3H-pyrazolo[1,5-a][1,3,5]triazin-4-one**
   1.22 g of 3-Benzyl-2-(1-bromo-2-methyl-n-propyl)-7-methoxy-3H-pyrazolo[1,5-a][1,3,5]triazin-4-one are dissolved in 19ml DMF, treated with 0.304 g NaN3the mixture is stirred for 90 min at ambient temperature. The resulting mixture is evaporated and the residue is treated with ethylacetate and water, dried with Na2SO4 and evaporated. The crude product is used without further purification.
**K25. 3-Benzyl-2-(1-bromo-2-methyl-n-propyl)-7-methoxy-3H-pyrazolo[1,5-a][1,3,5]triazin-4-one**
   1.55 g of 3-Benzyl-2-(1-hydroxy-2-methyl-n-propyl)-7-methoxy-3H-pyrazolo[1,5-a][1,3,5]triazin-4-one are treated with dichloromethane (67 mL) and tetrabromomethane 3.15 g. At 5 °C eine a solution of triphenylphosphine (2.49 g, 9.51 mmol) in Dichlormethan (10 mL) is added dropwise and the mixture is stirred overnight at ambient temperature. The solution is evaporated and the residue is purified by column chromatography. The title compound is obtained as colorless foam (1.22 g 66 %).
**K26. 3-Benzyl-2-(1-hydroxy-2-methyl-n-propyl)-7-methoxy-3H-pyrazolo[1,5-a][1,3,5]triazin-4-one**
   3.50 g of 2-(1-hydroxy-2-methyl-n-propyl)-7-methoxy-3H-pyrazolo[1,5-a][1,3,5]triazin-4-one, 5.08 g K2CO3 and 3.62 mL benzylbromide are dissolved in 50 ml DMF and are stirred for 2 days at ambient temperature. The resulting mixture is filtered, evaporated and the residue is washed with CHCl3 and water. After work-up of the organic phase, the crude product is purified by silica gel chromatography. 1.55 g of the title compound are obtained.
**K27. 2-(1-Hydroxy-2-methyl-n-propyl)-7-methoxy-3H-pyrazolo[1,5-a][1,3,5]triazin-4-one**
   4.28 g of 5-methoxy-2H-pyrazol-3-ylamine in acetonitrile (83 mL) are added 10.3 g 2-hydroxy-3-methyl-butyrimidic acid ethyl ester hydrochloride and 7.79 g sodium acetate portionwise. The mixture is stirred for 32h at ambient temperature. The mixture is evaporated and worked up with CHCl3 and a Na2CO3-solution. By adding THF and filtration, the filtrate was evaporated and the residue was treatted with dry acetonitrile (180 mL). After adding 10.6 mL TEA, 7.36 g CDI are added and stirred for 3d. The solvents were evaporated and the residue was purified bay silica gel flash chromatography. 3.50 g of the title compound were obtained.
**K28. 2-Hydroxy-3-methyl-butyrimidic acid ethyl ester Hydrochloride**
   20.0 g of isobutyraldehyde in diethylether (335 mL) are stirred with a solution of 19.9 g KCN and NaHCO3 (50.5 g, 601 mmol) in water (200 mL) overnight at ambient temperature. The waterphase was washed 3x with 200 mL diethylether, the organic phases were compbined and dried. By adding chloroacetic acid (280 mg) the cyanhydrine is distilled. (8 - 9 mbar, 150 °C). 22.8 g of the title compound are obtained.
**K29. 5-Methoxy-2H-pyrazol-3-ylamine**
   6.74 g Hydroxypyrazol-derivative are dissolved in dry THF (110 mL)and 1.04 mL MeOH and 7.71 g triphenylphosphine are added. After subsequent adding of 6.54 g diethyl azodicarboxylate below 10°C the mixture is stirred 16 h at ambient temperature. Subsequently 7.15 mL 99 % hydrazinhydrate are added dropwise, stirred for 1 hand the filtrate is evaporated. The residue is purified by silica gel chromatography. 1.10 g of the title compound are obtained as oil.
**K30. {3-[[1-(3-Benzyl-8-chloro-7-methoxy-4-oxo-3,4-dihydro-pyrazolo[1,5-a][1,3,5]triazin-2-yl)-2-methyl-n-propyl]-(4-methyl-benzoyl)-amino}-n-propyl}-carbamic acid tert-butyl ester**
   0.390 g of {3-[[1-(3-benzyl-7-methoxy-4-oxo-3,4-dihydro-pyrazolo[1,5-a][1,3,5] triazin-2-yl)-2-methyl-n-propyl]-(4-methyl-benzoyl)-amino]-n-propyl}-carbamic acid tert-butyl ester were dissolved in 1,3 ml CCl4. To this mixture were added 0.130 g NCS and the mixture is heated to 80 °C for 5h. The resulting mixture is evaporated and the residue is purified by silica gel chromatography. 260 mg of a colorless solid were abtained.
**K31. {3-[1-(3-Benzyl-8-chloro-4-oxo-3,4-dihydro-pyrazolo[1,5-a][1,3,5]triazin-2-yl)-n-propylamino]-n-propyl}-carbamic** acid **tert-butyl ester**
   568 mg of 2-(1-amino-n-propyl)-3-benzyl-8-chloro-3H-pyrazolo[1,5-a][1,3,5]triazin-4-one were dissolved in dry 7.8 ml THF and treated with 403 mg of (3-oxo-n-propyl)-carbamic acid tert-butyl ester and 1.98 g molsieve 4 Å. the mixture is stirred for 2 days. Subsequently, 7.8 ml ethanol and 449 mg sodium cyanoborohydride are added at 0°C and stirred for further 3 h. The reaction mixture is filtered, and the mixture is purified by silica gel chromatography. 240 mg of a yellow oil are obtained.
**K32. {3-[[1-(3-Benzyl-8-chloro-4-oxo-3,4-dihydro-pyrazolo[1,5-a][1,3,5]triazin-2-yl)-n-propyl]-(4-methylbenzoyl)-amino]-n-propyl}-carbamic** acid **tert-butyl** ester
   122 mg of {3-[1-(3-benzyl-8-chloro-4-oxo-3,4-dihydro-pyrazolo[1,5-a][1,3,5]triazin-2-yl)-n-propylamino]-n-propyl}-carbamic acid tert-butyl ester , are dissolved in dry THF (4 mL) and are treated with 106 µL, 77.3 mg triethylamine and 41 µL, 47.7 mg 4-methylbenzoylchloride and 0.771 mmol trethylamine and stirred for 3 d at amibent temperature. The mixture is purified by silica gel chromatography. 74 mg of the title compound are obtained as colorless solid.
**K33. {3-[[1-(3-Benzyl-8-chloro-4-oxo-3,4-dihydro-pyrazolo[1,5-a][1,3,5]triazin-2-yl)-n-propyl]-(4-chloro-benzoyl)-amino]-n-propyl}-carbamic acid tert-butyl ester**
   112 mg of of {3-[1-(3-benzyl-8-chloro-4-oxo-3,4-dihydro-pyrazolo[1,5-a][1,3,5]triazin-2-yl)-n-propylamino]-n-propyl}-carbamic acid tert-butyl ester are treated similarly to above with 45 µL, 62 mg 4-chlorobenzoylchloride to obtain 62mg of the title compound as colorless solid.
**K34. {3-[[1-(3-Benzyl-8-chloro-7-methoxy-4-oxo-3,4-dihydro-pyrazolo[1,5-a][1,3,5]triazin-2-yl)-2-methyl-n-propyl]-(4-chloro-benzoyl)-amino]-n-propyl}-carbamic acid tert-butyl ester**
   The title compound as colorless solid is obtained in a similar way from {3-[1-(3-Benzyl-8-ehloro-7-methoxy-4-oxo-3,4-dihydro-pyrazolo[1,5-a][1,3,5]triazin-2-yl)-2-methyl-n-propylamino]-n-propyl}-carbamic acid tert-butyl ester wth 4-chlorobenzoylchlorid.
**K35. {3-[[1-(3-Benzyl-8-chloro-7-methoxy-4-oxo-3,4-dihydro-pyrazolo[1,5-a][1,3,5]triazin-2-yl)-2-methyl-n-propyl]-(2-fluoro-4-methyl-benzoyl)-amino]-n-propyl}-carbamic acid tert-butyl ester**
   300 mg of {3-[1-(3-Benzyl-8-chloro-7-methoxy-4-oxo-3,4-dihydro-pyrazolo[1,5-a][1,3,5]triazin-2-yl)-2-methyl-n-propylamino]-n-propyl}-carbamic acid tert-butyl esterare siddolved in 10ml abs. THF, treated with 150 mg 2-fluoro-4-methylbenzoylchloride (1.5 eq) and 174 mg triethylamine (3 eq) and stirred for 3 days at ambient temperature. The mixture is purified by silica gel chromatography. 188 mg od a colorless solid are obtained.
**K36. {3-[[1-(3-Benzyl-8-chloro-7-methoxy-4-oxo-3,4-dihydro-pyrazolo[1,5-a][1,3,5]triazin-2-yl)-2-methyl-n-propyl]-(3-fluoro-4-methyl-benzoyl)-amino]-n-propyl}-carbamic acid tert-butyl ester**
   182 mg of the title compound as colorless solid are obtained in a similar way from {3-[1-(3-Benzyl-8-chloro-7-methoxy-4-oxo-3,4-dihydro-pyrazolo[1,5-a][1,3,5]triazin-2-yl)-2-methyl-n-propylamino]-n-propyl}-carbamic acid tert-butyl ester wth 3-Fluor-4-methylbenzoylchlorid.
**K37. {3-[[1-(3-Benzyl-8-chloro-7-methoxy-4-oxo-3,4-dihydro-pyrazolo[1,5-a][1,3,5]triazin-2-yl)-2-methyl-n-propyl]-(4-methoxy-benzoyl)-amino]-n-propyl}-carbamic acid tert-butyl ester**
   202 mg of the title compound as colorless solid are obtained from {3-[1-(3-Benzyl-8-chloro-7-methoxy-4-oxo-3,4-dihydro-pyrazolo[1,5-a][1,3,5]triazin-2-yl)-2-methyl-n-propylamino]-n-propyl}-carbamic acid tert-butyl ester wth 4-methoxybenzoylchlorid.
**K38. {3-[1-(3-Benzyl-8-chloro-7-methoxy-4-oxo-3,4-dihydro-pyrazolo[1,5-a][1,3,5]triazin-2-yl)-2-methyl-n-propylamino]-n-propyl}-carbamic acid tert-butyl ester**
   4.21 g of 2-(1-Amino-2-methyl-n-propyl)-3-benzyl-8-chloro-7-methoxy-3H-pyrazolo[1,5-a][1,3,5]triazin-4-one are dissolved in 50 ml abs. THF, and treated with 3.02g (3-oxo-n-propyl)-carbamic acid tert-butyl ester and mol sieve 4 A (ca. 5 g) and are stirred at ambient temperature overnight. The reaction mixture is treated with ethanol, cooled to 0°C and are treated portionwise with 2.93 g NaCNBH₃ at -10 °C for 10 min. Subsequently, the mixture is filtered and the crude product in the filtrate is purified by silica gel chromatography. 6.20 g of a colorless foam are obtained.
**K39. 2-(1-Amino-2-methyl-n-propyl)-3-benzyl-8-chloro-7-methoxy-3H-pyrazolo[1,5-a][1,3,5]triazin-4-one**
   7.50 g of 3-Benzyl-8-chloro-2-(1-azido-2-methyl-n-propyl)-7-methoxy-3H-pyrazolo[1,5-a][1,3,5]triazin-4-one in 400 ml THF are treated with 9.49 g PPh3 and 1.65 ml water. The reaction mixture is stirred for 24 h at 50 °C. The solvents are removed and the residue is purified by silica gel chromatography. 2.80 g of the title compound as yellowish, oily compound are obtained.
**K40. 3-Benzyl-8-chloro-2-(1-azido-2-methyl-n-propyl)-7-methoxy-3H-pyrazolo[1,5-a][1,3,5]triazin-4-one**
   8.23 g of 3-Benzyl-8-chloro-2-(1-chloro-2-methyl-n-propyl)-7-methoxy-3*H*-pyrazolo[1,5-a][1,3,5]triazin-4-one in 150 ml DMF are treated with 2.11g NaN₃. The mixture is stirred for ambient temperature for 2 days and subseqently quenched with water. The reaction mixture is extracted with diethyl ether. The organic phase is washed twice with a saturated NaCl- solution, dried and evaporated. 7.50 g of a title compound are obtained as yellowish solid.
**K41. 3-Benzyl-8-chloro-2-(1-chloro-2-methyl-n-propyl)-7-methoxy-3H-pyrazolo[1,5-a][1,3,5]triazin-4-one**
   9.66 g 3-Benzyl-2-isobutyl-7-methoxy-3H-pyrazolo[1,5-a][1,3,5]triazin-4-one are dissolved in 150 ml DMF and treated with 5.0g NCS. The reaction solution is stirred for 16h at ambient temperature and heated for 1 day to 70°C. After this, further 820 mg NCS are added and the mixture is heated to 70 °C for one further day. Subsequently water is added and the mixture is extractet 3x with diethyl ether. The combined organic phases are washed with saturated NaCl- solution, dried and evaporated. 8.23 g of the title compound are obtained as offwhite solid.

### Commercial utility

The compounds of formula I, of which I* and I** are preferred, and their pharmaceutically acceptable salts (= the compounds, or a salt, stereoisomer or a salt of a stereoisomer thereof, according to the invention ) have valuable pharmaceutical and/or pharmacological properties which make them commercially utilizable. It has been unexpectedly found that these derivatives are potent and highly efficacious inhibitors of cellular hyperproliferation and/or cell-cycle specific inducers of apoptosis in cancer cells. In particular, the compounds, or a salt, stereoisomer or a salt of a stereoisomer thereof, according to the invention act as inhibitors of the mitotic kinesin Eg5. The compounds, or a salt, stereoisomer or a salt of a stereoisomer thereof, according to the invention display cell-cycle dependent, anti-proliferative and/or apoptosis inducing activity. Thus, they are commercially applicable in the therapy of diseases responsive to the inhibition of this kinesin, such as the diseases mentioned below, in particular the treatment of hyperproliferative diseases and disorders responsive to the induction of apoptosis.

In the context of this invention, the term "hyperproliferation" is used to describe aberrant and/or dysregulated cellular growth, a hallmark of diseases like cancer. This hyperproliferation might be caused by single or multiple cellular / molecular alterations in respective cells and can be, in context of a whole organism, of benign or malignant behaviour. The term "inhibition of cell proliferation" is used herein to denote an ability of the compound to retard the growth of and/or kill a cell contacted with that compound as compared to cells not contacted with that compound. Most preferable this inhibition of cell proliferation is 100%, meaning that proliferation of all cells is stopped and/or cells undergo programmed cell death. In some preferred embodiments the contacted cell is a neoplastic cell. A neoplastic cell is defined as a cell with aberrant cell proliferation and/or the potential to metastasize to different tissues or organs. A benign neoplasia is described by hyperproliferation of cells, incapable of forming an aggressive, metastasizing tumor in-vivo. In contrast, a malignant neoplasia is described by cells with different cellular and biochemical abnormalities, e.g. capable of forming tumor metastasis. The aquired functional abnormalities of malignant neoplastic cells (also defined as "hallmarks of cancer") are limitless replicative potential, self-sufficiency in growth signals, insensitivity to anti-growth signals, evasion from apoptosis, sustained angiogenesis and tissue invasion and metastasis.

Hyperproliferative diseases and/or disorders responsive to the induction of apoptosis are diseases and/or disorders resulting from the abovementioned cellular conditions.

The term "inducer of apoptosis" is used herein to identify a compound which induces programmed cell death in cells contacted with that compound. Apoptosis is defined by complex biochemical events within the contacted cell, such as the activation of cystein specific proteinases ("caspases") and the fragmentation of chromatin. Induction of apoptosis in cells contacted with the compound might not necessarily be coupled with inhibition of cell proliferation. Preferably, the inhibition of cell proliferation and/or induction of apoptosis is specific to cells with aberrant cell growth (hyperproliferation). Thus, compared to cells with aberrant cell growth, normal proliferating or arrested cells are less sensitive or even insensitive to the proliferation inhibiting or apoptosis inducing activity of the compound. Finally, cytotoxic is used in a more general sense to identify compounds which kill cells by various mechanisms, including the induction of apoptosis / programmed cell death in a cell cycle dependent or cell-cycle independent manner.

The term "cell cycle specific" is used herein to identify a compound as inducing apoptosis and killing only proliferating cells actively passing a specific phase of the cell cycle, but not exerting this effect in resting, non-dividing cells. Continously proliferating cells are typical for diseases like cancer and characterized by cells passing all phases of the cell division cycle, namely in the G ("gap") 1, S ("DNA synthesis"), G2 and M ("mitosis") phase.

The mitotic kinesin Eg5 is an enzyme essential for the assembly and function of the bipolar mitotic spindle. Eg5 plays essential roles during various phases of mitosis. Drugs that perturb mitosis have proven clinically effective in the treatment of many cancers. Despite the diverse array of essential spindle proteins that could be exploited as targets for the discovery of novel cancer therapies, all spindle-targeted therapeutics in clinical use today act on only one protein, tubulin. Surprisingly, kinesin Eg5 expression is most abundant in proliferating human tissues, whereas it is absent from most postmitotic cells, such as e.g. human central nervous system neurons, pointing to an exclusive or almost confined role for Eg5 in cell proliferation. In contrary to drugs that directly interfere with microtubule dynamic instability, Eg5 kinesin inhibitors are expected not to disrupt microtubule-based cellular processes, e.g. neuronal transport, that are unrelated to proliferation. During mitosis, Eg5 is essentially involved in organizing microtubules into a bipolar structure that forms the mitotic spindle.
Experimental perturbation of Eg5 function causes a characteristic malformation or dysfunction of the mitotic spindle, frequently resulting in cell cycle arrest and cell death.

On account of their Eg5-inhibiting properties, the compounds, or a salt, stereoisomer or a salt of a stereoisomer thereof, according to the invention can be used to modulate mitotic spindle formation, thus causing prolonged cell cycle arrest in mitosis, which is frequently followed by apoptosis. By "modulate" herein is meant altering mitotic spindle formation, including increasing and decreasing spindle formation. By "mitotic spindle formation" herein is meant organization of microtubules into bipolar structures by mitotic kinesins. By "dysfunction of the mitotic spindle" herein is meant mitotic arrest and monopolar spindle formation. "Malformation of the mitotic spindle" encompasses the splaying of mitotic spindle poles, or otherwise causing morphological perturbation of the mitotic spindle.

On account of their Eg5-inhibiting properties, the compounds, or a salt, stereoisomer or a salt of a stereoisomer thereof, according to the invention can further be useful in the treatment of benign or malignant neoplasia.
A "neoplasia" is defined by cells displaying aberrant cell proliferation and/or survival and/or a block in differentiation. A "benign neoplasia" is described by hyperproliferation of cells, incapable of forming an aggressive, metastasizing tumor in-vivo. In contrast, a "malignant neoplasia" is described by cells with multiple cellular and biochemical abnormalities, capable of forming a systemic disease, for example forming tumor metastasis in distant organs.

The compounds, or a salt, stereoisomer or a salt of a stereoisomer thereof, according to the invention are capable of modulating, particularly inhibiting Eg5 activity. They are capable of modulating the mitotic spindle, particularly inhibiting mitosis.
Thus, the compounds, or a salt, stereoisomer or a salt of a stereoisomer thereof, according to the invention have cellular anti-proliferative properties. Thus, they modulate apoptosis and/or aberrant cell growth in the therapy of benign or malign neoplastic diseases, preferably cancer.

Various diseases are caused by aberrant cell proliferation ("hyperproliferation") as well as evasion from apoptosis. These diseases include e.g. benign hyperplasia like that of the prostate ("BPH") or colon epithelium, psoriasias, glomerulonephritis or osteoarthritis. Most importantly these diseases include malignant neoplasia commonly described as cancer and characterized by tumor cells finally metastasizing into distinct organs or tissues. Malignant neoplasia include solid and hematological tumors. Solid tumors are exemplified by tumors of the breast, bladder, bone, brain, central and peripheral nervous system, colon, endocrine glands (eg thyroid and adrenal cortex), esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva. Malignant neoplasia include inherited cancers exemplified by retinoblastoma and Wilms tumor. In addition, malignant neoplasia include primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

The invention therefore relates to a use of the compounds, or a salt, stereoisomer or a salt of a stereoisomer thereof, according to the invention in the manufacture of pharmaceutical compositions, a method of treatment or a combination according to the invention, in which the cancer to be treated is selected from the group consisting of cancer of the breast, bladder, bone, brain, central and peripheral nervous system, colon, endocrine glands, esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva; inherited cancers, retinomblastoma and Wilms tumor;
leukemia, lymphoma, non-Hodgkins disease, chronic and acute myeloid leukaemia, acute lymphoblastic leukemia, Hodgkins disease, multiple myeloma and T-cell lymphoma; myelodysplastic syndrome, plasma cell neoplasia, paraneoplastic syndromes, cancers of unknown primary site and AIDS related malignancies.

It is to be noted that a cancer disease as well as a malignant neoplasia does not necessarily require the formation of metastases in distant organs. Certain tumors exert devastating effects on the primary organ itself through their aggressive growth properties. These can lead to the destruction of the tissue and organ structure finally resulting in failure of the assigned organ function.

Neoplastic cell proliferation might affect normal cell behaviour and organ function. For example the formation of new blood vessels, a process described as neovascularization, is induced by tumors or tumor metastases. Compounds, or a salt, stereoisomer or a salt of a stereoisomer thereof, according to the invention can be commercially applicable for the treatment of pathophysiological relevant processes caused by benign or neoplastic cell proliferation, such as but not limited to neovascularization by unphysiological proliferation of vascular endothelial cells.

Drug resistance is of particular importance for the frequent failure of standard cancer therapeutics. This drug resistance is caused by various cellular and molecular mechanisms like overexpression of drug efflux pumps or mutation within the cellular target protein. The commercial applicability of the compounds, or a salt, stereoisomer or a salt of a stereoisomer thereof, according to the invention is not limited to 1^{st} line treatment of patients. Patients with resistance to defined cancer chemotherapeutics or target specific anti-cancer drugs (2^{nd} or 3^{rd} line treatment) can be also amenable for treatment with the compounds, or a salt, stereoisomer or a salt of a stereoisomer thereof, according to the invention.

Due to their cellular anti-proliferative properties, compounds according to the present invention may be also commercially usable for treatment of diseases associated with cell cycle and cell proliferation, such as, besides cancer discussed above, for example, fibroproliferative and differentiative disorders, psoriasis, rheumatoid arthritis, atherosclerosis, hyperplasia, restenosis, cardiac hypertrophy, (auto)immune disorders, fungal disorders, bone diseases, or acute or chronic inflammation.
Thus, the invention relates to compounds, or a salt, stereoisomer or a salt of a stereoisomer thereof, according to the invention for use in the treatment of diseases.

In particular, the compounds according to the present invention are commercially applicable for the treatment of hyperproliferative diseases and disorders responsive to the induction of apoptosis. In a preferred aspect, they are useful for the treatment, prevention or amelioration of hyperproliferative diseases and disorders responsive to the induction of apoptosis.
In a preferred aspect, the compounds, or a salt, stereoisomer or a salt of a stereoisomer thereof, according to the invention are useful in the treatment, prevention or amelioration of benign neoplasia or malignant neoplasia, particularly cancer, more particularly a cancer that is susceptible to Eg5 inhibition, more particularly any of the cancer diseases described above. Preferred is the treatment of said diseases.

Thus, the present invention further relates to compounds, or a salt, stereoisomer or a salt of a stereoisomer thereof, according to the invention for use in therapy, such as, for example, in the treatment, prevention or amelioration of hyperproliferative diseases and disorders responsive to the induction of apoptosis. In a preferred aspect, the invention relates to compounds, or a salt, stereoisomer or a salt of a stereoisomer thereof, according to the invention for use in the treatment, prevention or amelioration of benign neoplasia or malignant neoplasia, particularly cancer.

In the context of their properties, functions and usabilities mentioned herein, the compounds according to the present invention are expected to be distinguished by valuable and desirable effects related therewith, such as e.g. by low toxicity, superior bioavailability in general (such as e.g. good enteral absorption), superior therapeutic window, absence of significant side effects, and/or further beneficial effects related with their therapeutic and pharmaceutical suitability.

In a preferred aspect, the invention relates to a method for treating, preventing or ameliorating hyperproliferative diseases and disorders responsive to the induction of apoptosis in mammals, comprising administering to said mammals in need thereof a pharmaceutically active and therapeutically effective and tolerable amount of one or more of the compounds or a salt, stereoisomer or a salt of a stereoisomer thereof, according to the invention. Preferred is the method of treatment of said diseases and disorders.

In a particularly preferred aspect, the invention relates to a method of treatment of the diseases, disorders, conditions or illnesses mentioned above, particularly benign neoplasia or malignant neoplasia, comprising administering to said mammals in need thereof a pharmaceutically active and therapeutically effective and tolerable amount of one or more of the compounds, or a salt, stereoisomer or a salt of a stereoisomer thereof, according to the invention. In a particular aspect, the disease is cancer, more particularly a cancer that is susceptible to Eg5 inhibition, more particularly any of the cancer diseases described above.

The invention further includes a method of modulating, particularly inhibiting, Eg5 activity in cells comprising administering a pharmaceutically active and therapeutically effective and tolerable amount of one or more of the compounds, or a salt, stereoisomer or a salt of a stereoisomer thereof, according to the invention to a patient in need of such modulation, particularly inhibition.

The present invention further includes a method of modulating apoptosis or aberrant cell growth in the therapy of benign or malignant neoplastic diseases, e.g. cancer, comprising administering to a subject in need of such therapy a pharmaceutically active and therapeutically effective and tolerable amount of one or more of the compounds, or a salt, stereoisomer or a salt of a stereoisomer thereof, according to the invention.

The present invention further includes a method to modulate the mitotic spindle, i.e., for example, altering mitotic spindle formation, including decreasing spindle formation, or increasing or decreasing spindle pole separation causing malformation of the mitotic spindle poles, comprising administering a pharmaceutically active and therapeutically effective and tolerable amount of one or more of the compounds, or a salt, stereoisomer or a salt of a stereoisomer thereof, according to the invention to a patient in need of such modulation.

The present invention further includes a method to inhibit mitosis in cells comprising administering a pharmaceutically active and therapeutically effective and tolerable amount of one or more of the compounds, or a salt, stereoisomer or a salt of a stereoisomer thereof, according to the invention to a patient in need of such inhibition.

The present invention further includes a method for treating, preventing or ameliorating diseases and/or disorders associated with Eg5 kinesin activity, such as, for example, hyperproliferative diseases and/or disorders responsive to induction of apoptosis, for example, benign or malignant neoplasia, e.g. cancer, in a mammal comprising administering a pharmaceutically active and therapeutically effective and tolerable amount of one or more compounds according to the present invention to said mammal in need thereof.

The present invention further relates to the use of the compounds, or a salt, stereoisomer or a salt of a stereoisomer thereof, according to the invention for the production of pharmaceutical compositions which are employed for the treatment, prophylaxis and/or amelioration of one or more of the illnesses mentioned.

The present invention particularly relates to the use of the compounds, or a salt, stereoisomer or a salt of a stereoisomer thereof, according to the invention in the production of pharmaceutical compositions for the treatment, prevention or amelioration of hyperproliferative diseases and disorders responsive to the induction of apoptosis.
The invention particularly relates to the use of the compounds, or a salt, stereoisomer or a salt of a stereoisomer thereof, according to the invention in the production of pharmaceutical compositions for the treatment, prevention or amelioration of benign neoplasia or malignant neoplasia, particularly cancer, more particularly a cancer that is susceptible to Eg5 inhibition, more particularly any of the cancer diseases described above. Preferred is the use of the compounds, or a salt, stereoisomer or a salt of a stereoisomer thereof, according to the invention for the production of pharmaceutical compositions which are used in the treatment of mammals.

The invention further relates to a compound according to the invention or a pharmaceutically acceptable salt thereof, for the treatment, prevention or amelioration of hyperproliferative diseases and disorders responsive to the induction of apoptosis. Particularly, the invention relates to a compound according to the invention or a pharmaceutically acceptable salt thereof, for the treatment, prevention or amelioration of benign neoplasia or malignant neoplasia, particularly cancer.
The invention further relates to a pharmaceutical composition, comprising a compound according to the invention or a pharmaceutically acceptable salt thereof, for the treatment, prevention or amelioration of hyperproliferative diseases and disorders responsive to the induction of apoptosis. Particularly, the invention relates to a pharmaceutical composition, comprising a compound according to the invention or a pharmaceutically acceptable salt thereof, for the treatment, prevention or amelioration of benign neoplasia or malignant neoplasia, particularly cancer.

The present invention further relates to pharmaceutical compositions comprising one or more of the compounds, or a salt, stereoisomer or a salt of a stereoisomer thereof, according to the invention and a pharmaceutically acceptable carrier or diluent.

The present invention particularly relates to pharmaceutical compositions comprising one or more compounds, or a salt, stereoisomer or a salt of a stereoisomer thereof, according to the invention together with pharmaceutically acceptable auxiliaries and/or excipients.

The present invention further relates to a combination comprising one or more of the compounds, or a salt, stereoisomer or a salt of a stereoisomer thereof, according to the invention and pharmaceutically acceptable auxiliaries, excipients and/or vehicles, e.g. for treating, preventing or ameliorating benign neoplasia and malignant neoplasia, particularly cancer, such as e.g. any of those cancer diseases described above.

The present invention further relates to a composition comprising a therapeutically effective and tolerable amount of one or more compounds, or a salt, stereoisomer or a salt of a stereoisomer thereof, according to the invention together with the usual pharmaceutically acceptable vehicles, diluents and/or excipients for use in therapy, e.g. for treating, preventing or ameliorating hyperproliferative diseases, such as e.g. cancer, and/or disorders responsive to induction of apoptosis.

The present invention further relates to compounds, or a salt, stereoisomer or a salt of a stereoisomer thereof, according to the invention having Eg5 inhibiting properties. The present invention further relates to compounds, or a salt, stereoisomer or a salt of a stereoisomer thereof, according to the invention having anti-proliferative and/or apoptosis inducing activity.

The present invention further relates to pharmaceutical compositions according to the invention having Eg5 inhibiting properties. The present invention further relates to pharmaceutical compositions according to the invention having anti-proliferative activity. The present invention further relates to pharmaceutical compositions according to the invention having apoptosis inducing activity.

The invention further relates to the use of a pharmaceutical composition comprising one or more of the compounds, or a salt, stereoisomer or a salt of a stereoisomer thereof, according to the invention as sole active ingredient(s) and a pharmaceutically acceptable carrier or diluent in the manufacture of pharmaceutical products for the treatment and/or prophylaxis of the illnesses mentioned above.

Additionally, the invention relates to an article of manufacture, which comprises packaging material and a pharmaceutical agent contained within said packaging material, wherein the pharmaceutical agent is therapeutically effective inhibiting Eg5 and/or inhibiting cellular hyperproliferation and/or inducing apoptosis, ameliorating the symptoms of a Eg5 mediated disease and/or a hyperproliferative disease and/or a disorder responsive to the induction of apoptosis, and wherein the packaging material comprises a label or package insert which indicates that the pharmaceutical agent is useful for preventing or treating a Eg5 mediated disease and/or a hyperproliferative disease and/or a disorder responsive to the induction of apoptosis, and wherein said pharmaceutical agent comprises one or more compounds, or a salt, stereoisomer or a salt of a stereoisomer thereof, according to the invention. The packaging material, label and package insert otherwise parallel or resemble what is generally regarded as standard packaging material, labels and package inserts for pharmaceuticals having related utilities.

The pharmaceutical compositions according to the invention are prepared by processes which are known per se and familiar to the person skilled in the art. As pharmaceutical compositions, the compounds, or a salt, stereoisomer or a salt of a stereoisomer thereof, according to the invention are either employed as such, or preferably in combination with suitable pharmaceutical auxiliaries and/or excipients, e.g. in the form of tablets, coated tablets, dragees, pills, cachets, granules, capsules, caplets, suppositories, patches (e.g. as TTS), emulsions (such as e.g. micro-emulsions or lipid emulsions), suspensions (such as e.g. nano suspensions), gels, solubilisates or solutions (e.g. sterile solutions), or encapsuled in liposomes or as beta-cyclodextrine or beta-cyclodextrin derivative inclusion complexes or the like, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, vehicles, excipients, diluents, carriers or adjuvants which are suitable for the desired pharmaceutical formulations, preparations or compositions on account of his/her expert knowledge. In addition to solvents, gel formers, ointment bases and other active compound excipients, for example antioxidants, dispersants, emulsifiers, preservatives, solubilizers (such as e.g. polyoxyethylenglyceroltriricinoleat 35, PEG 400, Tween 80, Captisol, Solutol HS15 or the like), colorants, complexing agents, permeation promoters, stabilizers, fillers, binders, thickeners, disintegrating agents, buffers, pH regulators (e.g. to obtain neutral, alkaline or acidic formulations), polymers, lubricants, coating agents, propellants, tonicity adjusting agents, surfactants, flavorings, sweeteners or dyes, can be used.

In particular, auxiliaries and/or excipients of a type appropriate to the desired formulation and the desired mode of administration are used.

The administration of the compounds, pharmaceutical compositions or combinations according to the invention may be performed in any of the generally accepted modes of administration available in the art. Illustrative examples of suitable modes of administration include intravenous, oral, nasal, parenteral, topical, transdermal and rectal delivery. Oral and intravenous delivery are preferred.

For the treatment of dermatoses, the compounds of the invention can be in particular administered in the form of those pharmaceutical compositions which are suitable for topical application. For the production of the pharmaceutical compositions, the compounds of the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, lotions, pastes, gels or solutions.

The pharmaceutical compositions according to the invention can be prepared by processes known per se. The dosage of the compounds of the invention (= active compounds) is carried out in the order of magnitude customary for Eg5 inhibitors, inhibitors for cellular hyperproliferation or apoptosis inducers. Topical application forms (such as ointments) for the treatment of dermatoses thus contain the active compounds in a concentration of, for example, 0.1-99%. The customary dose in the case of systemic therapy (p.o.) may be between 0.03 and 60 mg/kg per day, (i. v.) may be between 0.03 and 60 mg/kg/h. In another embodiment, the customary dose in the case of systemic therapy (p.o.) is between 0.3 and 30 mg/kg per day, (i. v.) is between 0.3 and 30 mg/kg/h.

The choice of the optimal dosage regime and duration of medication, particularly the optimal dose and manner of administration of the active compounds necessary in each case can be determined by a person skilled in the art on the basis of his/her expert knowledge.

Depending upon the particular disease to be treated or prevented, additional therapeutic active agents, which are normally administered to treat or prevent that disease, may optionally be coadministered with the compounds, or a salt, stereoisomer or a salt of a stereoisomer thereof, according to the invention. As used herein, additional therapeutic agents that are normally administered to treat or prevent a particular disease are known as appropriate for the disease being treated.

For example, compounds, or a salt, stereoisomer or a salt of a stereoisomer thereof, according to the invention may be combined with one or more standard therapeutic agents used for treatment of the diseases as mentioned before.

In this context, the present invention further relates to a combination comprising
a first active ingredient, which is at least one compound or a salt, stereoisomer or a salt of a stereoisomer thereof, according to the invention, and
a second active ingredient, which is at least one anti-cancer agent.
Preferred is a combination for separate, sequential, simultaneous, concurrent or chronologically staggered use in the treatment, prevention or amelioration of hyperproliferative diseases and disorders responsive to the induction of apoptosis.

Preferred is a combination, in which the second active ingredient is one or more of those art-known anti-cancer agents that is mentioned herein below. Preferred is a combination for use in therapy of any of those diseases mentioned herein, particularly in the treatment, prevention or amelioration of hyperproliferative diseases and disorders responsive to the induction of apoptosis, more particularly in the treatment of cancer.

The term "combination" according to the invention may be present as a fixed combination, a non-fixed combination or a kit-of-parts.

A "fixed combination" is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A "kit-of-parts" is defined as a combination wherein the said first active ingredient and the said second active ingredient are present in more than one unit. One example of a "kit-of-parts" is a combination wherein the said first active ingredient and the said second active ingredient are present separately. The components of the kit-of-parts may be administered separately, sequentially, simultaneously, concurrently or chronologically staggered.

The present invention further relates to a pharmaceutical composition comprising
a first active ingredient, which is at least one compound according to the invention, and
a second active ingredient, which is at least one art-known anti-cancer agent, such as e.g. one or more of those mentioned herein above, and, optionally,
a pharmaceutically acceptable carrier or diluent, for separate, sequential, simultaneous, concurrent or chronologically staggered use in therapy.

The present invention further relates to a combination product comprising
a.) at least one compound according to the invention formulated with a pharmaceutically acceptable carrier or diluent, and
b.) at least one art-known anti-cancer agent, such as e.g. one or more of those mentioned herein above, formulated with a pharmaceutically acceptable carrier or diluent.

The present invention further relates to a kit-of-parts comprising a preparation of a first active ingredient, which is a compound according to the invention, and a pharmaceutically acceptable carrier or diluent; a preparation of a second active ingredient, which is an art-known anti-cancer agent, such as one of those mentioned above, and a pharmaceutically acceptable carrier or diluent; for simultaneous, concurrent, sequential, separate or chronologically staggered use in therapy. Optionally, said kit comprises instructions for its use in therapy, e.g. to treat hyperproliferative diseases and/or disorders responsive to the induction of apoptosis, such as e.g. cancer, more precisely, any of those cancer diseases described above.

The present invention further relates to a combined preparation comprising at least one compound according to the invention and at least one art-known anti-cancer agent for simultaneous, concurrent, sequential or separate administration.

The present invention further relates to combinations, compositions, formulations, preparations or kits according to the present invention having Eg5 inhibitory activity and/or anti-proliferative and/or apoptosis inducing properties.

In one particular embodiment, compounds, or a salt, stereoisomer or a salt of a stereoisomer thereof, according to the invention may be combined with one or more art-known anti-cancer agents, such as e.g. with one or more chemotherapeutic and/or target specific anti-cancer agents as described below.

Examples of known chemotherapeutic anti-cancer agents frequently used in combination therapy include, but not are limited to (i) alkylating/carbamylating agents such as Cyclophosphamid (Endoxan®), Ifosfamid (Holoxan®), Thiotepa (Thiotepa Lederle®), Melphalan (Alkeran®), or chloroethylnitrosourea (BCNU); (ii) platinum derivatives like cis-platin (Platinex® BMS), oxaliplatin, satraplatin or carboplatin (Cabroplat® BMS); (iii) antimitotic agents / tubulin inhibitors such as vinca alkaloids (vincristine, vinblastine, vinorelbine), taxanes such as Paclitaxel (Taxol®), Docetaxel (Taxotere®) and analogs as well as new formulations and conjugates thereof (like the nanoparticle formulation Abraxane® with paclitaxel bound to albumin), epothilones such as Epothilone B (Patupilone®), Azaepothilone (Ixabepilone®) or ZK-EPO, a fully synthetic epothilone B analog; (iv) topoisomerase inhibitors such as anthracyclines (exemplified by Doxorubicin / Adriblastin®), epipodophyllotoxines (examplified by Etoposide / Etopophos®) and camptothecin and camptothecin analogs (exemplified by Irinotecan / Camptosar® or Topotecan / Hycamtin®); (v) pyrimidine antagonists such as 5-fluorouracil (5-FU), Capecitabine (Xeloda®), Arabinosylcytosine / Cytarabin (Alexan®) or Gemcitabine (Gemzar®); (vi) purin antagonists such as 6-mercaptopurine (Puri-Nethol®), 6-thioguanine or fludarabine (Fludara®) and finally (vii) folic acid antagonists such as methotrexate (Farmitrexat®) or premetrexed (Alimta®).

Examples of anti-cancer agents, preferably target specific anti-cancer-agents, used in experimental or standard cancer therapy include but are not limited to (i) kinase inhibitors such as e.g. Imatinib (Glivec®), ZD-1839 / Gefitinib (Iressa®), Bay43-9006 (Sorafenib, Nexavar®), SU11248 / Sunitinib (Sutent®), OSI-774 / Erlotinib (Tarceva®), Dasatinib (Sprycel®), Lapatinib (Tykerb®), or, see also below, Vatalanib, Vandetanib (Zactima®) or Pazopanib; (ii) proteasome inhibitors such as PS-341 / Bortezumib (Velcade®); (iii) histone deacetylase inhibitors like SAHA (Zolinza®), PXD101, MS275, MGCD0103, Depsipeptide / FK228, NVP-LBH589, NVP-LAQ824, Valproic acid (VPA), CRA / PCI 24781, ITF2357, SB939 and butyrates (iv) heat shock protein 90 inhibitors like 17-allylaminogeldanamycin (17-AAG) or 17-dimethylaminogeldanamycin (17-DMAG); (v) vascular targeting agents (VTAs) like combretastin A4 phosphate or AVE8062 / AC7700 and anti-angiogenic drugs like the VEGF antibodies, such as Bevacizumab (Avastin®), or KDR tyrosine kinase inhibitors such as PTK787 / ZK222584 (Vatalanib) or Vandetanib (Zactima®) or Pazopanib; (vi) monoclonal antibodies such as Trastuzumab (Herceptin®) or Rituximab (MabThera / Rituxan®) or Alemtuzumab (Campath®) or Tositumomab (Bexxar®) or C225/ Cetuximab (Erbitux®) or Avastin (see above) or Bevacizumab or Panitumumab (Vectibix®) as well as mutants and conjugates of monoclonal antibodies, e.g. Gemtuzumab ozogamicin (Mylotarg®) or Ibritumomab tiuxetan (Zevalin®), and antibody fragments; (vii) oligonucleotide based therapeutics like G-3139 / Oblimersen (Genasense®) or the DNMT1 inhibitor MG98; (viii) Toll-like receptor / TLR 9 agonists like Promune®, TLR 7 agonists like Imiquimod (Aldara®) or Isatoribine and analogues thereof, or TLR 7/8 agonists like Resiquimod as well as immunostimulatory RNA as TLR 7/8 agonists; (ix) protease inhibitors (x) hormonal therapeutics such as anti-estrogens (e.g. Tamoxifen or Raloxifen), anti-androgens (e.g. Flutamide or Casodex), LHRH analogs (e.g. Leuprolide, Goserelin or Triptorelin) and aromatase inhibitors.

Other known anti-cancer agents which may be used for combination therapy include bleomycin, retinoids such as all-trans retinoic acid (ATRA), DNA methyltransferase inhibitors such as 5-Aza-2'-deoxycytidine (Decitabine, Dacogen®) and 5-azacytidine, alanosine, cytokines such as interleukin-2, interferons such as interferon α2 or interferon-y, death receptor agonists, such as TRAIL, DR4/5 agonistic antibodies, FasL and TNF-R agonists (e.g. TRAIL receptor agonists like mapatumumab or lexatumumab).

As exemplary anti-cancer agents, which may be useful in the combination therapy according to the present invention, any of the following drugs may be mentioned, without being restricted thereto,
5 FU, actinomycin D, ABARELIX, ABCIXIMAB, ACLARUBICIN, ADAPALENE, ALEMTUZUMAB, ALTRETAMINE, AMINOGLUTETHIMIDE, AMIPRILOSE, AMRUBICIN, ANASTROZOLE, ANCITABINE, ARTEMISININ, AZATHIOPRINE, BASILIXIMAB, BENDAMUSTINE, BEVACIZUMAB, BEXXAR, BICALUTAMIDE, BLEOMYCIN, BORTEZOMIB, BROXURIDINE, BUSULFAN, CAMPATH, CAPECITABINE, CARBOPLATIN, CARBOQUONE, CARMUSTINE, CETRORELIX, CHLORAMBUCIL, CHLORMETHINE, CISPLATIN, CLADRIBINE, CLOMIFENE, CYCLOPHOSPHAMIDE, DACARBAZINE, DACLIZUMAB, DACTINOMYCIN, DASATINIB, DAUNORUBICIN, DECITABINE, DESLORELIN, DEXRAZOXANE, DOCETAXEL, DOXIFLURIDINE, DOXORUBICIN, DROLOXIFENE, DROSTANOLONE, EDELFOSINE, EFLORNITHINE, EMITEFUR, EPIRUBICIN, EPITIOSTANOL, EPTAPLATIN, ERBITUX, ERLOTINIB, ESTRAMUSTINE, ETOPOSIDE, EXEMESTANE, FADROZOLE, FINASTERIDE, FLOXURIDINE, FLUCYTOSINE, FLUDARABINE, FLUOROURACIL, FLUTAMIDE, FORMESTANE, FOSCARNET, FOSFESTROL, FOTEMUSTINE, FULVESTRANT, GEFITINIB, GENASENSE, GEMCITABINE, GLIVEC, GOSERELIN, GUSPERIMUS, HERCEPTIN, IDARUBICIN, IDOXURIDINE, IFOSFAMIDE, IMATINIB, IMPROSULFAN, INFLIXIMAB, IRINOTECAN, IXABEPILONE, LANREOTIDE, LAPATINIB, LETROZOLE, LEUPRORELIN, LOBAPLATIN, LOMUSTINE, LUPROLIDE, MELPHALAN, MERCAPTOPURINE, METHOTREXATE, METUREDEPA, MIBOPLATIN, MIFEPRISTONE, MILTEFOSINE, MIRIMOSTIM, MITO-GUAZONE, MITOLACTOL, MITOMYCIN, MITOXANTRONE, MIZORIBINE, MOTEXAFIN, MYLOTARG, NARTOGRASTIM, NEBAZUMAB, NEDAPLATIN, NILUTAMIDE, NIMUSTINE, OCTREOTIDE, ORMELOXIFENE, OXALIPLATIN, PACLITAXEL, PALIVIZUMAB, PANITUMUMAB, PATUPILONE, PAZOPANIB, PEGASPARGASE, PEGFILGRASTIM, PEMETREXED, PENTETREOTIDE, PENTOSTATIN, PERFOSFAMIDE, PIPOSULFAN, PIRARUBICIN, PLICAMYCIN, PREDNIMUSTINE, PROCARBAZINE, PROPAGERMANIUM, PROSPIDIUM CHLORIDE, RALOXIFEN, RALTITREXED, RANIMUSTINE, RANPIRNASE, RASBURICASE, RAZOXANE, RITUXIMAB, RIFAMPICIN, RITROSULFAN, ROMURTIDE, RUBOXISTAURIN, SARGRAMOSTIM, SATRAPLATIN, SIROLIMUS, SOBUZOXANE, SORAFENIB, SPIROMUSTINE, STREPTOZOCIN, SUNITINIB, TAMOXIFEN, TASONERMIN, TEGAFUR, TEMOPORFIN, TEMOZOLOMIDE, TENIPOSIDE, TESTOLACTONE, THIOTEPA, THYMALFASIN, TIAMIPRINE, TOPOTECAN, TOREMIFENE, TRAIL, TRASTUZUMAB, TREOSULFAN, TRIAZIQUONE, TRIMETREXATE, TRIPTORELIN, TROFOSFAMIDE, UREDEPA, VALRUBICIN, VATALANIB, VANDETANIB, VERTEPORFIN, VINBLASTINE, VINCRISTINE, VINDESINE, VINORELBINE, VOROZOLE and ZEVALIN.

The anti-cancer agents mentioned herein above as combination partners of the compounds, or a salt, stereoisomer or a salt of a stereoisomer thereof, according to the invention are meant to include pharmaceutically acceptable derivatives thereof, such as e.g. their pharmaceutically acceptable salts.

The person skilled in the art is aware on the base of his/her expert knowledge of the kind, total daily dosage(s) and administration form(s) of the additional therapeutic agent(s) coadministered. Said total daily dosage(s) can vary within a wide range.

In practicing the present invention, the compounds, or a salt, stereoisomer or a salt of a stereoisomer thereof, according to the invention may be administered in combination therapy separately, sequentially, simultaneously, concurrently or chronologically staggered (such as e.g. as combined unit dosage forms, as separate unit dosage forms, as adjacent discrete unit dosage forms, as fixed or non-fixed combinations, as kit-of-parts or as admixtures) with one or more standard therapeutics (chemotherapeutic and/or target specific anti-cancer agents), in particular art-known anti-cancer agents, such as any of e.g. those mentioned above.

The combinations, e.g. compositions, preparations, formulations, kits or packages mentioned in the context of the combination therapy according to the invention may also include more than one of the compounds, or a salt, stereoisomer or a salt of a stereoisomer thereof, according to the invention and/or more than one of the art-known anti-cancer agents mentioned.

The first and second active ingredient of a combination or kit-of-parts according to the invention may be provided as separate formulations (i.e. independently of one another), which are subsequently brought together for simultaneous, concurrent, sequential, separate or chronologically staggered use in combination therapy; or packaged and presented together as separate components of a combination pack for simultaneous, concurrent, sequential, separate or chronologically staggered use in combination therapy.

The type of pharmaceutical formulation of the first and second active ingredient of a combination or kit-of-parts according to the invention can be the same, i.e. both ingredients are formulated in separate tablets or capsules, or can be different, i.e. suited for different administration forms, such as e.g. one active ingredient is formulated as tablet or capsule and the other is formulated for e.g. intravenous administration.

The amounts of the first and second active ingredients of the combinations, compositions or kits according to the invention may together comprise a therapeutically effective amount for the treatment, prophylaxis or amelioration of a (hyper)proliferative diseases and/or a disorder responsive to the induction of apoptosis, particularly one of those diseases mentioned herein, such as e.g. malignant or benign neoplasia, especially cancer, like any of those cancer diseases mentioned herein.

In addition, compounds according to the present invention can be used in the pre- or post-surgical treatment of cancer. In further addition, compounds of the present invention can be used in combination with radiation therapy.

A combination according to the invention can refer to a composition comprising both the compound(s) according to the invention and the other active anti-cancer agent(s) in a fixed combination (fixed unit dosage form), or a medicament pack comprising the two or more active ingredients as discrete separate dosage forms (non-fixed combination). In case of a medicament pack comprising the two or more active ingredients, the active ingredients are preferably packed into blister cards which are suited for improving compliance.

Each blister card preferably contains the medicaments to be taken on one day of treatment. If the medicaments are to be taken at different times of day, the medicaments can be disposed in different sections on the blister card according to the different ranges of times of day at which the medicaments are to be taken (for example morning and evening or morning, midday and evening). The blister cavities for the medicaments to be taken together at a particular time of day are accommodated in the respective range of times of day. The various times of day are, of course, also put on the blister in a clearly visible way. It is also possible, of course, for example to indicate a period in which the medicaments are to be taken, for example stating the times.

The daily sections may represent one line of the blister card, and the times of day are then identified in chronological sequence in this column.

Medicaments which must be taken together at a particular time of day are placed together at the appropriate time on the blister card, preferably a narrow distance apart, allowing them to be pushed out of the blister easily, and having the effect that removal of the dosage form from the blister is not forgotten.

In addition, the present invention further relates to:
- a pharmaceutical composition comprising one or more compounds according to the present invention together with customary pharmaceutical auxiliaries and/or excipients for treating, preventing and/or ameliorating (hyper) proliferative diseases and/or disorders responsive to induction of apoptosis as well as for modulating the Eg5 kinesin activity in a mammal;
- compounds according to the present invention for use in the treatment of diseases, for use in treating, preventing and/or ameliorating (hyper) proliferative diseases and/or disorders responsive to induction of apoptosis as well as for use in modulating the Eg5 kinesin activity in a mammal;
- a use of the compounds according to the present invention in the manufacture of pharmaceutical compositions for treating, preventing and/or ameliorating (hyper) proliferative diseases and/or disorders responsive to induction of apoptosis as well as for modulating the Eg5 kinesin activity in a mammal;
- a method for treating, preventing or ameliorating (hyper)proliferative diseases and/or disorders responsive to induction of apoptosis as well as for modulating the Eg5 kinesin activity in a mammal comprising administering a therapeutically effective and tolerable amount of one or more compounds according the present invention to said mammal in need thereof;
- a combination comprising
   a first active ingredient, which is at least one compound according to the present invention, and a second active ingredient, which is at least one anti-cancer agent selected from the group consisting of chemotherapeutic anti-cancer agents and target-specific anti-cancer agents,
   for separate, sequential, simultaneous, concurrent or chronologically staggered use in therapy, such as e.g. therapy of (hyper)proliferative diseases and/or disorders responsive to the induction of apoptosis;
- the use of said combinations according to the invention for the production of pharmaceutical compositions which are used in the treatment, prevention or amelioration of hyperproliferative diseases and disorders responsive to the induction of apoptosis;
- a commercial package comprising a said combination. In a preferred aspect, (hyper)proliferative diseases and disorders responsive to the induction of apoptosis include benign neoplasia and/or malignant neoplasia, e.g. cancer.

### Biological Investigations

The anti-proliferative / cytotoxic activity of the compounds described herein can be tested on subclones of RKO human colon adenocarcinoma cells (Schmidt et al., Oncogene 19, 2423-2429; 2000) using the Alamar Blue cell viability assay (described in O'Brien et al. Eur J Biochem 267, 5421-5426, 2000). The compounds are dissolved as 10 mM solutions in DMSO and subsequently diluted in semi-logarithmic steps. DMSO dilutions are further diluted 1:100 v:v into Dulbecco's modified Eagle's medium (DMEM) containing 10% fetal calf serum to a final concentration twice as much as the final concentration in the test. RKO subclones are seeded into 96 well flat bottom plates at a density of 4000 cells per well in a volume of 50 µl per well. 24 hours after seeding the 50 µl each of the compound dilutions in DMEM medium are added into each well of the 96 well plate. Each compound dilution is tested as triplicates. Wells containing untreated control cells are filled with 50 µl DMEM medium containing 1% DMSO. The cells are then incubated with the substances for 72 hours at 37°C in a humidified atmosphere containing 5% carbon dioxide. To determine the viability of the cells, 10 µl of an Alamar Blue solution (Biosource) are added and the fluorescence is measured at an extinction of 544 nm and an emission of 590 nm. For the calculation of the cell viability the emission value from untreated cells is set as 100% viability and the emission rates of treated cells are set in relation to the values of untreated cells. Viabilities are expressed as % values. The Graphpad Prism program is used for the calculation of EC₅₀ values for anti-proliferative / cytotoxic activity out of the obtained dose-response curves.

To determine the cell cycle specific mode of action, subclones of RKO colon adenocarcinoma cells (RKOp21 or RKOp27 as described by Schmidt et al. in Oncogene 19, 2423-2429; 2000) are seeded into 96 well flat bottom plates at a density of 16000 cells per well in a volume of 50 µl per well in DMEM growth medium with 10% FCS containing 10 µM Ponasterone A. 24 hours after seeding the 50 µl each of the compound dilutions in DMEM medium are added into each well of the 96-well plate. Each compound dilution is tested as triplicates. Wells containing untreated control cells are filled with 50 µl DMEM medium containing 1% DMSO. The cells are then incubated with the substances for 72 hours at 37°C in a humidified atmosphere containing 5% carbon dioxide. To determine the viability of the cells, 10 µl of an Alamar Blue solution (Biosource) are added and the fluorescence is measured at an extinction of 544 nm and an emission of 590 nm. For the calculation of the cell viability the emission value from untreated cells is set as 100% viability and the emission rates of treated cells are set in relation to the values of untreated cells. Viabilities are expressed as % values. The Graphpad Prism program (GraphPad Software, Inc) is used for the calculation of EC₅₀ values out of the obtained dose-response curves. Viability is compared of proliferating cells grown in the absence of the inducer Ponasterone A, versus viability of cells arrested by the expression of ectopic p27Kipl induced by Ponasterone A.

Representative values for anti-proliferation / cytotoxicity [measured as -log EC₅₀ (mol/l)] determined in the aforementioned assays follow from the following table A, in which the numbers of the compounds correspond to the numbers of the examples.

**Table A**

| **Anti-proliferative / cytotoxic activity on RKO colon cancer cells** | |
|---|---|
| | Examples |
| -log EC₅₀ [mol/l] RKO p27 uninduced (proliferating) >= 7.0 | 1, 1a, 2, 4, 5, 7, 8, 9, 10, 12, 13, 15, 17, 19, 20, 21 |
| -log EC₅₀ [mol/l] RKO p27 uninduced (proliferating) < 7.0 but >= 6.0 | 3, 6, 11, 14, 16, 18 3, 6, 11, 14, 16, 18 |

The value of -log EC₅₀ [mol/l] RKO p27 induced (arrested) was below the minimum determined by the assay specification (<= 4.0, <= 5.0, <= 5.5 or <= 6.0).

## Claims

1. Compounds of formula I 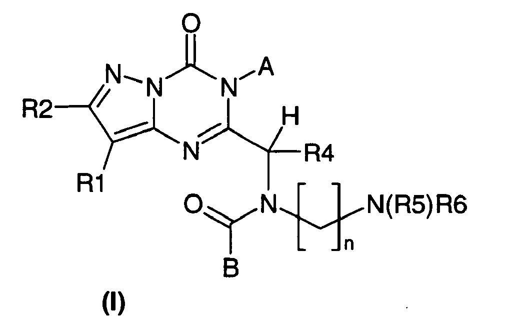 in which
R1 is hydrogen or halogen,
R2 is 1-4C-alkyl, 1-4C-alkoxy, hydrogen, 1-2C-haloalkoxy
A is Aryl-1-4C-alkyl, in which
Aryl is phenyl, or R3- and R31-substituted phenyl, in which
R3 is 1-4C-alkyl, trifluoromethyl, 1-4C-alkoxy, hydroxyl, halogen, or completely or predominantly fluorine-substituted 1-4C-alkoxy,
R31 is hydrogen, halogen, 1-4C-alkyl or 1-4C-alkoxy,
R4 is hydrogen, 1-4C-alkyl, 3-7C-cycloalkyl or 3-7C-cycloalkyl-1-4C-alkyl,
R5 is hydrogen or 1-4C-alkyl,
R6 is hydrogen or 1-4C-alkyl,
B is phenyl, or R7- and R71-substituted phenyl, in which
R7 is 1-4C-alkyl, trifluoromethyl, cyano, 1-4C-alkoxy, halogen, carboxyl, 1-4C-alkylcarbonyl, 1-4C-alkoxy-1-4C-alkyl, hydroxy-1-4C-alkyl, or completely or predominantly fluorine-substituted 1-4C-alkoxy,
R71 is hydrogen, halogen, 1-4C-alkyl or 1-4C-alkoxy, or
R7 and R71 when located in ortho position to each other and taken together are methylenedioxy or ethylenedioxy,
n is 2, 3, 4, 5 or 6,
and the salts, stereoisomers and the salts of the stereoisomers of these compounds.

2. Compounds of formula I according to claim 1, in which
R1 is hydrogen or halogen,
R2 is methyl or ethyl, methoxy or ethoxy, hydrogen, halomethoxy or haloethoxy,
A is Arylmethyl, in which
Aryl is phenyl, or R3- and R31-substituted phenyl, in which
R3 is methyl, ethyl, trifluoromethyl, methoxy, ethoxy, hydroxyl, halogen, difluoromethoxy or trifluoromethoxy,
R31 is hydrogen, halogen, methyl, ethyl, methoxy or ethoxy,
R4 is methyl, ethyl, n-propyl, isopropyl, sec-butyl, cyclopropyl or cyclopropylmethyl,
R5 is hydrogen, methyl, ethyl, n-propyl or isopropyl,
R6 is hydrogen, methyl, ethyl, n-propyl or isopropyl,
B is phenyl, or R7- and R71-substituted phenyl, in which
R7 is methyl, ethyl, trifluoromethyl, cyano, methoxy, ethoxy, halogen, carboxyl, acetyl, methoxymethyl, hydroxymethyl, difluoromethoxy or trifluoromethoxy,
R71 is hydrogen, halogen, methyl, ethyl, methoxy or ethoxy,
or
R7 and R71 when located in ortho position to each other and taken together are methylenedioxy or ethylenedioxy,
n is 2, 3 or 4,
and the salts, stereoisomers and the salts of the stereoisomers of these compounds.

3. Compounds of formula I according to claim 1, in which
R1 is hydrogen, chlorine or bromine,
R2 is methyl or ethyl, methoxy or ethoxy, hydrogen, halomethoxy or haloethoxy,
A is benzyl, halobenzyl (e.g. fluorobenzyl, chlorobenzyl or bromobenzyl), methylbenzyl, methoxybenzyl or hydroxybenzyl,
R4 is ethyl, n-propyl, isopropyl, sec-butyl or cyclopropyl, either
R5 and R6 are both hydrogen,
or
R5 is methyl, and
R6 is hydrogen,
or
R5 is ethyl, and
R6 is hydrogen,
or
R5 is n-propyl, and
R6 is hydrogen,
or
R5 is isopropyl, and
R6 is hydrogen,
or
R5 and R6 are both methyl,
B is phenyl, or R7- and R71-substituted phenyl, in which
R7 is methyl, ethyl, trifluoromethyl, cyano, methoxy, ethoxy, halogen, carboxyl, methoxymethyl or hydroxymethyl,
R71 is hydrogen, halogen, methyl or ethyl, or
R7 and R71 when located in ortho position to each other and taken together are methylenedioxy or ethylenedioxy,
n is 2 or 3,
and the salts, stereoisomers and the salts of the stereoisomers of these compounds.

4. Compounds of formula I according to any of the preceding claims comprising one or more of the following:
R1 is chlorine or bromine;
R2 is methyl, methoxy, difluoromethoxy or hydrogen;
A is benzyl;
R4 is ethyl or isopropyl;
either
R5 and R6 are both hydrogen,
or
R5 and R6 are both methyl;
B is 4-methylphenyl, 3-methylphenyl, 4-trifluoromethylphenyl, 4-methoxyphenyl, 4-fluorophenyl, 4-chlorophenyl, 4-bromophenyl or 3-fluoro-4-methylphenyl; and
n is 3;
and the salts, stereoisomers and the salts of the stereoisomers of these compounds.

5. Compounds of formula I according to claim 1, in which
R1 is hydrogen, chlorine, bromine or fluorine,
R2 is methyl, methoxy, difluoromethoxy or hydrogen,
A is benzyl,
R4 is ethyl, isopropyl, sec-butyl, cyclopropyl or cyclobutyl,
either
R5 and R6 are both hydrogen,
or
R5 and R6 are both methyl,
B is 4-methylphenyl, 3-methylphenyl, 4-fluorophenyl, 4-bromophenyl, 4-chlorophenyl, 4-methoxyphenyl, 4-trifluoromethylphenyl, 3-fluoro-4-methylphenyl, 2-fluoro-4-methylphenyl, 3,4-dichlorophenyl or 2,3-dichlorophenyl,
n is 2, 3 or 4,
and the salts, stereoisomers and the salts of the stereoisomers of these compounds.

6. Compounds of formula I according to claim 1, in which
R1 is chlorine, bromine or fluorine,
R2 is methyl, methoxy, difluoromethoxy or hydrogen,
A is benzyl,
R4 is ethyl, isopropyl, sec-butyl or cyclopropyl,
R5 and R6 are both hydrogen,
B is 4-methylphenyl, 3-methylphenyl, 4-fluorophenyl, 4-bromophenyl, 4-chlorophenyl, 4-methoxyphenyl, 3-fluoro-4-methylphenyl, 2-fluoro-4-methylphenyl or 3,4-dichlorophenyl
n is 3 or 4,
and the salts, stereoisomers and the salts of the stereoisomers of these compounds.

7. Compounds of formula I according to claim 1, in which
R1 is chlorine or hydrogen,
R2 is methyl, methoxy, difluoromethoxy or hydrogen,
R5 and R6 are both hydrogen,
B is 4-methylphenyl
n is 3,
provided that not both R1 and R2 are hydrogen,
and the salts, stereoisomers and the salts of the stereoisomers of these compounds.

8. Compounds of formula I according to any of the preceding claims, wherein said compounds have the configuration as shown in formula I* 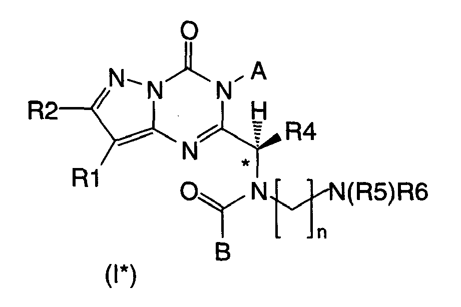 and the salts thereof.

9. Compounds of formula I, selected from:
(1) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide
(2) N-(2-Amino-ethyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide
(3) N-[(RS)-(3-Benzyl-8-chloro-7-methyl-4-oxo-3,4-dihydro-pyrazolo[1,5-a][1,3,5]triazin-2-yl)-methyl-n-propyl]-N-(2-dimethylamino-ethyl)-4-methyl-benzamide
(4) N-(3-Amino-n-propyl)-N-[(RS)-1-(3-benzyl-8-chloro-4-oxo-3,4-dihydro-pyrazolo[1,5-a][1,3,5]triazin-2-yl)-2-methyl-n-propyl]-4-methyl-benzamide
(5) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-chloro-benzamide
(6) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-fluoro-benzamide
(7) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3-methyl-benzamide
(8) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-bromo-benzamide
(9) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-trifluoromethyl-benzamide
(10) N-(3-Amino-n-propyl)-N-{(RS)-1-[3-benzyl-7-(1,1-difluoro-methoxy)-4-oxo-3,4-dihydro-pyrazolo[1,5-a][1,3,5]triazin-2-yl]-2-methyl-n-propyl}-4-methyl-benzamide
(11) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3,4-dichloro-benzamide
(12) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-2-fluoro-4-methyl-benzamide
(13) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-3-fluoro-4-methyl-benzamide
(14) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-2,3-dichloro-benzamide
(15) N-(3-Amino-n-propyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methoxy-benzamide
(16) N-(4-Amino-n-butyl)-N-[(RS)-1-(3-benzyl-8-chloro-7-methyl-4-oxo-3,4-dihydro-pyrazolo[1,5-a][1,3,5]triazin-2-yl)-2-methyl-n-propyl]-4-methyl-benzamide
(17) N-(3-Amino-n-propyl)-N-{(RS)-1-[3-benzyl-8-chloro-7-(1,1-difluoro-methoxy)-4-oxo-3,4-dihydro-pyrazolo[1,5-a][1,3,5]triazin-2-yl]-2-methyl-n-propyl}-4-methyl-benzamide
(18) N-[(RS)-(3-Benzyl-8-chloro-7-methyl-4-oxo-3,4-dihydro-pyrazolo[1,5-a][1,3,5]triazin-2-yl)-methyl-n-propyl]-N-(4-dimethylamino-n-butyl)-4-methyl-benzamide
(19) N-[(RS)-(3-Benzyl-8-chloro-7-methyl-4-oxo-3,4-dihydro-pyrazolo[1,5-a][1,3,5]triazin-2-yl)-methyl-n-propyl]-N-(3-dimethylamino-n-propyl)-4-methyl-benzamide
(20) N-(3-Amino-n-propyl)-N-[(RS)-1-(3-benzyl-7-methoxy-4-oxo-3,4-dihydro-pyrazolo[1,5-a][1,3,5]triazin-2-yl)-2-methyl-n-propyl]-4-methyl-benzamide
(21) N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-methyl-benzamide
(22) N-(3-Amino-n-propyl)-N-[(RS)-1-(3-benzyl-8-chloro-4-oxo-3,4-dihydro-pyrazolo[1,5-a][1,3,5]triazin-2-yl)-n-propyl]-4-methyl-benzamide
(23) N-(3-Amino-n-propyl)-N-[(RS)-1-(3-benzyl-8-chloro-4-oxo-3,4-dihydro-pyrazolo[1,5-a][1,3,5]triazin-2-yl)-n-propyl]-4-chloro-benzamide
(24) N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-2-fluoro-4-methyl-benzamide
(25) N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-3-fluoro-4-methyl-benzamide
(26) N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-methoxy-benzamide
(27) N-(3-Amino-n-propyl)-N-[(RS)-1-(3-benzyl-8-chloro-7-methoxy-4-oxo-3,4-dihydro-pyrazolo[1,5-a][1,3,5]triazin-2-yl)-2-methyl-n-propyl]-4-chloro-benzamide
and the salts, stereoisomers and the salts of the stereoisomers of these compounds.

10. Process for preparing a compound according to any of claims 1 to 9, comprising at least one of the steps
(i) benzoylation of a compound of formula XXXIII, with the meanings of R1, R2, A, R4, R5 and R6 as indicated for the compounds according to any of claims 1 to 9, or with at least one of R5 and R6 being part of a protective group, 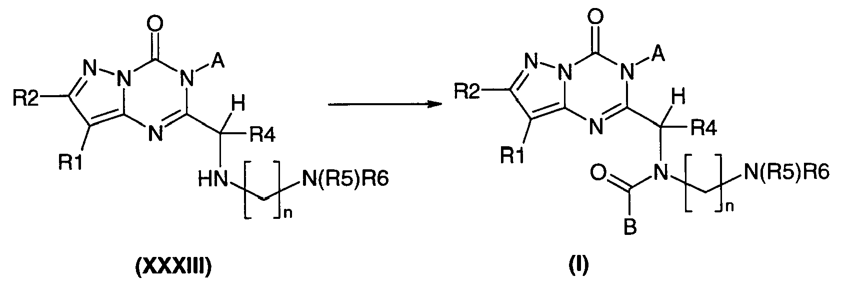
(ii) halogenation of a compound of formula I wherein R1 = H to give a compound of formula I wherein R1 = halogen, or 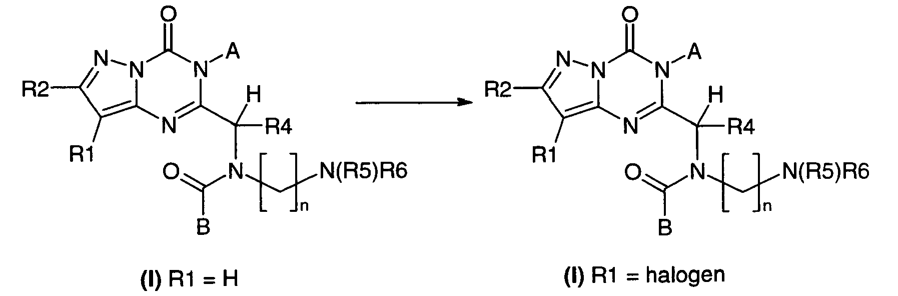
(iii) optionally the removal of protecting groups represented by at least one of R5 and R6 as indicated under (i).

11. A pharmaceutical composition comprising one or more compounds according to any of the claims 1 to 9 together with customary pharmaceutical auxiliaries and/or excipients.

12. Pharmaceutical composition according to claim 11 for treating, preventing and/or ameliorating (hyper) proliferative diseases and/or disorders responsive to induction of apoptosis, which include benign neoplasia and malignant neoplasia, including cancer.

13. Pharmaceutical composition according to claim 11 for modulating the Eg5 kinesin activity in a mammal.

14. Compounds according to any of the claims 1 to 9 for use in the treatment of diseases.

15. Compounds according to any of the claims 1 to 9 for use in treating, preventing and/or ameliorating (hyper) proliferative diseases and/or disorders responsive to induction of apoptosis, such as, for example, benign and/or malignant neoplasia, e.g. cancer.

16. Compounds according to any of the claims 1 to 9 for use in modulating the Eg5 kinesin activity in a mammal.

17. Use of the compounds according to any of the claims 1 to 9 in the manufacture of pharmaceutical compositions for treating, preventing and/or ameliorating (hyper)proliferative diseases and/or disorders responsive to induction of apoptosis, such as, for example, benign and/or malignant neoplasia, e.g. cancer.

18. Use of the compounds according to any of the claims 1 to 9 in the manufacture of pharmaceutical compositions for modulating the the Eg5 kinesin activity in a mammal.

19. A method for treating, preventing or ameliorating (hyper)proliferative diseases and/or disorders responsive to induction of apoptosis, such as, for example, benign or malignant neoplasia, e.g. cancer, in a mammal comprising administering a therapeutically effective and tolerable amount of one or more compounds according to any of the claims 1 to 9 to said mammal in need thereof.

20. A method for modulating Eg5 kinesin activity comprising administering a therapeutically effective and tolerable amount of one or more compounds according to any of the claims 1 to 9 to a mammal in need of said modulation.

21. A combination comprising
a first active ingredient, which is at least one compound according to any of the claims 1 to 9, and
a second active ingredient, which is at least one anti-cancer agent selected from the group consisting of chemotherapeutic anti-cancer agents and target-specific anti-cancer agents, for separate, sequential, simultaneous, concurrent or chronologically staggered use in therapy, such as e.g. therapy of (hyper)proliferative diseases of benign or malignant behaviour and/or disorders responsive to the induction of apoptosis, such as, for example, benign or malignant neoplasia, e.g. cancer.

22. The pharmaceutical composition according to claim 12, the compounds according to claims 15, the use according to claim 17 or the method according to claim 19, wherein the treating, preventing and/or ameliorating comprises administering separately, simultaneously, concurrently, sequentially or chronologically staggered to said patient in need thereof
an amount of a first active compound, which is a compound according to any of the claims 1 to 9, and
an amount of at least one second active compound, said second active compound being an anti-cancer agent selected from the group consisting of chemotherapeutic anti-cancer agents and target-specific anti-cancer agents,
wherein the amounts of the first active compound and said second active compound result in a therapeutic effect.

23. The combination or the pharmaceutical composition, the compounds, the use or the method according to claim 21 or 22, in which said chemotherapeutic anti-cancer agents are selected from (i) alkylating/carbamylating agents including Cyclophosphamid, Ifosfamid, Thiotepa, Melphalan and chloroethylnitrosourea; (ii) platinum derivatives including cis-platin, oxaliplatin, satraplatin and carboplatin; (iii) antimitotic agents / tubulin inhibitors including vinca alkaloids, such as e.g. vincristine, vinblastine or vinorelbine, taxanes, such as e.g. Paclitaxel, Docetaxel and analogs as well as formulations and conjugates thereof, and epothilones, such as e.g. Epothilone B, Azaepothilone or ZK-EPO; (iv) topoisomerase inhibitors including anthracyclines, such as e.g. Doxorubicin, epipodophyllotoxines, such as e.g. Etoposide, and camptothecin and camptothecin analogs, such as e.g. Irinotecan or Topotecan; (v) pyrimidine antagonists including 5-fluorouracil, Capecitabine, Arabinosylcytosine / Cytarabin and Gemcitabine; (vi) purin antagonists including 6-mercaptopurine, 6-thioguanine and fludarabine; and (vii) folic acid antagonists including methotrexate and pemetrexed.

24. The combination or the pharmaceutical composition, the compounds, the use or the method according to claim 21 or 22 in which said target-specific anti-cancer agents are selected from (i) kinase inhibitors including Imatinib, ZD-1839 / Gefitinib, BAY43-9006 / Sorafenib, SU11248 / Sunitinib and OSI-774 / Erlotinib; (ii) proteasome inhibitors including PS-341 / Bortezomib; (iii) histone deacetylase inhibitors including SAHA, PXD101, MS275, MGCD0103, Depsipeptide / FK228, NVP-LBH589, NVP-LAQ824, Valproic acid (VPA) and butyrates; (iv) heat shock protein 90 inhibitors including 17-allylaminogeldanamycin (17-AAG); (v) vascular targeting agents (VAT) including combretastatin A4 phosphate and AVE8062 / AC7700, and anti-angiogenic drugs including VEGF antibodies, such as e.g. Bevacizumab, and KDR tyrosine kinase inhibitors, such as e.g. PTK787 / ZK222584 (Vatalanib); (vi) monoclonal antibodies including Trastuzumab, Rituximab, Alemtuzumab, Tositumab, Cetuximab and Bevacizumab as well as mutants and conjugates of monoclonal antibodies, such as e.g. Gemtuzumab ozogamicin or Ibritumomab tiuxetan, and antibody fragments; (vii) oligonucleotide based therapeutics including G-3139 / Oblimersen; (viii) Toll-like receptor / TLR 9 agonists including Promune®, TLR 7 agonists including Imiquimod and Isatoribine and analogues thereof, or TLR 7/8 agonists including Resiquimod as well as immunostimulatory RNA as TLR 7/8 agonists; (ix) protease inhibitors; (x) hormonal therapeutics including anti-estrogens, such as e.g. Tamoxifen or Raloxifen, anti-androgens, such as e.g. Flutamide or Casodex, LHRH analogs, such as e.g. Luprolide, Goserelin or Triptorelin, and aromatase inhibitors; bleomycin; retinoids including all-trans retinoic acid (ATRA); DNA methyltransferase inhibitors including the 2-deoxycytidine derivative Decitabine and 5-azacytidine; alanosine; cytokines including interleukin-2; interferons including interferon α2 and interferon-γ; and death receptor agonists including TRAIL, DR4/5 agonistic antibodies, FasL and TNF-R agonists.

25. The pharmaceutical composition according to claim 12, the compounds according to claims 15, the use according to claim 17 or the method according to claim 19 in which said cancer is selected from the group consisting of
cancer of the breast, bladder, bone, brain, central and peripheral nervous system, colon, endocrine glands, esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva; inherited cancers, retinomblastoma and Wilms tumor; leukemia, lymphoma, non-Hodgkins disease, chronic and acute myeloid leukaemia, acute lymphoblastic leukemia, Hodgkins disease, multiple myeloma and T-cell lymphoma; myelodysplastic syndrome, plasma cell neoplasia, paraneoplastic syndromes, cancers of unknown primary site and AIDS related malignancies.
